(19) Europäisches Patentamt
European Patent Office
Office européen des brevets

(11) EP 3 645 028 B1

(12) EUROPEAN PATENT SPECIFICATION

(45) Date of publication and mention of the grant of the patent:
**09.07.2025 Bulletin 2025/28**

(21) Application number: **18823785.3**

(22) Date of filing: **26.06.2018**

(51) International Patent Classification (IPC):
***G16B 20/20*** *(2019.01)* ***C07K 14/74*** *(2006.01)*
***C12Q 1/6883*** *(2018.01)* ***C12Q 1/6886*** *(2018.01)*
***G01N 33/564*** *(2006.01)* ***G01N 33/574*** *(2006.01)*
***G01N 33/569*** *(2006.01)*

(52) Cooperative Patent Classification (CPC):
**G01N 33/56977; C07K 14/70539; C12Q 1/6883; C12Q 1/6886; G01N 33/564; G01N 33/574; G16B 20/20;** A61K 38/00; C12Q 2600/156; G01N 2800/50

(86) International application number:
**PCT/US2018/039455**

(87) International publication number:
**WO 2019/005764 (03.01.2019 Gazette 2019/01)**

# (54) MHC-1 GENOTYPE RESTRICTS THE ONCOGENIC MUTATIONAL LANDSCAPE

DIE ONKOGENE MUTATIONSLANDSCHAFT EINSCHRÄNKENDER MHC-1-GENOTYP

GÉNOTYPE DU CMH-I LIMITANT LE PAYSAGE MUTATIONNEL ONCOGÈNE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **27.06.2017 US 201762525539 P**

(43) Date of publication of application:
**06.05.2020 Bulletin 2020/19**

(73) Proprietors:
- **Institute For Cancer Research d/b/a The Research Institute of Fox Chase Cancer Center**
  **Philadelphia, PA 19111-2497 (US)**
- **Universitat Pompeu-Fabra**
  **08005 Barcelona (ES)**
- **The Regents of the University of California**
  **Oakland, CA 94607-5200 (US)**

(72) Inventors:
- **FONT-BURGADA, Joan**
  **Philadelphia, PA 19111-2497 (US)**
- **ROSSELL, David**
  **08005 Barcelona (ES)**
- **CARTER, Hannah, K.**
  **Oakland, CA 94607-5200 (US)**
- **MARTY, Rachel**
  **Oakland, CA 94607-5200 (US)**

(74) Representative: **Bassil, Nicholas Charles**
**Kilburn & Strode LLP**
**Lacon London**
**84 Theobalds Road**
**London WC1X 8NL (GB)**

(56) References cited:
**US-A1- 2013 330 335** **US-A1- 2014 038 901**
**US-A1- 2016 069 895** **US-A1- 2016 102 359**
**US-A1- 2017 175 197**

- **JAHAN S. KHALILI ET AL: "In silico prediction of tumor antigens derived from functional missense mutations of the cancer gene census", ONCOIMMUNOLOGY, vol. 1, no. 8, 1 November 2012 (2012-11-01), pages 1281 - 1289, XP055144271, ISSN: 2162-4011, DOI: 10.4161/onci.21511**

EP 3 645 028 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **LIU X SHIRLEY ET AL: "Applications of Immunogenomics to Cancer", CELL, ELSEVIER, AMSTERDAM NL, vol. 168, no. 4, 9 February 2017 (2017-02-09), pages 600 - 612, XP029935434, ISSN: 0092-8674, DOI: 10.1016/ J.CELL.2017.01.014**
- **MARTY, R ET AL.: "MHC-I genotype restricts the oncogenic mutational landscape", CELL, vol. 171, no. 6, 30 November 2017 (2017-11-30), pages 1 - 12, XP085296990**
- **KIM, M ET AL.: "Empirical prediction of genomic susceptibilities for multiple cancer classes", PROCEEDINGS OF THE NATIONAL ACADEMY OF SCIENCES, vol. 111, no. 5, 4 February 2014 (2014-02-04), pages 1921 - 1926, XP055566847**

## Description

### Field

The present disclosure is directed to computer-implemented methods of determining the risk of a subject having or developing a cancer based on the affinity of MHC-I for oncogenic mutations.

### Background

Avoiding immune destruction is a hallmark of cancer (Hanahan and Weinberg, Cell, 2011, 144, 646-674), suggesting that the ability of the immune system to detect and eliminate neoplastic cells is a major deterrent to tumor progression. Recent studies have demonstrated that the immune system is capable of eliminating tumors when the mechanisms that tumor cells employ to evade detection are countered (Brahmer et al., N. Engl. J. Med., 2012, 366, 2455-2465; Hodi et al., N. Engl. J. Med., 2010, 363, 711-723; and Topalian et al., N. Engl. J. Med., 2012, 366, 2443-2454). This discovery has motivated new efforts to identify the characteristics of tumors that render them susceptible to immunotherapy (Rizvi et al., Science, 2015, 348, 124-128; and Rooney et al., Cell, 2015, 160, 48-61). Less attention has been directed toward the role of the immune system in shaping the tumor genome prior to immune evasion; however, such early interactions may have important implications for the characteristics of the developing tumor.

While the potential of manipulating the immune system for treating cancer has now been clearly demonstrated, its role in determining characteristics of tumors remains poorly understood in humans. The theory of cancer immunosurveillance dictates that the immune system should exert a negative selective pressure on tumor cell populations through elimination of tumor cells that harbor antigenic mutations or aberrations. Under this model, tumor precursor cells with antigenic variants would be at higher risk for immune elimination and, conversely, tumor cell populations that continue to expand should be biased toward cells that avoid producing neoantigens.

One major mechanism by which tumor cells can be detected is the antigen presentation pathway. Endogenous peptides generated within tumor cells are bound to the MHC-I complex and displayed on the cell surface where they are monitored by T cells. Mutations in tumors that affect protein sequence have the potential to elicit a cytotoxic response by generating neoantigens. In order for this to happen, the mutated protein product must be cleaved into a peptide, transported to the endoplasmic reticulum, bound to an MHC-I molecule, transported to the cell surface, and recognized as foreign by a T cell (Schumacher and Schreiber, Science, 2015, 348, 69-74). According to the theory of cancer immunosurveillance, the immune system exerts a negative selective pressure on those tumor cells that harbor antigenic mutations or aberrations. Tumor precursor cells presenting antigenic variants would be at higher risk for immune elimination and, conversely, tumors that grow would be biased toward those that successfully avoid immune elimination. Immune evasion could be achieved by either losing or failing to acquire antigenic variants.

In model organisms, there is strong experimental evidence that immunosurveillance sculpts the genomes of tumors through detection and elimination of cancer cells early in tumor progression (DuPage et al., Nature, 2012, 482, 405-409; Kaplan et al., Proc. Natl. Acad. Sci. USA, 1998, 95, 7556-7561; Koebel et al., Nature, 2007, 450, 903-907; Matsushita et al., Nature, 2012, 482, 400-404; and Shankaran et al., Nature, 2001, 410, 1107-111). In humans, the observed frequency of neoantigens has been reported to be unexpectedly low in some tumor types (Rooney et al., Cell, 2015, 160, 48-61), suggesting that immunoediting could be taking place. However, this phenomenon has been challenging to study systematically, in part due to the highly polymorphic nature of the HLA locus where the genes that encode MHC-I proteins are located (over 10,000 distinct alleles for the three genes documented to date; Robinson et al., Nucleic Acids Res., 2015, 43, D423-D431).

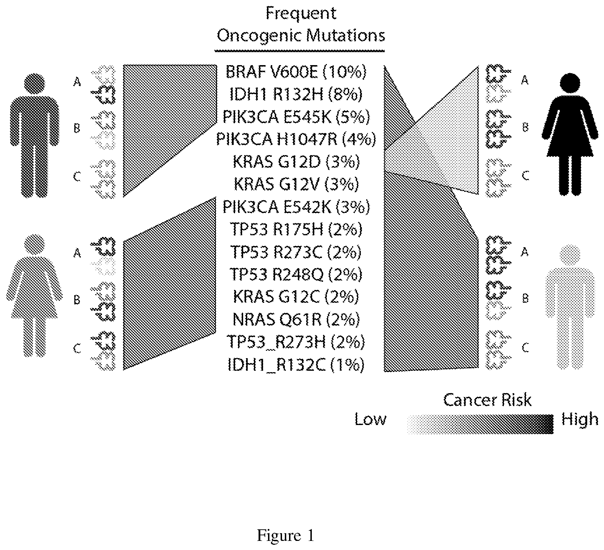

The polymorphic nature of the HLA locus raises the possibility that the set of oncogenic mutations that create neoantigens may differ substantially among individuals. Indeed, neoantigens found to drive tumor regression in response to immunotherapy were almost always unique to the responding tumor (Lu et al., Int. Immunol., 2016, 28, 365-370). Several studies have also reported that nonsynonymous mutation burden, rather than the presence of any particular mutation, is the common factor among responsive tumors (Rizvi et al., Science, 2015, 348, 124-128). The paucity of recurrent oncogenic mutations driving effective responses to immunotherapy is suggestive that these mutations may less frequently be antigenic, possibly as a result of selective pressure by the immune system during tumor development. This suggests that that recurrent oncogenic mutations are immune-selected early on during tumor initiation and that this selection should strongly depend on the capability of the MHC-I to effectively present recurrent oncogenic mutations (see, Figure 1). A direct inference that can be drawn from this hypothesis is that the capability of the set of MHC-I alleles carried by an individual to present oncogenic mutations may play a key role in determining which oncogenic mutations can be recognized by that individual's immune system. Hence, determining the MHC-I genotype of any individual can lead directly to a prediction of the subset of the oncogenic peptidome that individual's immune system would be able to detect, with important implications for predicting individual cancer susceptibility.

Accordingly, there is a need for an effective model capable of predicting which oncogenic mutations are detectable by an individual's MHC-I-based immunosurveillance system. Such a model would help assess an individual's susceptibility to various cancers. In addition, a need exists for a model capable of predicting oncogenic mutations that are not efficiently presented to the MHC-I-based immunosurveillance system. Such a model would help in the development of diagnostic assays aimed at early detection of oncogenic and pre-oncogenic conditions. US 2016/102359 discloses a gene for predicting or diagnosing the prognosis of breast cancer, more specifically a genetic marker for predicting or diagnosing the prognosis of breast cancer, including TRBC1 (T cell receptor beta constant 1), BTN3A2 (butyrophilin, subfamily 3 member A2) or HLA-DPA1 (major histocompatibility complex, class II, DP alpha 1).

**Summary**

The invention is as set out in the appended set of claims. The present disclosure provides computer implemented methods for determining whether a subject is at risk of having or developing a cancer or an autoimmune disease, the method comprising: a) genotyping the subject's major histocompatibility complex class I (MHC-I); and b) scoring the ability of the subject's MHC-I to present a mutant cancer-associated peptide or an autoimmune-associated peptide based upon a library of known cancer-associated peptide sequences or autoimmune-associated peptide sequences derived from subjects, wherein the produced score is the MHC-I presentation score; wherein: i) if the subject is a poor MHC-I presenter of specific mutant cancer-associated peptides, the subject has an increased likelihood of having or developing the cancer for which the specific mutant cancer-associated peptides are associated; ii) if the subject is a good MHC-I presenter of specific mutant cancer-associated peptides, the subject has a decreased likelihood of having or developing the cancer for which the specific mutant cancer-associated peptides are associated; iii) if the subject is a poor MHC-I presenter of specific autoimmune-associated peptides, the subject has a decreased likelihood of having or developing autoimmunity for which the specific autoimmune-associated peptides are associated; or iv) if the subject is a good MHC-I presenter of specific autoimmune-associated peptides, the subject has an increased likelihood of having or developing autoimmunity for which the specific autoimmune-associated peptides are associated.

The present disclosure also provides computing systems for determining whether a subject is at risk of having or developing a cancer or an autoimmune disease, the system comprising: a) a communication system for using a library of cancer-associated peptides or autoimmune-associated peptides derived from subjects; and b) a processor for scoring the ability of the subject's major histocompatibility complex class I (MHC-I) to present a mutant cancer-associated peptide or an autoimmune-associated peptide based upon a library of cancer-associated peptides or autoimmune-associated peptides derived from subjects, wherein the produced score is the MHC-I presentation score.

The present disclosure also provides methods of detecting an early stage breast invasive carcinoma (BRCA) in a subject, the method comprising the steps of: a) obtaining a biological sample from the subject; and b) assaying the sample for the presence of any of the B-Raf Proto-Oncogene (BRAF) V600E mutation, Phosphatidylinositol-4,5-Bisphosphate 3-Kinase Catalytic Subunit Alpha (PIK3CA) E545K mutation, PIK3CA E542K mutation, PIK3CA H1047R mutation, Kirsten Rat Sarcoma Viral Oncogene Homolog (KRAS) G12D mutation, KRAS G13D mutation, KRAS G12V mutation, KRAS A146T mutation, TP53 R175H mutation, TP53 H179R mutation, TP53 mutation, TP53 R248Q mutation, TP53 R273C mutation, TP53 R273H mutation, TP53 R282W mutation, Keratin Associated Protein 4-11 (KRTAP4-11) L161V mutation, Mab-21 Domain Containing 2 (MB21D2) Q311E, mutation, HLA-A Q78R mutation, Harvey Rat Sarcoma Viral Oncogene Homolog (HRAS) G13V mutation, Isocitrate Dehydrogenase (NADP(+)) 1 (IDH1) R132H mutation, IDH1 R132C mutation, IDH1 R132G mutation, IDH2 R172K mutation, IDH1 R132S mutation, Capicua Transcriptional Repressor (CIC) R215W mutation, Phosphoglucomutase 5 (PGM5) I98V mutation, Tripartite Motif Containing 48 (TRIM48) Y192H mutation, or F-Box And WD Repeat Domain Containing 7 (FBXW7) R465C mutation, wherein the presence of any one of these mutations indicates the presence of early stage breast invasive carcinoma.

The present disclosure also provides methods of detecting an early stage colon adenocarcinoma (COAD) in a subject, the method comprising the steps of: a) obtaining a biological sample from the subject; and b) assaying the sample for the presence of any of the BRAF V600E mutation, Neuroblastoma RAS Viral Oncogene Homolog (NRAS) Q61R mutation, NRAS Q61K mutation, NRAS Q61L mutation, IDH1 R132S mutation, Mitogen-Activated Protein Kinase Kinase 1 (MAP2K1) P124S mutation, Rac Family Small GTPase 1 (RAC1) P29S mutation, Protein Phosphatase 6 Catalytic Subunit (PPP6C) R301C mutation, Cyclin Dependent Kinase Inhibitor 2A (CDKN2A) P114L mutation, Keratin Associated Protein 4-11 (KRTAP4-11) L161V mutation, KRTAP4-11 M93V mutation, HRAS Q61R mutation, HLA-A Q78R mutation, Zinc Finger Protein 799 (ZNF799) E589G mutation, Zinc Finger Protein 844 (ZNF844) R447P mutation, or RNA Binding Motif Protein 10 (RBM10) E184D mutation, wherein the presence of any one of these mutations indicates the presence of early stage colon adenocarcinoma.

The present disclosure also provides methods of detecting an early stage head and neck squamous cell carcinoma (HNSC) in a subject, the method comprising the steps of: a) obtaining a biological sample from the subject; and b) assaying the sample for the presence of any of the IDH1 R132H mutation, IDH1 R132C mutation, IDH1 R132G mutation, IDH1 R132S mutation, IDH2 R172K mutation, TP53 H179R mutation, TP53 R273C mutation, TP53 R273H mutation, CIC R215W mutation, or HLA-A Q78R mutation, wherein the presence of any one of these mutations indicates the presence of early stage head and neck squamous cell carcinoma.

The present disclosure also provides methods of detecting an early stage brain lower grade glioma (LGG) in a subject, the method comprising the steps of: a) obtaining a biological sample from the subject; and b) assaying the sample for the presence of any of the IDH1 R132H mutation, IDH1 R132C mutation, IDH1 R132G mutation, IDH1 R132S mutation, IDH2 R172K mutation, TP53 H179R mutation, TP53 R273C mutation, TP53 R273H mutation, CIC R215W mutation, or HLA-A Q78R mutation, wherein the presence of any one of these mutations indicates the presence of early stage brain lower grade glioma.

The present disclosure also provides methods of detecting an early stage lung adenocarcinoma (LUAD), in a subject, the method comprising the steps of: a) obtaining a biological sample from the subject; and b) assaying the sample for the presence of any of the BRAF V600E mutation, PIK3CA E545K mutation, KRAS G12D mutation, KRAS G13D mutation, KRAS A146T mutation, TP53 R175H mutation, KRAS G12V mutation, TP53 R248Q mutation, TP53 R273C mutation TP53 R273H mutation, TP53 R282W mutation, PGM5 I98V mutation, TRIM48 Y192H mutation, PIK3CA E545K mutation, KRAS G13D mutation, PIK3CA H1047R mutation, or FBXW7 R465C mutation, wherein the presence of any one of these mutations indicates the presence of early stage lung adenocarcinoma.

The present disclosure also provides methods of detecting an early stage lung squamous cell carcinoma (LUSC) in a subject, the method comprising the steps of: a) obtaining a biological sample from the subject; and b) assaying the sample for the presence of any of the PIK3CA H1047R mutation, PIK3CA E545K mutation, PIK3CA E542K mutation, TP53 R175H mutation, PIK3CA N345K mutation, AKT Serine/Threonine Kinase 1 (AKT1) E17K mutation, Splicing Factor 3b Subunit 1 (SF3B1) K700E mutation, or PIK3CA H1047L mutation, wherein the presence of any one of these mutations indicates the presence of early stage lung squamous cell carcinoma.

The present disclosure also provides methods of detecting an early stage skin cutaneous melanoma (SKCM) in a subject, the method comprising the steps of: a) obtaining a biological sample from the subject; and b) assaying the sample for the presence of any of the BRAF V600E mutation, PIK3CA E545K mutation, KRAS G12D mutation, KRAS G13D mutation, KRAS A146T mutation, KRAS G12V mutation, TP53 R175H mutation, TP53 H179R mutation, TP53 R248Q mutation TP53 R273C mutation, TP53 R273H mutation, TP53 R282W mutation, IDH1 R132H mutation, IDH1 R132C mutation, IDH1 R132G mutation, IDH1 R132S mutation, IDH2 R172K mutation, CIC R215W mutation, or HLA-A Q78R mutation, NRAS Q61R mutation, NRAS Q61K mutation, NRAS Q61L mutation, MAP2K1 P124S mutation, RAC1 P29S mutation, PPP6C R301C mutation, CDKN2A P114L mutation, KRTAP4-11 L161V mutation, KRTAP4-11 M93V mutation, HRAS Q61R mutation, ZNF799 E589G mutation, ZNF844 R447P mutation, or RBM10 E184D mutation, wherein the presence of any one of these mutations indicates the presence of early stage skin cutaneous melanoma.

The present disclosure also provides methods of detecting an early stage stomach adenocarcinoma (STAD) in a subject, the method comprising the steps of: a) obtaining a biological sample from the subject; and b) assaying the sample for the presence of any of the KRAS G12C mutation, KRAS G12V mutation, Epidermal Growth Factor Receptor (EGFR) L858R mutation, KRAS G12D mutation, KRAS G12A mutation, U2 Small Nuclear RNA Auxiliary Factor 1 (U2AF1) S34F mutation, KRTAP4-11 L161V mutation, KRTAP4-11 R121K mutation, Eukaryotic Translation Elongation Factor 1 Beta 2 (EEF1B2) R42H mutation, or KRTAP4-11 M93V mutation, wherein the presence of any one of these mutations indicates the presence of early stage stomach adenocarcinoma.

The present disclosure also provides methods of detecting an early stage thyroid carcinoma (THCA) in a subject, the method comprising the steps of: a) obtaining a biological sample from the subject; and b) assaying the sample for the presence of any of the BRAF V600E mutation, PIK3CA E545K mutation, KRAS G12D mutation, KRAS G13D mutation, TP53 R175H mutation, KRAS G12V mutation, TP53 R248Q mutation, KRAS A146T mutation, TP53 R273H mutation, HRAS Q61R mutation, HLA-A Q78R mutation, TP53 R282W mutation, NRAS Q61R mutation, NRAS Q61K mutation, IDH1 R132C mutation, MAP2K1 P124S mutation, RAC1 P29S mutation, NRAS Q61L mutation, PPP6C R301C mutation, CDKN2A P114L mutation, KRTAP4-11 L161V mutation, KRTAP4-11 M93V mutation, ZNF799 E589G mutation, ZNF844 R447P mutation, or RBM10 E184D mutation, wherein the presence of any one of these mutations indicates the presence of early stage thyroid carcinoma.

The present disclosure also provides methods of detecting an early stage uterine corpus endometrial carcinoma (UCEC) in a subject, the method comprising the steps of: a) obtaining a biological sample from the subject; and b) assaying the sample for the presence of any of the BRAF V600E mutation, PIK3CA H1047R mutation, PIK3CA E545K mutation, PIK3CA E542K mutation, TP53 R175H mutation, PIK3CA N345K mutation, AKT Serine/Threonine Kinase 1 (AKT1) E17K mutation, Splicing Factor 3b Subunit 1 (SF3B1) K700E mutation, KRAS G12C mutation, KRAS G12V mutation, Epidermal Growth Factor Receptor (EGFR) L858R mutation, KRAS G12D mutation, KRAS G12A mutation, KRAS G12V mutation, KRAS G13D mutation, TP53 R175H mutation, TP53 R248Q mutation, KRAS A146T mutation, TP53 R273H mutation, TP53 R282W mutation, U2 Small Nuclear RNA Auxiliary Factor 1 (U2AF1) S34F mutation, KRTAP4-11 L161V mutation, KRTAP4-11 R121K mutation, Eukaryotic Translation Elongation Factor 1 Beta 2 (EEF1B2) R42H mutation, or KRTAP4-11 M93V mutation, wherein the presence of any one of these mutations indicates the presence of early stage uterine corpus endometrial carcinoma.

**Brief Description Of The Drawings**

Figure 1 shows MHC-I genotype immune selection in cancer; schematic representing individuals and their combinations of MHCs; each individual's MHCs are better equipped to present specific mutations, rendering them less likely to develop cancer harboring those mutations.

Figure 2A shows a graphical representation of calculating the presentation score for a particular residue, each residue can be presented in 38 different peptides of differing lengths between 8 and 11.

Figure 2B shows single-allele MS data from Abelin et al. (Abelin et al., Mass Immunity, 2017, 46, 315-326) compared to a random background of peptides to determine the best residue-centric score for quantifying of extracellular presentation (best rank score shown).

Figure 2C shows a ROC curve showing the accuracy of the best rank residue presentation score for classifying the extracellular presentation of a residue by an MHC allele; the aggregated presentation scores for MS data from 16 different alleles was compared to a random set of residues with the same 16 alleles.

Figure 2D shows the fraction of native residues found for the list of mutations identified in five different cancer cell lines for strong (rank <0.5) and weak (0.5% rank <2) binders; the mutated version of the residue is assumed to be presented if the mutation does not disrupt the binding motif.

Figure 3A shows the number of 8-11-mer peptides that differed from the native sequence for recurrent in-frame indels pan-cancer.

Figure 3B shows the distribution of residue-centric presentation scores for MS-observed peptides and randomly selected residues for best rank.

Figure 3C shows the distribution of residue-centric presentation scores for MS-observed peptides and randomly selected residues for summation (rank < 2).

Figure 3D shows the distribution of residue-centric presentation scores for MS-observed peptides and randomly selected residues for summation (rank <0.5).

Figure 3E shows the distribution of residue-centric presentation scores for MS-observed peptides and randomly selected residues for best rank with cleavage.

Figure 3F shows the log of the ratio between the fraction of MS-observed residues and the fraction of random residues detected over regular score intervals for best rank.

Figure 3G shows the log of the ratio between the fraction of MS-observed residues and the fraction of random residues detected over regular score intervals for summation (rank < 2).

Figure 3H shows the log of the ratio between the fraction of MS-observed residues and the fraction of random residues detected over regular score intervals for summation (rank <0.5).

Figure 3I shows the log of the ratio between the fraction of MS-observed residues and the fraction of random residues detected over regular score intervals for best rank with cleavage.

Figure 3J shows a ROC curve revealing the accuracy of classification for several different presentation scoring schemes.

Figure 3K shows a heatmap showing the AUCs for the 16 alleles for each presentation scoring scheme.

Figure 4A shows a bar chart representing the number of peptides recovered from the mass spectrometry data for each HLA allele (cell lines: HeLa, FHIOSE, SKOV3, 721.221, A2780, and OV90).

Figure 4B shows a bar chart representing the fraction of select residues with high and low presentation scores from the mass spectrometry data from the HLA-A*01:02 allele; values are shown for both the randomly selected residues and the oncogenic residues.

Figure 5A shows a non-parametric estimate of GAM-based mutation probability vs. affinity.

Figure 5B shows a non-parametric estimate of GAM-based logit-mutation probability vs. log-affinity.

Figure 5C shows a non-parametric estimate of frequency of mutation for affinity in groups.

Figure 6A shows a within-residues analysis odds ratio and 95% CIs by cancer type.

Figure 6B shows a within-subjects analysis odds ratio and 95% CIs by cancer type.

Figure 7A shows a within-residues analysis odds ratio and 95% CIs by cancer type for cancer types with ≥ 100 subjects.

Figure 7B shows a within-subjects analysis odds ratio and 95% CIs by cancer type for cancer types with ≥ 100 subjects.

**Description Of Embodiments**

The terminology used herein is for the purpose of describing particular embodiments only and is not intended to be limiting. Various terms relating to aspects of disclosure are used throughout the specification and claims. Such terms are to be given their ordinary meaning in the art, unless otherwise indicated. Other specifically defined terms are to be construed in a manner consistent with the definition provided herein.

Unless otherwise expressly stated, it is in no way intended that any method or aspect set forth herein be construed as requiring that its steps be performed in a specific order. Accordingly, where a method claim does not specifically state in the claims or descriptions that the steps are to be limited to a specific order, it is in no way intended that an order be inferred, in any respect. This holds for any possible non-express basis for interpretation, including matters of logic with respect to arrangement of steps or operational flow, plain meaning derived from grammatical organization or punctuation, or the number or type of aspects described in the specification.

As used herein, the singular forms "a," "an" and "the" include plural referents unless the context clearly dictates otherwise.

As used herein, the terms "subject" and "subject" are used interchangeably. A subject may include any animal, including mammals. Mammals include, without limitation, farm animals (e.g., horse, cow, pig), companion animals (e.g., dog, cat), laboratory animals (e.g., mouse, rat, rabbits), and non-human primates. In some embodiments, the subject is a human being.

The present disclosure provides computer implemented methods for determining whether a subject is at risk of having or developing a cancer or an autoimmune disease, the method comprising: a) genotyping the subject's major histocompatibility complex class I (MHC-I); and b) scoring the ability of the subject's MHC-I to present a mutant cancer-associated peptide or an autoimmune-associated peptide based upon a library of known cancer-associated peptide sequences or autoimmune-associated peptide sequences derived from subjects, wherein the produced score is the MHC-I presentation score; wherein: i) if the subject is a poor MHC-I presenter of specific mutant cancer-associated peptides, the subject has an increased likelihood of having or developing the cancer for which the specific mutant cancer-associated peptides are associated; ii) if the subject is a good MHC-I presenter of specific mutant cancer-associated peptides, the subject has a decreased likelihood of having or developing the cancer for which the specific mutant cancer-associated peptides are associated; iii) if the subject is a poor MHC-I presenter of specific autoimmune-associated peptides, the subject has a decreased likelihood of having or developing autoimmunity for which the specific autoimmune-associated peptides are associated; or iv) if the subject is a good MHC-I presenter of specific autoimmune-associated peptides, the subject has an increased likelihood of having or developing autoimmunity for which the specific autoimmune-associated peptides are associated.

As used herein, the term "genotype" refers to the identity of the alleles present in an individual or a sample. In the context of the present disclosure, a genotype preferably refers to the description of the human leukocyte antigen (HLA) alleles present in an individual or a sample. The term "genotyping" a sample or an individual for an HLA allele consists of determining the specific allele or the specific nucleotide carried by an individual at the HLA locus.

A mutation is "correlated" or "associated" with a specified phenotype (e.g. cancer susceptibility, etc.) when it can be statistically linked (positively or negatively) to the phenotype. Methods for determining whether a polymorphism or allele is statistically linked are well known in the art and described below. The cancer or autoimmune disease-associated mutation may result in a substitution, insertion, or deletion of one or more amino acids within a protein. In some embodiments, the mutant peptides described herein carry known oncogenic mutations that have poor MHC-I-mediated presentation to the immune system due to low affinity of a subject's HLA allele for that particular mutation.

As used herein, the term "oncogene" refers to a gene which is associated with certain forms of cancer. Oncogenes can be of viral origin or of cellular origin. An oncogene is a gene encoding a mutated form of a normal protein (i.e., having an "oncogenic mutation") or is a normal gene which is expressed at an abnormal level (e.g., over-expressed). Over-expression can be caused by a mutation in a transcriptional regulatory element (e.g., the promoter), or by chromosomal rearrangement resulting in subjecting the gene to an unrelated transcriptional regulatory element. The normal cellular counterpart of an oncogene is referred to as "proto-oncogene." Proto-oncogenes generally encode proteins which are involved in regulating cell growth, and are often growth factor receptors. Numerous different oncogenes have been implicated in tumorigenesis. Tumor suppressor genes (e.g., p53 or p53-like genes) are also encompassed by the term "proto-oncogene." Thus, a mutated tumor suppressor gene which encodes a mutated tumor suppressor protein or which is expressed at an abnormal level, in particular an abnormally low level, is referred to herein as "oncogene." The terms "oncogene protein" refer to a protein encoded by an oncogene.

As used herein, the term "mutation" refers to a change introduced into a parental sequence, including, but not limited to, substitutions, insertions, and deletions (including truncations). The consequences of a mutation include, but are not limited to, the creation of a new character, property, function, phenotype or trait not found in the protein encoded by the parental sequence.

Methods of detection of cancer-associated mutations are well known in the art and comprise detection of the nucleic acid and/or protein having a known oncogenic mutation in a test sample or a control sample.

In some embodiments, the methods rely on the detection of the presence or absence of an oncogenic mutation in a population of cells in a test sample relative to a standard (for example, a control sample). In some embodiments, such methods involve direct detection of oncogenic mutations via sequencing known oncogenic mutations loci. In some embodiments, such methods utilize reagents such as oncogenic mutation-specific polynucleotides and/or oncogenic mutation-specific antibodies. In particular, the presence or absence of an oncogenic mutation may be determined by detecting the presence of mutated messenger RNA (mRNA), for example, by DNA-DNA hybridization, RNA-DNA hybridization, reverse transcription-polymerase chain reaction (PGR), real time quantitative PCR, differential display, and/or TaqMan PCR. Any one or more of hybridization, mass spectroscopy (e.g., MALDI-TOF or SELDI-TOF mass spectroscopy), serial analysis of gene expression, or massive parallel signature sequencing assays can also be performed. Non-limiting examples of hybridization assays include a singleplex or a multiplexed aptamer assay, a dot blot, a slot blot, an RNase protection assay, microarray hybridization, Southern or Northern hybridization analysis and in situ hybridization (e.g., fluorescent *in situ* hybridization (FISH)).

For example, these techniques find application in microarray-based assays that can be used to detect and quantify the amount of gene transcripts having oncogenic mutations using cDNA-based or oligonucleotide-based arrays. Microarray technology allows multiple gene transcripts having oncogenic mutations and/or samples from different subjects to be analyzed in one reaction. Typically, mRNA isolated from a sample is converted into labeled nucleic acids by reverse transcription and optionally *in vitro* transcription (cDNAs or cRNAs labelled with, for example, Cy3 or Cy5 dyes) and hybridized in parallel to probes present on an array (see, for example, Schulze et al., Nature Cell. Biol., 2001, 3, E190; and Klein et al., J. Exp. Med., 2001, 194, 1625-1638). Standard Northern analyses can be performed if a sufficient quantity of the test cells can be obtained. Utilizing such techniques, quantitative as well as size-related differences between oncogenic transcripts can also be detected.

In some embodiments, oncogenic mutations are detected using reagents that are specific for these mutations. Such reagents may bind to a target gene or a target gene product (e.g., mRNA or protein), gene product having an oncogenic mutation can be specifically detected. Such reagents may be nucleic acid molecules that hybridize to the mRNA or cDNA of target gene products. Alternatively, the reagents may be molecules that label mRNA or cDNA for later detection, e.g., by binding to an array. The reagents may bind to proteins encoded by the genes of interest. For example, the reagent may be an antibody or a binding protein that specifically binds to a protein encoded by a target gene having an oncogenic mutation of interest. Alternatively, the reagent may label proteins for later detection, e.g., by binding to an antibody on a panel. In some embodiments, reagents are used in histology to detect histological and/or genetic changes in a sample.

Numerous cohorts of mutations associated with particular cancers have been identified in human cancer subjects (e.g., The Cancer Genome Atlas (TCGA) Research Network (world wide web at "cancergenome.nih.gov/"), Nature, 2014, 507, 315-22; and Jiang et al., Bioinformatics, 2007, 23, 306-13). TCGA contains complete exomes of numerous cancer subject cohorts having particular cancer types.

In some embodiments, a custom cancer or autoimmune disease library is obtained by whole genome sequencing of a cohort of at least 100 subjects having cancer or autoimmune disease of interest. In some embodiments, a custom cancer or autoimmune disease library is obtained by whole genome sequencing of a cohort of at least 90 subjects having cancer or autoimmune disease of interest. In some embodiments, a custom cancer or autoimmune disease library is obtained by whole genome sequencing of a cohort of at least 80 subjects having cancer or autoimmune disease of interest. In some embodiments, a custom cancer or autoimmune disease library is obtained by whole genome sequencing of a cohort of at least 70 subjects having cancer or autoimmune disease of interest. In some embodiments, a custom cancer or autoimmune disease library is obtained by whole genome sequencing of a cohort of at least 60 subjects having cancer or autoimmune disease of interest. In some embodiments, a custom cancer or autoimmune disease library is obtained by whole genome sequencing of a cohort of at least 50 subjects having cancer or autoimmune disease of interest. In some embodiments, a custom cancer or autoimmune disease library is obtained by whole genome sequencing of a cohort of at least 40 subjects having cancer or autoimmune disease of interest. In some embodiments, a custom cancer or autoimmune disease library is obtained by whole genome sequencing of a cohort of at least 30 subjects having cancer or autoimmune disease of interest. In some embodiments, a custom cancer or autoimmune disease library is obtained by whole genome sequencing of a cohort of at least 25 subjects having cancer or autoimmune disease of interest. In some embodiments, a custom cancer or autoimmune disease library is obtained by whole genome sequencing of a cohort of at least 20 subjects having cancer or autoimmune disease of interest. In some embodiments, a custom cancer or autoimmune disease library is obtained by whole genome sequencing of a cohort of at least 15 subjects having cancer or autoimmune disease of interest.

In some embodiments, a custom cancer or autoimmune disease library is obtained by Genome Wide Association Studies (GWAS) using approaches well known in the art. For example, association of a mutation to a phenotype optionally includes performing one or more statistical tests for correlation. Many statistical tests are known, and most are computer-implemented for ease of analysis. A variety of statistical methods of determining associations/correlations between phenotypic traits and biological markers are known and can be applied to the methods described herein (e.g., Hartl, A Primer of Population Genetics Washington University, Saint Louis Sinauer Associates, Inc. Sunderland, Mass., 1981, ISBN: 0-087893-271-2). A variety of appropriate statistical models are described in Lynch and Walsh, Genetics and Analysis of Quantitative Traits, Sinauer Associates, Inc. Sunderland Mass., 1998, ISBN 0-87893-481-2. These models can, for example, provide for correlations between genotypic and phenotypic values, characterize the influence of a locus on a phenotype, sort out the relationship between environment and genotype, determine dominance or penetrance of genes, determine maternal and other epigenetic effects, determine principle components in an analysis (via principle component analysis, or "PCA"), and the like. The references cited in these texts provide considerable further detail on statistical models for correlating markers and phenotype.

In some embodiments, all the tumor associated mutations are evaluated in the analysis according to the methods described herein. In some embodiments, only the driver mutations are evaluated in the analysis. As used herein, the term "driver mutation" refers to the subset of mutations within a tumor cell that confer a growth advantage. Methods of identifying driver mutations are known in the art and are described in, for example, PCT Publication No. WO 2012/159754. Alternatively, other criteria for driver mutation selection may be used. For example, the mutations that occur in known oncogenes and have been observed in multiple TCGA samples or in genomic sequences of multiple subjects can be selected.

In some embodiments, the mutations that occur in the 100 most highly ranked oncogenes and observed in at least one TCGA sample or in at least one subject genomic sequence are selected as driver mutations. In some embodiments, the mutations that occur in the 100 most highly ranked oncogenes (e.g., as described by Davoli et al., Cell, 2013, 155, 948-962) and observed in at least two TCGA samples or in at least two subject genomic sequences are selected as driver mutations. In some embodiments, the mutations that occur in the 100 most highly ranked oncogenes and observed in at least three TCGA samples or in at least three subject genomic sequences are selected as driver mutations. In some embodiments, the mutations that occur in the 100 most highly ranked oncogenes and observed in at least four TCGA samples or in at least four subject genomic sequences are selected as driver mutations. In some embodiments, the mutations that occur in the 100 most highly ranked oncogenes and observed in at least five TCGA samples or in at least five subject genomic sequences are selected as driver mutations. In some embodiments, the mutations that occur in the 50 most highly ranked oncogenes and observed in at least one TCGA sample or in at least one subject genomic sequence are selected as driver mutations. In some embodiments, the mutations that occur in the 50 most highly ranked oncogenes and observed in at least two TCGA samples or in at least two subject genomic sequences are selected as driver mutations. In some embodiments, the mutations that occur in the 50 most highly ranked oncogenes and observed in at least three TCGA samples or in at least three subject genomic sequences are selected as driver mutations. In some embodiments, the mutations that occur in the 50 most highly ranked oncogenes and observed in at least four TCGA samples or in at least four subject genomic sequences are selected as driver mutations. In some embodiments, the mutations that occur in the 50 most highly ranked oncogenes and observed in at least five TCGA samples or in at least five subject genomic sequences are selected as driver mutations. In some embodiments, the mutations that occur in the 20 most highly ranked oncogenes and observed in at least one TCGA sample or in at least one subject genomic sequence are selected as driver mutations. In some embodiments, the mutations that occur in the 20 most highly ranked oncogenes and observed in at least two TCGA samples or in at least two subject genomic sequences are selected as driver mutations. In some embodiments, the mutations that occur in the 20 most highly ranked oncogenes and observed in at least three TCGA samples or in at least three subject genomic sequences are selected as driver mutations. In some embodiments, the mutations that occur in the 20 most highly ranked oncogenes and observed in at least four TCGA samples or in at least four subject genomic sequences are selected as driver mutations. In some embodiments, the mutations that occur in the 20 most highly ranked oncogenes and observed in at least five TCGA samples or in at least five subject genomic sequences are selected as driver mutations. In some embodiments, the mutations that occur in the 10 most highly ranked oncogenes and observed in at least one TCGA sample or in at least one subject genomic sequence are selected as driver mutations. In some embodiments, the mutations that occur in the 10 most highly ranked oncogenes and observed in at least two TCGA samples or in at least two subject genomic sequences are selected as driver mutations. In some embodiments, the mutations that occur in the 10 most highly ranked oncogenes and observed in at least three TCGA samples or in at least three subject genomic sequences are selected as driver mutations. In some embodiments, the mutations that occur in the 10 most highly ranked oncogenes and observed in at least four TCGA samples or in at least four subject genomic sequences are selected as driver mutations. In some embodiments, the mutations that occur in the 10 most highly ranked oncogenes and observed in at least five TCGA samples or in at least five subject genomic sequences are selected as driver mutations.

In some embodiments, the selected mutations are further limited to those that would result in predictable protein sequence changes that could generate neoantigens, including missense mutations and in-frame insertions and deletions. In some embodiments, the set of 1018 mutations occurring in one of the 100 most highly ranked oncogenes or tumor suppressors, observed in at least three TCGA samples, and resulting in predictable protein sequence changes that could generate neoantigens, including missense mutations and in-frame insertions and deletions can be selected (see, Tables 24 and 25).

The MHC-I presentation scores for the driver mutation sites can be determined through a residue-centric approach using prediction algorithms. These prediction algorithms can either scan an existing protein sequence from a pathogen for putative T-cell epitopes, or they can predict, whether *de novo* designed peptides bind to a particular MHC molecule. Many such prediction algorithms are commonly known. Examples include, but are not limited to, SVRMHCdb (world wide web at "svrmhc.umn.edu/SVRMHCdb"; Wan et al., BMC Bioinformatics, 2006, 7, 463), SYFPEITHI (world wide web at "syfpeithi.de"), MHCPred (world wide web at "jenner.ac.uk/MHCPred"), motif scanner (world wide web at "hcv.lanl.gov/content/immuno/motif scan/motif scan"), and NetMHCpan (world wide web at "cbs.dtu.dk/services/ NetMHCpan") for MHC I binding epitopes. In some embodiments, the MHC-I presentation scores are obtained using the NetMHCPan 3.0 tool. The values obtained using this tool reflect the affinity of a peptide encompassing an oncogenic mutation for that subject's MHC-I allele, and thereby predict the likelihood of that peptide to be presented by the subject's MHC-I allele, thus generating neoantigens.

In some embodiments the ability of the subject's MHC-I to present a mutant cancer-associated peptide or an autoimmune-associated peptide is determined through fitting a statistical model. In some embodiments, the statistical model is a logistic regression model.

Logistic regression is part of a category of statistical models called generalized linear models. Logistic regression can allow one to predict a discrete outcome, such as group membership, from a set of variables that may be continuous, discrete, dichotomous, or a mix of any of these. The dependent or response variable is dichotomous, for example, one of two possible types of cancer. Logistic regression models the natural log of the odds ratio, i.e., the ratio of the probability of belonging to the first group (P) over the probability of belonging to the second group (1-P), as a linear combination of the different expression levels (in log-space). The logistic regression output can be used as a classifier by prescribing that a case or sample will be classified into the first type if P is large, such as a usual default where P is greater than 0.5 or 50% but depending on the desired sensitivity or specificity or the diagnostic test, thresholds other than 0.5 can be considered. Alternatively, the calculated probability P can be used as a variable in other contexts, such as a 1D or 2D threshold classifier.

A statistical model is disclosed which is a binary logistic regression model, wherein MHC-I affinities for a cancer or autoimmune disease-associated mutations are evaluated as independent variables. A statistical model is disclosed which is an additive logistic regression model correlating affinity of a subject's MHC-I allele for a peptide encompassing an oncogenic mutation and the probability of mutations occurring across subjects "across-subject model". A statistical model is disclosed which is a random effects logistic regression model that follows a model equation:

$$\text{logit}\,(P(y_{ij} = 1 \mid x_{ij})) = \beta_j + \gamma \log(x_{ij}) \qquad (3),$$

wherein $y_{ij}$ is a binary mutation matrix $y_{ij} \in \{0,1\}$ indicating whether a subject $i$ has a mutation $j$; $x_{ij}$ is a binary mutation matrix indicating predicted MHC-I binding affinity of subject i having mutation $j$; $\gamma$ measures the effect of the log-affinities on the mutation probability; and $\beta_j \sim N(0, \phi_\beta)$ are random effects capturing mutation specific effects (e.g., different occurrence frequencies among mutations).

The statistical model of the invention is a mixed-effects logistic regression model that follows a model equation:

$$\text{logit}\,(P(y_{ij} = 1 \mid x_{ij})) = \eta_j + \gamma \log(x_{ij}) \qquad (1),$$

wherein $y_{ij}$ is a binary mutation matrix $y_{ij} \in \{0,1\}$ indicating whether a subject $i$ has a mutation $j$; $x_{ij}$ is a binary mutation matrix indicating predicted MHC-I binding affinity of subject $i$ having mutation $j$; $\gamma$ measures the effect of the log-affinities on the mutation probability; and $\eta_j \sim N(0, \phi_\eta)$ are random effects capturing different mutation rates among subjects, wherein the model tests the null hypothesis that $\gamma = 0$ and calculates odds ratios for MHC-I affinity of a mutation and presence of a cancer or autoimmune disease.

This model correlates the affinity of a subject's MHC-I allele for a peptide encompassing an oncogenic mutation and the probability of mutations occurring within subjects "within-subject model." In other words, the model is testing whether the affinity of a subject's MHC-I allele for a particular oncogenic mutation has any impact on probability this mutation occurring within a subject, or which mutation a subject is more likely to undergo.

In some embodiments, the predicted MHC-I affinity for a given mutation (represented in the above equations with the term $x_{ij}$) is obtained by aggregating MHC-I binding affinities of a set comprising one or more mutant cancer-associated peptides or a set comprising one or more autoimmune disorder-associated peptides by referring to a pre-determined dataset of peptides binding to MHC-I molecules encoded by at least 16 different HLA alleles. In some embodiments, the predicted MHC-I affinity is obtained by aggregating MHC-I binding affinities of a set comprising one or more mutant cancer-associated peptides or a set comprising one or more autoimmune-associated peptides by referring to a pre-determined dataset of peptides binding to MHC-I molecules encoded by at least six common HLA alleles. In some embodiments, the predicted MHC-I affinity is the simple sum of six values of the MHC-I binding affinities for six common HLA alleles. In some embodiments, the predicted MHC-I affinity is the sum of the inverse of the six values of the MHC-I binding affinities for six common HLA alleles. In some embodiments, the predicted MHC-I affinity is the inverse of sum of the inverse of the six values of the MHC-I binding affinities for six common HLA alleles. In some embodiments, MHC-I affinity is a Subject Harmonic-mean Best Rank (PHBR) score, which is the harmonic mean of the six common HLA alleles.

In some embodiments, the predicted MHC-I affinity (such as the PHBR score) is determined for a peptide encompassing a driver mutation. In some embodiments, the peptide used to obtain a predicted MHC-I affinity (such as the PHBR score) is 6 amino acids long, and the driver mutation position is located at or near the center of the peptide. In some embodiments, the peptide used to obtain a predicted MHC-I affinity (such as the PHBR score) is 7 amino acids long, and the driver mutation position is located at or near the center of the peptide. In some embodiments, the peptide used to obtain a predicted MHC-I affinity (such as the PHBR score) is 8 amino acids long, and the driver mutation position is located at or near the center of the peptide. In some embodiments, the peptide used to obtain a predicted MHC-I affinity (such as the PHBR score) is 9 amino acids long, and the driver mutation position is located at or near the center of the peptide. In some embodiments, the peptide used to obtain a predicted MHC-I affinity (such as the PHBR score) is 10 amino acids long, and the driver mutation position is located at or near the center of the peptide. In some embodiments, the peptide used to obtain a predicted MHC-I affinity (such as the PHBR score) is 11 amino acids long, and the driver mutation position is located at or near the center of the peptide. In some embodiments, the peptide used to obtain a predicted MHC-I affinity (such as the PHBR score) is 12 amino acids long, and the driver mutation position is located at or near the center of the peptide. In some embodiments, the peptide used to obtain a predicted MHC-I affinity (such as the PHBR score) is 13 amino acids long, and the driver mutation position is located at or near the center of the peptide.

In some embodiments, the predicted MHC-I affinity (such as the PHBR score) represents an aggregate of MHC-I binding affinities of all 6-amino acid-long peptides encompassing a driver mutation, wherein the driver mutation is located at any position along the peptide. In some embodiments, the predicted MHC-I affinity (such as the PHBR score) represents an aggregate of MHC-I binding affinities of all 7-amino acid-long peptides encompassing a driver mutation, wherein the driver mutation is located at any position along the peptide. In some embodiments, the predicted MHC-I affinity (such as the PHBR score) represents an aggregate of MHC-I binding affinities of all 8-amino acid-long peptides encompassing a driver mutation, wherein the driver mutation is located at any position along the peptide. In some embodiments, the predicted MHC-I affinity (such as the PHBR score) represents an aggregate of MHC-I binding affinities of all 9-amino acid-long peptides encompassing a driver mutation, wherein the driver mutation is located at any position along the peptide. In some embodiments, the predicted MHC-I affinity (such as the PHBR score) represents an aggregate of MHC-I binding affinities of all 10 amino acid-long peptides encompassing a driver mutation, wherein the driver mutation is located at any position along the peptide. In some embodiments, the predicted MHC-I affinity (such as the PHBR score) represents an aggregate of MHC-I binding affinities of all 11-amino acid-long peptides encompassing a driver mutation, wherein the driver mutation is located at any position along the peptide. In some embodiments, the predicted MHC-I affinity (such as the PHBR score) represents an aggregate of MHC-I binding affinities of all 12-amino acid-long peptides encompassing a driver mutation, wherein the driver mutation is located at any position along the peptide. In some embodiments, the predicted MHC-I affinity (such as the PHBR score) represents an aggregate of MHC-I binding affinities of all 13-amino acid-long peptides encompassing a driver mutation, wherein the driver mutation is located at any position along the peptide.

In some embodiments, the predicted MHC-I affinity (such as the PHBR score) represents a combination of aggregate MHC-I binding affinity scores of all 6- and 7-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 7- and 8-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 8- and 9-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 9- and 10-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 10- and 11-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 11- and 12-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 12-and 13-amino acid peptides encompassing a driver mutation, wherein the driver mutation is located at any position along the peptide. In some embodiments, the predicted MHC-I affinity (such as the PHBR score) ore represents a combination of aggregate MHC-I binding affinity scores of any two length-determined sets of peptides encompassing a driver mutation, wherein the driver mutation is located at any position along the peptide, and wherein each set comprises equal length 6- to 13-amino acids long peptides.

In some embodiments, the predicted MHC-I affinity (such as the PHBR score) represents a combination of aggregate MHC-I binding affinity scores of all 6-, 7-, and 8-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 7-, 8-, and 9-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 8-, 9-, and 10-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 9-, 10-, and 11-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 10-, 11-, and 12-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 11-, 12-, and 13-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of any three length-determined sets of peptides encompassing a driver mutation, wherein the driver mutation is located at any position along the peptide, and wherein each set comprises equal length 6-to 13-amino acids long peptides.

In some embodiments, the predicted MHC-I affinity (such as the PHBR score) represents a combination of aggregate MHC-I binding affinity scores of all 6-, 7-, 8- and 9-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 7-, 8-9-, and 10-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 8-, 9-,10-, and 11- amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 9-, 10- 11-, and 12-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of all 10- 11-, 12-, and 13-amino acid peptides encompassing a driver mutation, wherein the driver mutation is located at any position along the peptide. In some embodiments, the predicted MHC-I affinity (such as the PHBR score) represents a combination of aggregate MHC-I binding affinity scores of any four length-determined sets of peptides encompassing a driver mutation, wherein the driver mutation is located at any position along the peptide, and wherein each set comprises equal length 6-to 13-amino acids long peptides. In some embodiments, the predicted MHC-I affinity (such as the PHBR score) represents a combination of aggregate MHC-I binding affinity scores of any five length-determined sets of peptides encompassing a driver mutation, wherein the driver mutation is located at any position along the peptide, and wherein each set comprises equal length 6-to 13- amino acids long peptides. In some embodiments, the predicted MHC-I affinity (such as the PHBR score) represents a combination of aggregate MHC-I binding affinity scores of any six length-determined sets of peptides encompassing a driver mutation, wherein the driver mutation is located at any position along the peptide, and wherein each set comprises equal length 6-to 13-amino acids long peptides. In some embodiments, the predicted MHC-I affinity (such as the PHBR score) represents a combination of aggregate MHC-I binding affinity scores of all 6-, 7-, 8-, 9-, 10-, 11, 12-, and 13-amino acids long encompassing a driver mutation, wherein the driver mutation is located at any position along the peptide.

In some embodiments, the predicted MHC-I affinity (such as the PHBR score) is obtained using wild type peptide sequences. In some embodiments, the predicted MHC-I affinity (such as the PHBR score) is obtained using peptide sequences containing a driver mutation. In some embodiments, the predicted MHC-I affinity (such as the PHBR score) is obtained using peptides containing wild-type sequences and a driver mutation.

The individual peptides' the predicted MHC-I affinities can be combined in several ways. In some embodiments, the predicted MHC-I affinities are combined through assigning the best rank among the peptides in a set. In some embodiments, predicted MHC-I affinities are combined through calculating the number of peptides having MHC-I affinity below a certain threshold (e.g., <2 for MHC-I binders and <0.5 for MHC-I strong binders). In some embodiments, predicted MHC-I affinities are combined through assigning the best rank weighted by predicted proteasomal cleavage. In some embodiments, predicted MHC-I affinities are combined by referring to a pre-determined dataset of peptides binding to MHC-I molecules encoded by at least 16 different HLA alleles. In some embodiments, predicted MHC-I affinities are combined by referring to a pre-determined dataset of peptides binding to MHC-I molecules encoded by at least 6 common HLA alleles.

In some embodiments, the mixed-effects logistic regression model following the model equation (1) can be used to evaluate a subject's risk of developing or having a pre-detection stage of many types cancer. As used herein, the term "cancer" refers to refers to a cellular disorder characterized by uncontrolled or disregulated cell proliferation, decreased cellular differentiation, inappropriate ability to invade surrounding tissue, and/or ability to establish new growth at ectopic sites. The term "cancer" further encompasses primary and metastatic cancers. Specific examples of cancers include, but are not limited to, Acute Lymphoblastic Leukemia, Adult; Acute Lymphoblastic Leukemia, Childhood; Acute Myeloid Leukemia, Adult; Adrenocortical Carcinoma; Adrenocortical Carcinoma, Childhood; AIDS-Related Lymphoma; AIDS-Related Malignancies; Anal Cancer; Astrocytoma, Childhood Cerebellar; Astrocytoma, Childhood Cerebral; Bile Duct Cancer, Extrahepatic; Bladder Cancer; Bladder Cancer, Childhood; Bone Cancer, Osteosarcoma/Malignant Fibrous Histiocytoma; Brain Stem Glioma, Childhood; Brain Tumor, Adult; Brain Tumor, Brain Stem Glioma, Childhood; Brain Tumor, Cerebellar Astrocytoma, Childhood; Brain Tumor, Cerebral Astrocytoma/Malignant Glioma, Childhood; Brain Tumor, Ependymoma, Childhood; Brain Tumor, Medulloblastoma, Childhood; Brain Tumor, Supratentorial Primitive Neuroectodermal Tumors, Childhood; Brain Tumor, Visual Pathway and Hypothalamic Glioma, Childhood; Brain Tumor, Childhood (Other); Breast Cancer; Breast Cancer and Pregnancy; Breast Cancer, Childhood; Breast Cancer, Male; Bronchial Adenomas/Carcinoids, Childhood: Carcinoid Tumor, Childhood; Carcinoid Tumor, Gastrointestinal; Carcinoma, Adrenocortical; Carcinoma, Islet Cell; Carcinoma of Unknown Primary; Central Nervous System Lymphoma, Primary; Cerebellar Astrocytoma, Childhood; Cerebral Astrocytoma/Malignant Glioma, Childhood; Cervical Cancer; Childhood Cancers; Chronic Lymphocytic Leukemia; Chronic Myelogenous Leukemia; Chronic Myeloproliferative Disorders; Clear Cell Sarcoma of Tendon Sheaths; Colon Cancer; Colorectal Cancer, Childhood; Cutaneous T-Cell Lymphoma; Endometrial Cancer; Ependymoma, Childhood; Epithelial Cancer, Ovarian; Esophageal Cancer; Esophageal Cancer, Childhood; Ewing's Family of Tumors; Extracranial Germ Cell Tumor, Childhood; Extragonadal Germ Cell Tumor; Extrahepatic Bile Duct Cancer; Eye Cancer, Intraocular Melanoma; Eye Cancer, Retinoblastoma; Gallbladder Cancer; Gastric (Stomach) Cancer; Gastric (Stomach) Cancer, Childhood; Gastrointestinal Carcinoid Tumor; Germ Cell Tumor, Extracranial, Childhood; Germ Cell Tumor, Extragonadal; Germ Cell Tumor, Ovarian; Gestational Trophoblastic Tumor; Glioma. Childhood Brain Stem; Glioma. Childhood Visual Pathway and Hypothalamic; Hairy Cell Leukemia; Head and Neck Cancer; Hepatocellular (Liver) Cancer, Adult (Primary); Hepatocellular (Liver) Cancer, Childhood (Primary); Hodgkin's Lymphoma, Adult; Hodgkin's Lymphoma, Childhood; Hodgkin's Lymphoma During Pregnancy; Hypopharyngeal Cancer; Hypothalamic and Visual Pathway Glioma, Childhood; Intraocular Melanoma; Islet Cell Carcinoma (Endocrine Pancreas); Kaposi's Sarcoma; Kidney Cancer; Laryngeal Cancer; Laryngeal Cancer, Childhood; Leukemia, Acute Lymphoblastic, Adult; Leukemia, Acute Lymphoblastic, Childhood; Leukemia, Acute Myeloid, Adult; Leukemia, Acute Myeloid, Childhood; Leukemia, Chronic Lymphocytic; Leukemia, Chronic Myelogenous; Leukemia, Hairy Cell; Lip and Oral Cavity Cancer; Liver Cancer, Adult (Primary); Liver Cancer, Childhood (Primary); Lung Cancer, Non-Small Cell; Lung Cancer, Small Cell; Lymphoblastic Leukemia, Adult Acute; Lymphoblastic Leukemia, Childhood Acute; Lymphocytic Leukemia, Chronic; Lymphoma, AIDS-Related; Lymphoma, Central Nervous System (Primary); Lymphoma, Cutaneous T-Cell; Lymphoma, Non-Hodgkin's, Adult; Lymphoma, Non-Hodgkin's, Childhood; Lymphoma, Non-Hodgkin's During Pregnancy; Lymphoma, Primary Central Nervous System; Macroglobulinemia, Waldenstrom's; Male Breast Cancer; Malignant Mesothelioma, Adult; Malignant Mesothelioma, Childhood; Malignant Thymoma; Medulloblastoma, Childhood; Melanoma; Melanoma, Intraocular; Merkel Cell Carcinoma; Mesothelioma, Malignant; Metastatic Squamous Neck Cancer with Occult Primary; Multiple Endocrine Neoplasia Syndrome, Childhood; Multiple Myeloma/Plasma Cell Neoplasm; Mycosis Fungoides; Myelodysplasia Syndromes; Myelogenous Leukemia, Chronic; Myeloid Leukemia, Childhood Acute; Myeloma, Multiple; Myeloproliferative Disorders, Chronic; Nasal Cavity and Paranasal Sinus Cancer; Nasopharyngeal Cancer; Nasopharyngeal Cancer, Childhood; Neuroblastoma; Neurofibroma; Non-Hodgkin's Lymphoma, Adult; Non-Hodgkin's Lymphoma, Childhood; Non-Hodgkin's Lymphoma During Pregnancy; Non-Small Cell Lung Cancer; Oral Cancer, Childhood; Oral Cavity and Lip Cancer; Oropharyngeal Cancer; Osteosarcoma/Malignant Fibrous Histiocytoma of Bone; Ovarian Cancer, Childhood; Ovarian Epithelial Cancer; Ovarian Germ Cell Tumor; Ovarian Low Malignant Potential Tumor; Pancreatic Cancer; Pancreatic Cancer, Childhood, Pancreatic Cancer, Islet Cell; Paranasal Sinus and Nasal Cavity Cancer; Parathyroid Cancer; Penile Cancer; Pheochromocytoma; Pineal and Supratentorial Primitive Neuroectodermal Tumors, Childhood; Pituitary Tumor; Plasma Cell Neoplasm/Multiple Myeloma; Pleuropulmonary Blastoma; Pregnancy and Breast Cancer; Pregnancy and Hodgkin's Lymphoma; Pregnancy and Non-Hodgkin's Lymphoma; Primary Central Nervous System Lymphoma; Primary Liver Cancer, Adult; Primary Liver Cancer, Childhood; Prostate Cancer; Rectal Cancer; Renal Cell (Kidney) Cancer; Renal Cell Cancer, Childhood; Renal Pelvis and Ureter, Transitional Cell Cancer; Retinoblastoma; Rhabdomyosarcoma, Childhood; Salivary Gland Cancer; Salivary Gland Cancer, Childhood; Sarcoma, Ewing's Family of Tumors; Sarcoma, Kaposi's; Sarcoma (Osteosarcoma)/Malignant Fibrous Histiocytoma of Bone; Sarcoma, Rhabdomyosarcoma, Childhood; Sarcoma, Soft Tissue, Adult; Sarcoma, Soft Tissue, Childhood; Sezary Syndrome; Skin Cancer; Skin Cancer, Childhood; Skin Cancer (Melanoma); Skin Carcinoma, Merkel Cell; Small Cell Lung Cancer; Small Intestine Cancer; Soft Tissue Sarcoma, Adult; Soft Tissue Sarcoma, Childhood; Squamous Neck Cancer with Occult Primary, Metastatic; Stomach (Gastric) Cancer; Stomach (Gastric) Cancer, Childhood; Supratentorial Primitive Neuroectodermal Tumors, Childhood; T-Cell Lymphoma, Cutaneous; Testicular Cancer; Thymoma, Childhood; Thymoma, Malignant; Thyroid Cancer; Thyroid Cancer, Childhood; Transitional Cell Cancer of the Renal Pelvis and Ureter; Trophoblastic Tumor, Gestational; Unknown Primary Site, Cancer of, Childhood; Unusual Cancers of Childhood; Ureter and Renal Pelvis, Transitional Cell Cancer; Urethral Cancer; Uterine Sarcoma; Vaginal Cancer; Visual Pathway and Hypothalamic Glioma, Childhood; Vulvar Cancer; Waldenstrom's Macro globulinemia; and Wilms' Tumor. Many additional types of cancer are known in the art. As used herein, cancer cells, including tumor cells, refer to cells that divide at an abnormal (increased) rate or whose control of growth or survival is different than for cells in the same tissue where the cancer cell arises or lives. Cancer cells include, but are not limited to, cells in carcinomas, such as squamous cell carcinoma, basal cell carcinoma, sweat gland carcinoma, sebaceous gland carcinoma, adenocarcinoma, papillary carcinoma, papillary adenocarcinoma, cystadenocarcinoma, medullary carcinoma, undifferentiated carcinoma, bronchogenic carcinoma, melanoma, renal cell carcinoma, hepatoma-liver cell carcinoma, bile duct carcinoma, cholangiocarcinoma, papillary carcinoma, transitional cell carcinoma, choriocarcinoma, semonoma, embryonal carcinoma, mammary carcinomas, gastrointestinal carcinoma, colonic carcinomas, bladder carcinoma, prostate carcinoma, and squamous cell carcinoma of the neck and head region; sarcomas, such as fibrosarcoma, myxosarcoma, liposarcoma, chondrosarcoma, osteogenic sarcoma, chordosarcoma, angiosarcoma, endotheliosarcoma, lymphangiosarcoma, synoviosarcoma and mesotheliosarcoma; hematologic cancers, such as myelomas, leukemias (e.g., acute myelogenous leukemia, chronic lymphocytic leukemia, granulocytic leukemia, monocytic leukemia, lymphocytic leukemia), and lymphomas (e.g., follicular lymphoma, mantle cell lymphoma, diffuse large cell lymphoma, malignant lymphoma, plasmocytoma, reticulum cell sarcoma, or Hodgkin's disease); and tumors of the nervous system including glioma, meningioma, medulloblastoma, schwannoma, or epidymoma.

In some embodiments, mixed-effects logistic regression model following the model equation (1) can be used to evaluate a subject's risk of developing or having a pre-detection stage of an adrenocortical carcinoma (ACC), a bladder urothelial carcinoma (BLCA), a breast invasive carcinoma (BRCA), a cervical squamous cell carcinoma and endocervical adenocarcinoma (CESC), a colon adenocarcinoma (COAD), a lymphoid neoplasm diffuse large B-cell lymphoma (DLBC), a glioblastoma multiforme (GBM), a head and neck squamous cell carcinoma (HNSC), a kidney chromophobe (KICH), a kidney renal clear cell carcinoma (KIRC), a kidney renal papillary cell carcinoma (KIRP), an acute myeloid leukemia (LAML), a brain lower grade glioma (LGG), a liver hepatocellular carcinoma (LIHC), a lung adenocarcinoma (LUAD), lung squamous cell carcinoma (LUSC), a mesothelioma (MESO), an ovarian serous cystadenocarcinoma (OV), a pancreatic adenocarcinoma (PAAD), a pheochromocytoma and paraganglioma (PCPG), a prostate adenocarcinoma (PRAD), a rectum adenocarcinoma (READ), a sarcoma (SARC), a skin cutaneous melanoma (SKCM), a stomach adenocarcinoma (STAD), a testicular germ cell tumors (TGCT), a thyroid carcinoma (THCA), a uterine corpus endometrial carcinoma (UCEC), a uterine carcinosarcoma (UCS), or a uveal melanoma (UVM).

The mixed-effects logistic regression model following the model equation (1) can be also used to evaluate a subject's risk of developing or having a pre-detection stage of an autoimmune disease. As used herein, the term "autoimmune disease" refers to disorders wherein the subjects own immune system mistakenly attacks itself, thereby targeting the cells, tissues, and/or organs of the subjects own body, for example through MHC-I- mediated presentation of subject's proteins (see e.g., Matzaraki et al., Genome Biol., 2017, 18, 76). For example, the autoimmune reaction is directed against the nervous system in multiple sclerosis and the gut in Crohn's disease, in other autoimmune disorders such as systemic lupus erythematosus (lupus), affected tissues and organs may vary among individuals with the same disease. One person with lupus may have affected skin and joints whereas another may have affected skin, kidney, and lungs. Ultimately, damage to certain tissues by the immune system may be permanent, as with destruction of insulin-producing cells of the pancreas in Type 1 diabetes mellitus. Specific autoimmune disorders whose risk can be assessed using methods of this disclosure include without limitation, autoimmune disorders of the nervous system (e.g., multiple sclerosis, myasthenia gravis, autoimmune neuropathies such as Guillain-Barre, and autoimmune uveitis), autoimmune disorders of the blood (e.g., autoimmune hemolytic anemia, pernicious anemia, and autoimmune thrombocytopenia), autoimmune disorders of the blood vessels (e.g., temporal arteritis, anti-phospholipid syndrome, vasculitides such as Wegener's granulomatosis, and Bechet's disease), autoimmune disorders of the skin (e.g., psoriasis, dermatitis herpetiformis, pemphigus vulgaris, and vitiligo), autoimmune disorders of the gastrointestinal system (e.g., Crohn's disease, ulcerative colitis, primary biliary cirrhosis, and autoimmune hepatitis), autoimmune disorders of the endocrine glands (e.g., Type 1 or immune-mediated diabetes mellitus, Grave's disease, Hashimoto's thyroiditis, autoimmune oophoritis and orchitis, and autoimmune disorder of the adrenal gland); and autoimmune disorders of multiple organs (including connective tissue and musculoskeletal system diseases) (e.g., rheumatoid arthritis, systemic lupus erythematosus, scleroderma, polymyositis, dennatomyositis, spondyloarthropathies such as ankylosing spondylitis, and Sjogren's syndrome). In addition, other immune system mediated diseases, such as graft-versus-host disease and allergic disorders, are also included in the definition of immune disorders herein.

The present disclosure also provides computing systems for determining whether a subject is at risk of having or developing a cancer or an autoimmune disease, the system comprising: a) a communication system for using a library of cancer-associated peptides or autoimmune-associated peptides derived from subjects; and b) a processor for scoring the ability of the subject's major histocompatibility complex class I (MHC-I) to present a mutant cancer-associated peptide or an autoimmune-associated peptide based upon a library of cancer-associated peptides or autoimmune-associated peptides derived from subjects, wherein the produced score is the MHC-I presentation score.

Using the mixed-effects logistic regression model following the model equation (1) it has been surprisingly and unexpectedly found that oncogenic mutations associated with one cancer type are predictive of other cancer types. Thus, for example, the 10 residues highly mutated in a breast invasive carcinoma (BRCA), specifically, PIK3CA_H1047R, PIK3CA_E545K, PIK3CA_E542K, TP53_R175H, PIK3CA_N345K, AKT1_E17K, SF3B1_K700E, PIK3CA_H1047L, TP53_R273H, and TP53_Y220C, are predictive (odds ratio >1.2, p value ≤0.05) of a colon adenocarcinoma (COAD), a head and neck squamous cell carcinoma (HNSC), a glioblastoma multiforme (GBM), a brain lower grade glioma (LGG), an ovarian serous cystadenocarcinoma (OV), a pancreatic adenocarcinoma (PAAD), a stomach adenocarcinoma (STAD), and a uterine carcinosarcoma (UCS). At the same time, surprisingly and unexpectedly, the set of BRCA-associated mutations was not predictive of BRCA (see, Example 4 and Tables 12-23).

The present disclosure also provides methods of detecting a cancer, such as an early stage cancer, in a subject, the method comprising the steps of: a) obtaining a biological sample from the subject; b) assaying the sample for the presence of a cancer-associated mutation, c) genotyping the HLA locus of the subject; and d) scoring the likelihood of the MHC-I-mediated presentation of the mutations found in step (b) by the subject's MHC-I allele as determined in step (c), wherein the poor presentation score indicates the presence of cancer, such as early stage cancer, in the subject.

The present disclosure also provides methods of detecting an autoimmune disease, such as an early stage autoimmune disease, in a subject, the method comprising the steps of: a) obtaining a biological sample from the subject; b) assaying the sample for the presence of an autoimmune-associated peptide, c) genotyping the HLA locus of the subject; and d) scoring the likelihood of the MHC-I-mediated presentation of the autoimmune-associated peptides found in step (b) by the subject's MHC-I allele as determined in step (c), wherein the poor presentation score indicates the presence of an autoimmune disease, such as an early stage autoimmune disease, in the subject.

As used herein, "biological sample" refers to any sample that can be from or derived from a human subject, e.g., bodily fluids (blood, saliva, urine etc.), biopsy, tissue, and/or waste from the subject. Thus, tissue biopsies, stool, sputum, saliva, blood, lymph, tears, sweat, urine, vaginal secretions, or the like can be screened for the presence of one or more specific mutations, as can essentially any tissue of interest that contains the appropriate nucleic acids. These samples are typically taken, following informed consent, from a subject by standard medical laboratory methods. The sample may be in a form taken directly from the subject, or may be at least partially processed (purified) to remove at least some non-nucleic acid material.

In some embodiments, the cancer is a breast invasive carcinoma (BRCA), and the corresponding predictive mutations comprise one or more of B-Raf Proto-Oncogene (BRAF) V600E mutation, Phosphatidylinositol-4,5-Bisphosphate 3-Kinase Catalytic Subunit Alpha (PIK3CA) E545K mutation, PIK3CA E542K mutation, PIK3CA H1047R mutation, Kirsten Rat Sarcoma Viral Oncogene Homolog (KRAS) G12D mutation, KRAS G13D mutation, KRAS G12V mutation, KRAS A146T mutation, TP53 R175H mutation, TP53 H179R mutation, TP53 mutation, TP53 R248Q mutation, TP53 R273C mutation, TP53 R273H mutation, TP53 R282W mutation, Keratin Associated Protein 4-11 (KRTAP4-11) L161V mutation, Mab-21 Domain Containing 2 (MB21D2) Q311E, mutation, HLA-A Q78R mutation, Harvey Rat Sarcoma Viral Oncogene Homolog (HRAS) G13V mutation, Isocitrate Dehydrogenase (NADP(+)) 1 (IDH1) R132H mutation, IDH1 R132C mutation, IDH1 R132G mutation, IDH2 R172K mutation, IDH1 R132S mutation, Capicua Transcriptional Repressor (CIC) R215W mutation, Phosphoglucomutase 5 (PGM5) I98V mutation, Tripartite Motif Containing 48 (TRIM48) Y192H mutation, or F-Box And WD Repeat Domain Containing 7 (FBXW7) R465C mutation, wherein the presence of any one of these mutations indicates the presence of breast invasive carcinoma.

In some embodiments, the cancer is a colon adenocarcinoma (COAD) and the corresponding predictive mutations comprise one or more of BRAF V600E mutation, Neuroblastoma RAS Viral Oncogene Homolog (NRAS) Q61R mutation, NRAS Q61K mutation, NRAS Q61L mutation, IDH1 R132S mutation, Mitogen-Activated Protein Kinase Kinase 1 (MAP2K1) P124S mutation, Rac Family Small GTPase 1 (RAC1) P29S mutation, Protein Phosphatase 6 Catalytic Subunit (PPP6C) R301C mutation, Cyclin Dependent Kinase Inhibitor 2A (CDKN2A) P114L mutation, Keratin Associated Protein 4-11 (KRTAP4-11) L161V mutation, KRTAP4-11 M93V mutation, HRAS Q61R mutation, HLA-A Q78R mutation, Zinc Finger Protein 799 (ZNF799) E589G mutation, Zinc Finger Protein 844 (ZNF844) R447P mutation, or RNA Binding Motif Protein 10 (RBM10) E184D mutation, wherein the presence of any one of these mutations indicates the presence of colon adenocarcinoma.

In some embodiments, the cancer is a head and neck squamous cell carcinoma (HNSC) and the corresponding predictive mutations comprise one or more of IDH1 R132H mutation, IDH1 R132C mutation, IDH1 R132G mutation, IDH1 R132S mutation, IDH2 R172K mutation, TP53 H179R mutation, TP53 R273C mutation, TP53 R273H mutation, CIC R215W mutation, or HLA-A Q78R mutation, wherein the presence of any one of these mutations indicates the presence of head and neck squamous cell carcinoma.

In some embodiments, the cancer is a brain lower grade glioma (LGG) and the corresponding predictive mutations comprise one or more of IDH1 R132H mutation, IDH1 R132C mutation, IDH1 R132G mutation, IDH1 R132S mutation, IDH2 R172K mutation, TP53 H179R mutation, TP53 R273C mutation, TP53 R273H mutation, CIC R215W mutation, or HLA-A Q78R mutation, wherein the presence of any one of these mutations indicates the presence of brain lower grade glioma.

In some embodiments, the cancer is a lung adenocarcinoma (LUAD) and the corresponding predictive mutations comprise one or more of BRAF V600E mutation, PIK3CA E545K mutation, KRAS G12D mutation, KRAS G13D mutation, KRAS A146T mutation, TP53 R175H mutation, KRAS G12V mutation, TP53 R248Q mutation, TP53 R273C mutation TP53 R273H mutation, TP53 R282W mutation, PGM5 I98V mutation, TRIM48 Y192H mutation, PIK3CA E545K mutation, KRAS G13D mutation, PIK3CA H1047R mutation, or FBXW7 R465C mutation, wherein the presence of any one of these mutations indicates the presence of lung adenocarcinoma.

In some embodiments, the cancer is a lung squamous cell carcinoma (LUSC) and the corresponding predictive mutations comprise one or more of PIK3CA H1047R mutation, PIK3CA E545K mutation, PIK3CA E542K mutation, TP53 R175H mutation, PIK3CA N345K mutation, AKT Serine/Threonine Kinase 1 (AKT1) E17K mutation, Splicing Factor 3b Subunit 1 (SF3B1) K700E mutation, or PIK3CA H1047L mutation, wherein the presence of any one of these mutations indicates the presence of lung squamous cell carcinoma.

In some embodiments, the cancer is a skin cutaneous melanoma (SKCM) and the corresponding predictive mutations comprise one or more of BRAF V600E mutation, PIK3CA E545K mutation, KRAS G12D mutation, KRAS G13D mutation, KRAS A146T mutation, KRAS G12V mutation, TP53 R175H mutation, TP53 H179R mutation, TP53 R248Q mutation TP53 R273C mutation, TP53 R273H mutation, TP53 R282W mutation, IDH1 R132H mutation, IDH1 R132C mutation, IDH1 R132G mutation, IDH1 R132S mutation, IDH2 R172K mutation, CIC R215W mutation, or HLA-A Q78R mutation, NRAS Q61R mutation, NRAS Q61K mutation, NRAS Q61L mutation, MAP2K1 P124S mutation, RAC1 P29S mutation, PPP6C R301C mutation, CDKN2A P114L mutation, KRTAP4-11 L161V mutation, KRTAP4-11 M93V mutation, HRAS Q61R mutation, ZNF799 E589G mutation, ZNF844 R447P mutation, or RBM10 E184D mutation, wherein the presence of any one of these mutations indicates the presence of skin cutaneous melanoma.

In some embodiments, the cancer is a stomach adenocarcinoma (STAD) and the corresponding predictive mutations comprise one or more of KRAS G12C mutation, KRAS G12V mutation, Epidermal Growth Factor Receptor (EGFR) L858R mutation, KRAS G12D mutation, KRAS G12A mutation, U2 Small Nuclear RNA Auxiliary Factor 1 (U2AF1) S34F mutation, KRTAP4-11 L161V mutation, KRTAP4-11 R121K mutation, Eukaryotic Translation Elongation Factor 1 Beta 2 (EEF1B2) R42H mutation, or KRTAP4-11 M93V mutation, wherein the presence of any one of these mutations indicates the presence of stomach adenocarcinoma.

In some embodiments, the cancer is a thyroid carcinoma (THCA) and the corresponding predictive mutations comprise one or more of BRAF V600E mutation, PIK3CA E545K mutation, KRAS G12D mutation, KRAS G13D mutation, TP53 R175H mutation, KRAS G12V mutation, TP53 R248Q mutation, KRAS A146T mutation, TP53 R273H mutation, HRAS Q61R mutation, HLA-A Q78R mutation, TP53 R282W mutation, NRAS Q61R mutation, NRAS Q61K mutation, IDH1 R132C mutation, MAP2K1 P124S mutation, RAC1 P29S mutation, NRAS Q61L mutation, PPP6C R301C mutation, CDKN2A P114L mutation, KRTAP4-11 L161V mutation, KRTAP4-11 M93V mutation, ZNF799 E589G mutation, ZNF844 R447P mutation, or RBM10 E184D mutation, wherein the presence of any one of these mutations indicates the presence of thyroid carcinoma.

In some embodiments, the cancer is a uterine corpus endometrial carcinoma (UCEC) and the corresponding predictive mutations comprise one or more of BRAF V600E mutation, PIK3CA H1047R mutation, PIK3CA E545K mutation, PIK3CA E542K mutation, TP53 R175H mutation, PIK3CA N345K mutation, AKT Serine/Threonine Kinase 1 (AKT1) E17K mutation, Splicing Factor 3b Subunit 1 (SF3B1) K700E mutation, KRAS G12C mutation, KRAS G12V mutation, Epidermal Growth Factor Receptor (EGFR) L858R mutation, KRAS G12D mutation, KRAS G12A mutation, KRAS G12V mutation, KRAS G13D mutation, TP53 R175H mutation, TP53 R248Q mutation, KRAS A146T mutation, TP53 R273H mutation, TP53 R282W mutation, U2 Small Nuclear RNA Auxiliary Factor 1 (U2AF1) S34F mutation, KRTAP4-11 L161V mutation, KRTAP4-11 R121K mutation, Eukaryotic Translation Elongation Factor 1 Beta 2 (EEF1B2) R42H mutation, or KRTAP4-11 M93V mutation, wherein the presence of any one of these mutations indicates the presence of uterine corpus endometrial carcinoma.

In any of the embodiments described herein, the presence of any one of the mutations may indicate the presence of an early stage cancer.

The present disclosure also provides diagnostic kits comprising detection agents for one or more cancer or autoimmune disease-associated mutations. A kit may optionally further comprise a container with a predetermined amount of one or more purified molecules, either protein or nucleic acid having a cancer or autoimmune disease-associated mutation according to the present disclosure, for use as positive controls. Each kit may also include printed instructions and/or a printed label describing the methods disclosed herein in accordance with one or more of the embodiments described herein. Kit containers may optionally be sterile containers. The kits may also be configured for research use only applications whether on clinical samples, research use samples, cell lines and/or primary cells.

Suitable detection agents comprise any organic or inorganic molecule that specifically bind to or interact with proteins or nucleic acids having a cancer or autoimmune disease-associated mutation. Non-limiting examples of detection agents include proteins, peptides, antibodies, enzyme substrates, transition state analogs, cofactors, nucleotides, polynucleotides, aptamers, lectins, small molecules, ligands, inhibitors, drugs, and other biomolecules as well as non-biomolecules capable of specifically binding the analyte to be detected.

In some embodiments, the detection agents comprise one or more label moiety(ies). In embodiments employing two or more label moieties, each label moiety can be the same, or some, or all, of the label moieties may differ.

In some embodiments, the label moiety comprises a chemiluminescent label. The chemiluminescent label can comprise any entity that provides a light signal and that can be used in accordance with the methods and devices described herein. A wide variety of such chemiluminescent labels are known (see, e.g., U.S. Patent Nos. 6,689,576, 6,395,503, 6,087,188, 6,287,767, 6,165,800, and 6,126,870). Suitable labels include enzymes capable of reacting with a chemiluminescent substrate in such a way that photon emission by chemiluminescence is induced. Such enzymes induce chemiluminescence in other molecules through enzymatic activity. Such enzymes may include peroxidase, beta-galactosidase, phosphatase, or others for which a chemiluminescent substrate is available. In some embodiments, the chemiluminescent label can be selected from any of a variety of classes of luminol label, an isoluminol label, etc. In some embodiments, the detection agents comprise chemiluminescent labeled antibodies.

Likewise, the label moiety can comprise a bioluminescent compound. Bioluminescence is a type of chemiluminescence found in biological systems in which a catalytic protein increases the efficiency of the chemiluminescent reaction. The presence of a bioluminescent compound is determined by detecting the presence of luminescence. Suitable bioluminescent compounds include, but are not limited to luciferin, luciferase, and aequorin.

In some embodiments, the label moiety comprises a fluorescent dye. The fluorescent dye can comprise any entity that provides a fluorescent signal and that can be used in accordance with the methods and devices described herein. Typically, the fluorescent dye comprises a resonance-delocalized system or aromatic ring system that absorbs light at a first wavelength and emits fluorescent light at a second wavelength in response to the absorption event. A wide variety of such fluorescent dye molecules are known in the art. For example, fluorescent dyes can be selected from any of a variety of classes of fluorescent compounds, non-limiting examples include xanthenes, rhodamines, fluoresceins, cyanines, phthalocyanines, squaraines, bodipy dyes, coumarins, oxazines, and carbopyronines. In some embodiments, for example, where detection agents contain fluorophores, such as fluorescent dyes, their fluorescence is detected by exciting them with an appropriate light source, and monitoring their fluorescence by a detector sensitive to their characteristic fluorescence emission wavelength. In some embodiments, the detection agents comprise fluorescent dye labeled antibodies.

In embodiments using two or more different detection agents, which bind to or interact with different analytes, different types of analytes can be detected simultaneously. In some embodiments, two or more different detection agents, which bind to or interact with the one analyte, can be detected simultaneously. In embodiments using two or more different detection agents, one detection agent, for example a primary antibody, can bind to or interact with one or more analytes to form a detection agent-analyte complex, and second detection agent, for example a secondary antibody, can be used to bind to or interact with the detection agent-analyte complex.

In some embodiments, two different detection agents, for example antibodies for both phospho and non-phospho forms of analyte of interest can enable detection of both forms of the analyte of interest. In some embodiments, a single specific detection agent, for example an antibody, can allow detection and analysis of both phosphorylated and non-phosphorylated forms of a analyte, as these can be resolved in the fluid path. In some embodiments, multiple detection agents can be used with multiple substrates to provide color-multiplexing. For example, the different chemiluminescent substrates used would be selected such that they emit photons of differing color. Selective detection of different colors, as accomplished by using a diffraction grating, prism, series of colored filters, or other means allow determination of which color photons are being emitted at any position along the fluid path, and therefore determination of which detection agents are present at each emitting location. In some embodiments, different chemiluminescent reagents can be supplied sequentially, allowing different bound detection agents to be detected sequentially.

Throughout the specification the word "comprising," or variations such as "comprises" or "comprising," will be understood to imply the inclusion of a stated element, integer or step, or group of elements, integers or steps, but not the exclusion of any other element, integer or step, or group of elements, integers or steps. The methods, systems, and kits described herein may suitably "comprise", "consist of", or "consist essentially of", the steps, elements, and/or reagents recited herein.

In order that the subject matter disclosed herein may be more efficiently understood, examples are provided below. It should be understood that these examples are for illustrative purposes only and are not to be construed as limiting the claimed subject matter in any manner.

## Examples

### Example 1: MHC-I Affinity-Based Scoring Scheme for Mutated Residues

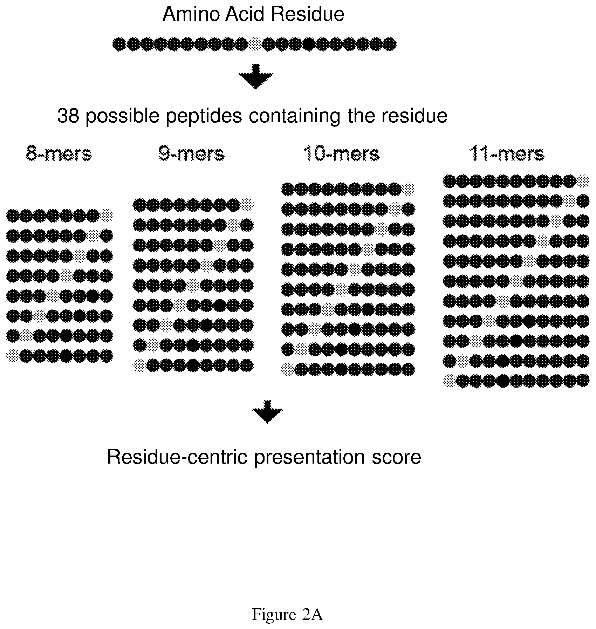

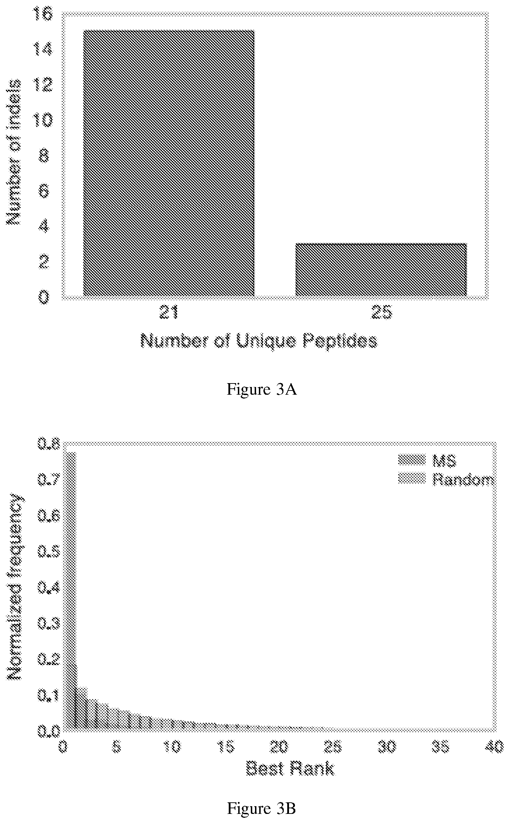

To study the influence of MHC-I genotype in shaping the genomes of tumors, a qualitative residue-centric presentation score was developed, and its potential to predict whether a sequence containing a residue will be presented on the cell surface was evaluated. The score relies on aggregating MHC-I binding affinities across possible peptides that include the residue of interest. MHC-I peptide binding affinity predictions were obtained using the NetMHCPan3.0 tool (Vita et al., Nucleic Acids Res., 2015, 43, D405-D412), and following published recommendations (Nielsen and Andreatta, Genome Med., 2016, 8, 33), peptides receiving a rank threshold <2 and <0.5 were designated MHC-I binders and strong binders respectively. For evaluation of missense mutations, the score was based on the affinities of all 38 possible peptides of length 8-11 that incorporate the amino acid position of interest (Figure 2A), while for insertions and deletions, any resulting novel peptides of length 8-11 were considered (Figure 3A).

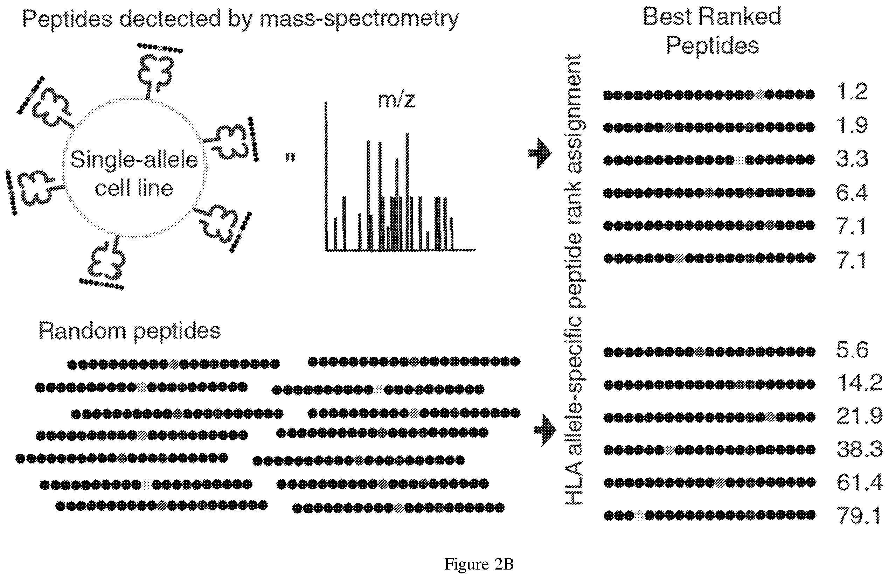

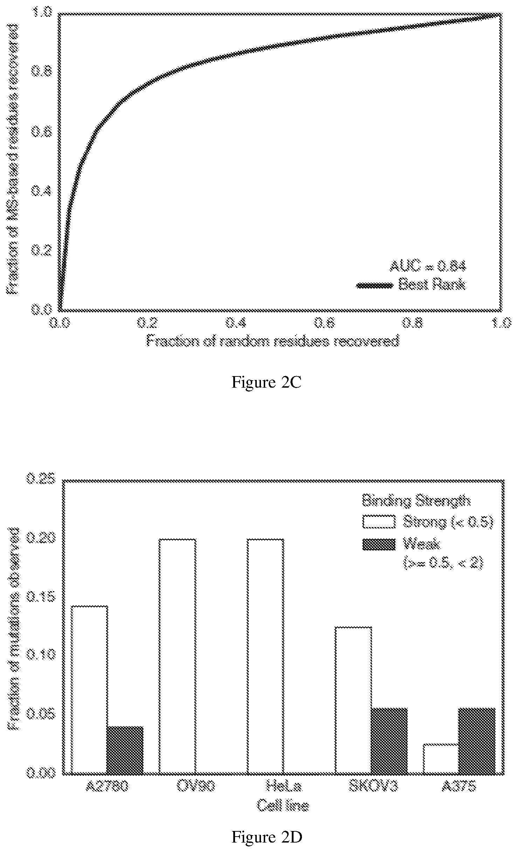

Several strategies were evaluated for combining peptide affinities to approximate presentation of a specific residue on the cell surface using an existing dataset of peptides bound to MHC-I molecules encoded by 16 different HLA alleles in monoallelic lymphoblastoid cell lines determined using mass spectrometry (MS) (Abelin et al., Mass Immunity, 2017, 46, 315-326), the most comprehensive database of cell surface presented peptides currently available. These strategies included assigning the best rank among peptides, the total number of peptides with rank <2, the total number of peptides with rank <0.5, and the best rank weighted by predicted proteasomal cleavage (Figures 3B-3K). The ability of these scores to discriminate these MS-derived residues from a size-matched set of randomly selected residues (STAR Methods) were compared. The best rank score (Figure 2B) provided the most reliable prediction that a particular residue position would be included in a sequence presented by the MHC-I on the cell surface (Figure 2C); thus, this score was used for all subsequent analysis.

To test the best rank score's ability to assess the presentation of cancer-related mutations, sets of expressed mutations in 5 cancer cell lines (A375, A2780, OV90, HeLa, and SKOV3) were scored to predict which would be presented by an HLA-A*02:01-derived MHC-I (see, Tables 1A and 1B for A375; Tables 2A and 2B for A2780; Tables 3A and 3B for OV90; Tables 4A and 4B for HeLa; and Tables 5A and 5B for SKOV3). Unless a mutation affects an anchor position, a peptide harboring a single amino acid change has a modest impact on peptide binding affinity and should be presented on the cell surface provided that the corresponding native sequence is presented.

Table 1A: A375 Peptide Panel

| Peptide # | A375 (High) | Allele | | Rank |
|---|---|---|---|---|
| 1 | PLEC_A398T | HLA-A*02:01 | WT | 5.3 |
| | | HLA-A*02:01 | MUT | 8.2 |
| | | | | |
| 2 | PLEC_A398T | HLA-A*02:01 | WT | 0.2 |
| | | HLA-A*02:01 | MUT | 0.3 |
| | A375 (Med) | Allele | | Rank |
| 3 | MYOF_I353T | HLA-A*02:01 | WT | 1.5 |
| | | HLA-A*02:01 | MUT | 1.8 |
| | | | | |
| 5 | RSF1_V956I | HLA-A*02:01 | MUT | 1.5 |
| | | HLA-A*02:01 | WT | 1.6 |
| | | | | |
| 6 | SEC24C_N944S | HLA-A*02:01 | MUT | 2.6 |
| | | HLA-A*02:01 | WT | 3.1 |

Two different peptides (Peptides 1 and 2) are presented from this source protein, overlapping the residue of interest. In none of them the residue is at an anchor position.

For Peptides 3, 5, and 6, the residue is not at an anchor position.

Table 1B: A375 Predicted Binders

| Strong binders | | Weak binders | |
|---|---|---|---|
| Gene | Residue | Gene | Residue |
| ABCC10 | A88 | ABCC10 | A45 |
| ADTRP | S95 | ADTRP | S113 |
| ARHGEF2 | G538 | ANK2 | A1359 |
| CCDC27 | R125 | APOBEC3D | E163 |
| CD5 | V289 | ARHGEF2 | G537 |

(continued)

| Strong binders | | Weak binders | |
|---|---|---|---|
| Gene | Residue | Gene | Residue |
| COL6A6 | R37 | ARID4B | H766 |
| CRELD1 | L14 | ASNSD1 | P551 |
| DCAF4L2 | D84 | BTN2A1 | V185 |
| F2RL3 | L83 | BTNL3 | S231 |
| FOSL2 | V266 | CD1A | S147 |
| GRIK2 | T740 | CD1D | R92 |
| GTF3C2 | P605 | CYP24A1 | P449 |
| HERC2 | I3905 | DDX43 | I283 |
| HIST3H2A | V108 | DOCK11 | E1549 |
| ILDR2 | S308 | FAM46D | S66 |
| LGR6 | S654 | LHX8 | S108 |
| LGR6 | S741 | MAGEB6 | I316 |
| LGR6 | S793 | MTUS1 | D297 |
| LOXHD1 | I768 | **MYOF*** | **I353** |
| METTL8 | H105 | NBEAL2 | D1092 |
| NIPA1 | V310 | NELL1 | V237 |
| OR4A16 | P282 | NKAIN3 | D92 |
| OR51V1 | S252 | NLRP3 | K942 |
| PAPPA2 | N1344 | PLCE1 | K2110 |
| PCDHB2 | G331 | PLEC | A239 |
| PHC2 | R312 | PLXDC2 | T451 |
| **PLEC*** | **A398** | PPP4R1L | T271 |
| PROKR2 | A283 | PTGES2 | A272 |
| SLC2A14 | N67 | PTPRD | G262 |
| SLC36A4 | L117 | PXDNL | P1432 |
| SNAP47 | P94 | RALGAPA2 | S1164 |
| TACC3 | S190 | **RSF1*** | **V956** |
| TBX15 | S238 | SCN11A | M1707 |
| THBS3 | V747 | **SEC24C*** | **N944** |
| TLR8 | F346 | SEMA3F | E216 |
| TRRAP | S722 | SLA | T66 |
| TTN | P28517 | SLC20A1 | P270 |
| UBQLN2 | R249 | SLIT2 | P266 |
| USP19 | N697 | SLITRK2 | P60 |
| | | STK11IP | A955 |
| | | TGIF1 | S4 |
| | | TM9SF4 | P463 |
| | | TTN | D4445 |
| | | TTN | I26997 |
| | | TTN | K8183 |
| | | TTN | P2812 |
| | | TTN | P28515 |
| | | TTN | P9639 |
| | | UBQLN2 | N250 |
| | | WDR19 | S555 |
| | | XDH | G1007 |
| | | ZFHX4 | A60 |
| | | ZNF431 | R145 |

(continued)

| Strong binders | | Weak binders | |
|---|---|---|---|
| Gene | Residue | Gene | Residue |
| | | ZNF814 | K162 |
| Observed from MS (*). | | | |

Table 2A: A2780 Peptide Panel

| Peptide # | A2780 (High) | Allele | | Rank |
|---|---|---|---|---|
| 1 | MAP3K5_M375**V** | HLA-A*02:01 | WT | 0.6 |
| | | HLA-A*02:01 | MUT | 0.6 |
| | | | | |
| 2 | NET1_M159**T** | HLA-A*02:01 | WT | 1.1 |
| | | HLA-A*02:01 | MUT | 1.2 |
| 3 | NET1_M159**T** | HLA-A*02:01 | WT | 14 |
| | | HLA-A*02:01 | MUT | 15 |
| | | | | |
| 4 | NET1_M159**T** | HLA-A*02:01 | WT | 2.5 |
| | | HLA-A*02:01 | MUT | 2.6 |
| | A2780 (Med) | Allele | | Rank |
| 5 | GYS1_L353**F** | HLA-A*02:01 | WT | 0.5 |
| | | HLA-A*02:01 | MUT | 4.9 |

For Peptide 1, the residue is not at an anchor position. Three different peptides (Peptides 2, 3, and 4) are presented from this source protein, overlapping the residue of interest. In none of them the residue is at an anchor position. For Peptide 5, the residue is at an anchor position.

Table 2B: A2780 Predicted Binders

| Strong binders | | Weak binders | |
|---|---|---|---|
| Gene | Residue | Gene | Residue |
| ADAM21 | D101 | ATG16L1 | Q136 |
| CRAT | A610 | BIRC6 | R4218 |
| HHIPL1 | R237 | C2orf16 | F731 |
| IFI44L | P280 | CCDC82 | R383 |
| **MAP3K5*** | **M375** | CFTR | G314 |
| MAP7D2 | T682 | COL6A3 | D773 |
| NET1 | M105 | COL9A1 | M184 |
| **NET1*** | **M159** | CRIPAK | R250 |
| NHSL1 | V501 | DNAH10 | S1076 |
| NHSL1 | V505 | DNAH10 | S894 |
| NSUN4 | Q331 | DYSF | L960 |
| NUPL2 | P314 | EPB41L3 | R375 |
| PHGDH | S277 | GNAS | P335 |
| PROM1 | D200 | **GYS1*** | **L353** |
| | | KANK1 | S860 |
| | | KCND1 | F363 |
| | | KIFC1 | R210 |
| | | LRP5 | M637 |

(continued)

| Strong binders | | Weak binders | |
|---|---|---|---|
| Gene | Residue | Gene | Residue |
| | | NPHP1 | V623 |
| PBX1 | E250 | | |
| PHGDH | S311 | | |
| SMARCA4 | T910 | | |
| TTLL12 | R425 | | |
| UAP1L1 | G275 | | |
| WDR76 | K450 | | |
| Observed from MS (*). | | | |

Table 3A: OV90 Peptide Panel

| Peptide # | OV90 (High) | Allele | | Rank |
|---|---|---|---|---|
| 1 | AMMECR1L_P124**A** | HLA-A*02:01 | WT | 1.7 |
| | | HLA-A*02:01 | MUT | 2 |
| | | | | |
| 2 | IFI27L2_V82**F** | HLA-A*02:01 | MUT | 1.8 |
| | | HLA-A*02:01 | WT | 3.7 |
| | | | | |
| 3 | IFI27L2_V82**F** | HLA-A*02:01 | MUT | 0.7 |
| | | HLA-A*02:01 | WT | 0.8 |

For Peptide 1, the residue is not at an anchor position. Two different peptides (Peptides 2 and 3) are presented from this source protein, overlapping the residue of interest. In none of them the residue is at an anchor position.

Table 3B: OV90 Predicted Binders

| Strong binders | | Weak binders | |
|---|---|---|---|
| Gene | Residue | Gene | Residue |
| AHNAK2 | K4708 | ABCA9 | P1447 |
| **AMMECR1L*** | **P124** | APOB | M495 |
| ATP8B2 | D1078 | CRHBP | T71 |
| CDKN2A | A86 | CRISPLD 1 | M17 |
| FBXW11 | S521 | E2F2 | R256 |
| GPR153 | T48 | FAM193A | T616 |
| HUNK | R168 | FGFR4 | P352 |
| **IFI27L2*** | **V82** | MLKL | M122 |
| KIDINS220 | F1047 | NEK4 | R788 |
| VRTN | T152 | SLC12A8 | G190 |
| | | SLC12A8 | L366 |
| | | ZFYVE26 | R385 |
| Observed from MS (*). | | | |

Table 4A: HeLA Peptide Panel

| Peptide # | HeLa (High) | Allele | | Rank |
|---|---|---|---|---|
| 1 | CRB1_P876L | HLA-A*02:01 | WT | 0.3 |
| | | HLA-A*02:01 | MUT | 0.9 |

For Peptide 1, the residue is not at an anchor position.

Table 4B: HeLa Predicted Binders

| Strong binders | | Weak binders | |
|---|---|---|---|
| Gene | Residue | Gene | Residue |
| **CRB1*** | **P876** | ADCY1 | K348 |
| DIP2B | C934 | BAZ2B | A1146 |
| FAM86C1 | R64 | CCDC142 | V549 |
| FUT10 | S89 | CCDC142 | V556 |
| TPTE2 | R407 | CRIPAK | P208 |
| | | DCC | S383 |
| | | DOCK3 | K520 |
| | | FAM98C | E181 |
| | | GRIK2 | A490 |
| | | MPDU1 | T89 |
| | | NDST2 | V297 |
| | | OBSCN | A7599 |
| | | PCLO | T3520 |
| | | PDE3A | Y814 |
| | | PLEC | C4071 |
| | | RABGGTA | R486 |
| | | RIPK4 | H231 |
| | | SASS6 | A452 |
| | | SLC16A5 | N284 |
| | | SNRNP200 | S1087 |
| | | UGGT1 | S126 |
| USP35 | | | L581 |
| ZNF500 | | | P249 |

Observed from MS (*).

Table 5A: SKOV3 Peptide Panel

| SKOV3 (High) | Allele | | Rank |
|---|---|---|---|
| DHX38_L812**V** | HLA-A*02:01 | MUT | 2.5 |
| | HLA-A*02:01 | WT | 2.7 |
| | | | |
| DHX38_L812**V** | HLA-A*02:01 | WT | 0.2 |
| | HLA-A*02:01 | MUT | 1 |
| | | | |
| MEF2D_Y33**H** | HLA-A*02:01 | WT | 0.5 |
| | HLA-A*02:01 | MUT | 1.3 |
| | | | |
| UBE4B_E936**D** | HLA-A*02:01 | WT | 0.2 |
| | HLA-A*02:01 | MUT | 0.3 |
| SKOV3 (Med) | Allele | | Rank |
| DOCK10_P364**Q** | HLA-A*02:01 | WT | 2.9 |
| | HLA-A*02:01 | MUT | 4.3 |

(continued)

| SKOV3 (High) | Allele | | Rank |
|---|---|---|---|
| RBM47_R251**H** | HLA-A*02:01 | MUT | 1.3 |
| | HLA-A*02:01 | WT | 2.3 |

Two different peptides (Peptides 1 and 2) are presented from this source protein, overlapping the residue of interest. In Peptide 1, the residue is not at an anchor position.

In Peptide 2, the residue is at an anchor position. For Peptides 3, 4, 5, and 6, the residue is not at an anchor position.

Table 5B: SKOV3 Predicted Binders

| Strong binders | | Weak binders | |
|---|---|---|---|
| Gene | Residue | Gene | Residue |
| ABCD1 | S342 | ABCD1 | S157 |
| ADRA2A | A63 | AHSA1 | E220 |
| B4GALNT2 | V510 | ANO7 | C875 |
| CUL4B | I663 | ASPRV1 | E322 |
| **DHX38*** | **L812** | BAAT | G72 |
| DNAAF1 | P571 | C17orf53 | N563 |
| FZD3 | F8 | CLIP3 | F318 |
| HCN4 | V319 | CTDP1 | F816 |
| KLHL26 | R252 | CUL4B | I668 |
| LIMK2 | G499 | CUL4B | I681 |
| LIMK2 | G520 | DISP1 | A562 |
| MANBA | E745 | DOCK10 | P358 |
| **MEF2D*** | **Y33** | **DOCK10*** | **P364** |
| NPHP4 | V883 | FBXW7 | R266 |
| PIGN | F5 | FBXW7 | R505 |
| PTGER4 | A180 | FKBP10 | V337 |
| SLC18A1 | T39 | HSF1 | N65 |
| TCF7L2 | N452 | IRGQ | M241 |
| TMEM175 | A471 | ITGA8 | A100 |
| TREML2 | C115 | KRTAP13-4 | A138 |
| TUFM | G29 | LPIN2 | L763 |
| **UBE4B*** | **E936** | 3-Mar | R143 |
| ZFHX3 | 1935 | MED13L | T28 |
| ZNF233 | D384 | MTMR2 | I544 |
| | | MVK | A270 |
| | | ONECUT2 | R407 |
| | | OR5AC2 | Y253 |
| | | PDE6A | R102 |
| | | **RBM47*** | **R251** |
| | | SELENBP1 | S354 |
| | | SLC24A3 | G613 |
| | | STRA6 | C256 |
| | | TBC1D17 | Y326 |
| | | TCEANC2 | R187 |
| | | WRNIP 1 | V429 |
| | | ZC3H7B | T226 |

Observed from MS (*).

Analyzing a database of native peptides found in complex with an HLA-A*02:01 MHC-I in these 5 cell lines, across cell lines, 9.8% of mutations predicted to strongly bind and 4.0% of mutations predicted to bind an HLA-A*02:01 MHC-I at any strength were also supported by MS-derived peptides (Figure 2D). These experimental results validate the ability of a score derived from MHC-I binding affinities to identify mutations with a higher likelihood of generating neoantigens and support the application of this score to evaluate MHC-I genotype as a determinant of the antigenic potential of recurrent mutations in tumors.

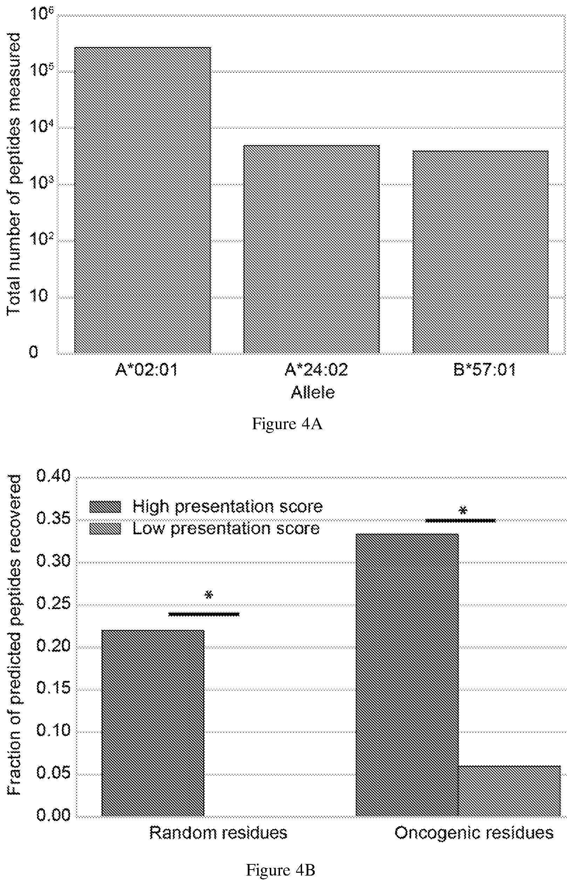

The formation of a stable complex is a prerequisite for antigen presentation, but does not ensure that an antigen will be displayed on the cell surface. The presentation score was experimentally validated for different peptides using three of the most common HLA alleles. HLA alleles A*24:02, A*02:01, and B*57:01 were overexpressed in six cell lines (HeLa, FHIOSE, SKOV3, 721.221, A2780, and OV90). HLA-peptide complexes were purified from the cell surface, and the bound peptides were isolated. Their sequence was determined using mass spectrometry (Patterson et al., Mol. Cancer Ther., 2016, 15, 313-322; and Trolle et al., J. Immunol., 2016, 196, 1480-1487). The amount of mass spectrometry (MS) data obtained for each allele differed substantially, rendering A*24:02 and B*57:01 underpowered to detect differences (Figure 4A). First, balanced numbers of random human peptides to bind or not bind these HLA-alleles were selected based on the score. Residues with high HLA allele-specific presentation scores were far more likely to be detected in complex with the MHC-I molecule on the cell surface than residues with low presentation scores ($p = 3.3 \times 10^{-7}$, Figure 4B, Table 6). Next, the presentation of balanced numbers of recurrent oncogenic mutations predicted to bind or not bind these same HLA alleles were evaluated. It was observed that recurrent oncogenic mutations receiving a high presentation score were also more likely to generate peptides observed in complex with the MHC-I molecule on the cell surface ($p = 0.0003$, Figure 4B). Thus, these experimental results validate the expectation that when considering a given amino acid residue, a higher number of peptides containing the residue that are predicted to stably bind to an MHC-I allele will correlate with a higher number of peptide neoantigens displayed on the cell surface by that allele and therefore a greater potential to engage Tcell receptors.

**Example 2: Statistical Analysis of Affinity Score vs. Presence Of Mutation**

The data consists of a 9176×1018 binary mutation matrix $y_{ij} \in \{0,1\}$, indicating that subject *i* has/does not have a mutation in residue *j*. Another 9176×1018 matrix containing the predicted affinity $x_{ij}$ of subject i for mutation *j*. All analyses below are restricted to the 412 residues that presented mutations in ≥ 5 subjects.

The question considered was whether $x_{ij}$ have an effect on $y_{ij}$ within subjects, or, in other words whether affinity scores help predict, within a given subject, which residues are likely to undergo mutations.

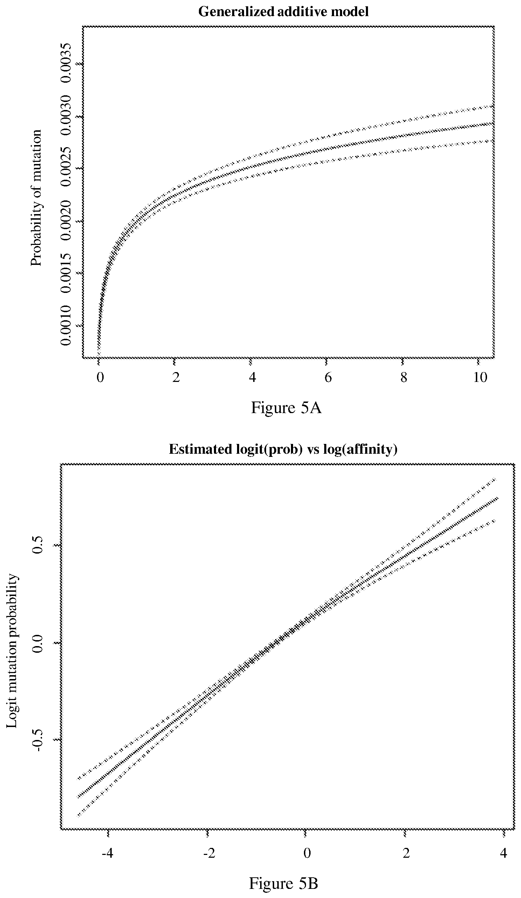

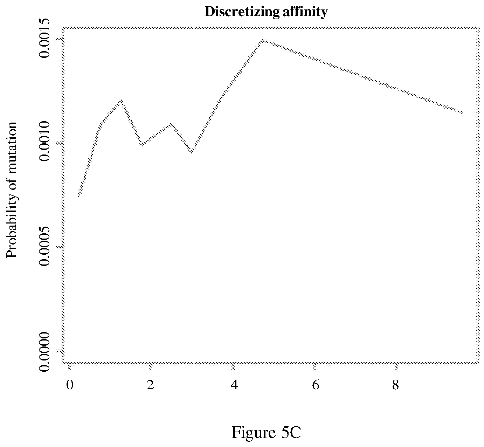

To address the above question, logistic regression models were used. An important issue in such models is to capture adequately the type of effect that $x_{ij}$ has on $y_{ij}$, e.g. is it linear (in some sense), or all that matters is whether the affinity is beyond a certain threshold. To this end an additive logistic regression with non-linear effects for the affinity, was fitted via function gam in R package mgcv. The estimated mutation probability as a function of affinity, $P(y_{ij} = 1 \mid x_{ij})$, is portrayed in Figure 5A. The corresponding logit mutation probabilities as a function of the log-affinity is shown in Figure 5B, revealing that the association between the two is linear. This justifies considering a linear effect of $\log(x_{ij})$ on the logit mutation probability. As a check, Figure 5C shows the estimated mutation probabilities based on discretizing the affinity scores into groups, = showing a similar pattern than the top panel (i.e. reinforcing that the GAM provides a good fit for the data).

The following random-effects model was considered:

$$\text{logit}\,(P(y_{ij} = 1 \mid x_{ij})) = \eta_i + \gamma \log(x_{ij}), \qquad (1)$$

where $y_{ij}$ is a binary mutation matrix $y_{ij} \in \{0,1\}$ indicating whether a subject *i* has a mutation *j*; $x_{ij}$ is a binary mutation matrix indicating predicted MHC-I binding affinity of subject *i* having mutation *j*; $\gamma$ measures the effect of the log-affinities on the mutation probability; and $\eta_j \sim N(0, \phi_\eta)$ are random effects capturing different mutation rates among subjects.

The question corresponds testing the null hypothesis that $\gamma = 0$ in the model above. This mixed effects logistic regression gave a highly significant result (R output in Table 6), indicating that the affinity score does have a within-subjects impact on the occurrence of mutation. The estimated random effects standard deviation was $\phi_\eta$ = 0:505, indicating that overall mutation rates differ across subjects.

Table 6: Model (1) R output

| Fixed effects: | | | | |
|---|---|---|---|---|
| | Estimate | Std. Error | z value | Pr(>\|z\|) |
| (Intercept) | -6.353366 | 0.016581 | -383.2 | $<2e^{-16}$ *** |

(continued)

| Fixed effects: | | | | |
|---|---|---|---|---|
| | Estimate | Std. Error | z value | Pr(>\|z\|) |
| log(x[sel]) | 0.184880 | 0.008602 | 21.5 | <2e-16 *** |
| | | | | |
| Random effects: | | | | |
| Groups Name | Variance | Std. Dev. | | |
| pat[sel] (Intercept) | 0.2555 | 0.5054 | | |
| Number of obs: | 3780512 | groups: pat[sel], 9176 | | |

As a final check the following model with both subject and residue random effects was considered:

$$\text{logit}\ (P(y_{ij} = 1 \mid x_{ij})) = \eta_i + \beta_j + \gamma \log(x_{ij}), \qquad (2)$$

where $\eta_j \sim N(0, \phi_\eta)$, $\beta_j \sim N(0, \phi_\beta)$ The results are analogous to the previous analyses. The R output is in Table 7.

Table 7: Model (2) R output

| Fixed effects: | | | | |
|---|---|---|---|---|
| | Estimate | Std. Error | z value | Pr(>\|z\|) |
| (Intercept) | -6.92161 | 0.04365 | -158.57 | <2e-16 *** |
| log(x[sel]) | 0.01790 | 0.01100 | 1.63 | 0.104 |
| | | | | |
| Random effects: | | | | |
| Groups Name | Variance | Std. Dev. | | |
| pat[sel] (Intercept) | 0.2109 | 0.4592 | | |
| gene[sel] (Intercept) | 0.6214 | 0.7883 | | |
| Number of obs: | 3780512 | groups: pat[sel], 9176; gene[sel], 412 | | |

Table 8 summarizes the results in terms of odds ratios (i.e. the increase in the odds of mutation for a +1 increase in log-affinity). The odds-ratio for the within- subjects model (Question 3) is virtually identical to the global model, the predictive power of a_nity within a subject is similar to the overall predictive power. A unit increase in log-a_nity (equivalently, a 2.7 fold increase in the affinity) increases the odds of mutation by 15.9%. In contrast, the odds-ratio for the within-residues model is close to 1, signaling that within residues the a_nity score has practically negligible predictive power.

Table 8: Odds ratios for log- affinity

| | Odds Ratio | 95% CI | P-value |
|---|---|---|---|
| Within-subjects (Model (1)) | 1.203 | (1.183,1.224) | $< 2 \times 10^{-16}$ |
| Within-residues & subjects (Model (2)) | 1.018 | (0.996,1.040) | 0.1040 |
| Global: model with no random effects. Within-residues: model with residue random effects. Within-subjects: model with subject random effects. | | | |

**Example 3: Separate Analysis for Each Cancer Type**

The within-residues and within-subjects analyses were carried out, selecting only the subjects with a specific cancer type (the number of subjects with each cancer type are indicated in Table 9). Following random-effects model was

considered.

$$\text{logit}\,(P(y_{ij} = 1 \mid x_{ij})) = \beta_j + \gamma \log(x_{ij}), \qquad (3)$$

where $\gamma$ measures the effect of the log-affinities on the mutation probability and $\beta_j \sim N(0, \phi_\beta)$ are random effects capturing residue-specific effects (e.g. whether one residue has an overall higher probability of mutation than another). The null hypothesis $\gamma = 0$ was tested. The model in (3) was fitted via function glmer from R package lme4. The analysis was restricted to residues with $\geq 5$ mutations, as the remaining residues contain little information and result in an unmanageable increase in the computational burden ($\geq 3$ and $\geq 10$ mutations, were also checked, obtaining similar results).

Table 9: The number of subjects analyzed for each cancer type in model (3)

| Cancer | Number of subjects |
|---|---|
| ACC | 91 |
| BLCA | 409 |
| BRCA | 897 |
| CESC | 55 |
| COAD | 396 |
| DLBC | 36 |
| GBM | 390 |
| HNSC | 503 |
| KICH | 66 |
| KIRC | 333 |
| KIRP | 281 |
| LAML | 138 |
| LGG | 506 |
| LIHC | 361 |
| LUAD | 565 |
| LUSC | 487 |
| MESO | 82 |
| OV | 403 |
| PAAD | 175 |
| PCPG | 179 |
| PRAD | 492 |
| READ | 135 |
| SARC | 172 |
| SKCM | 467 |
| STAD | 435 |
| TGCT | 144 |
| THCA | 484 |
| UCEC | 359 |
| UCS | 57 |
| UVM | 78 |

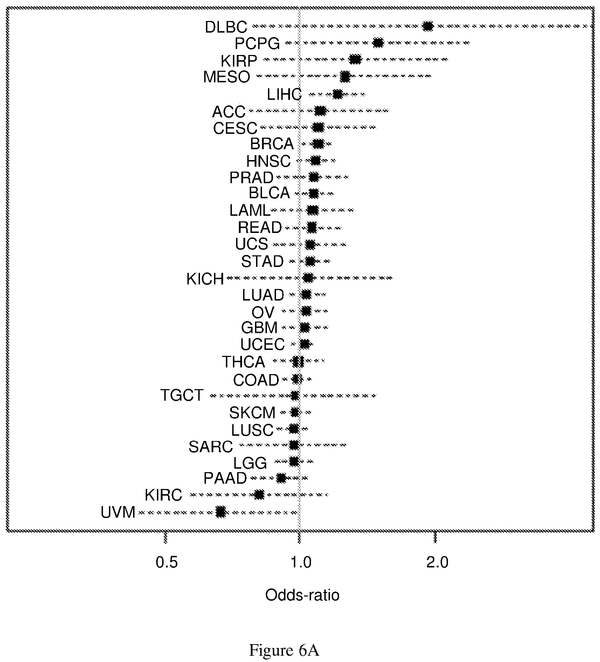

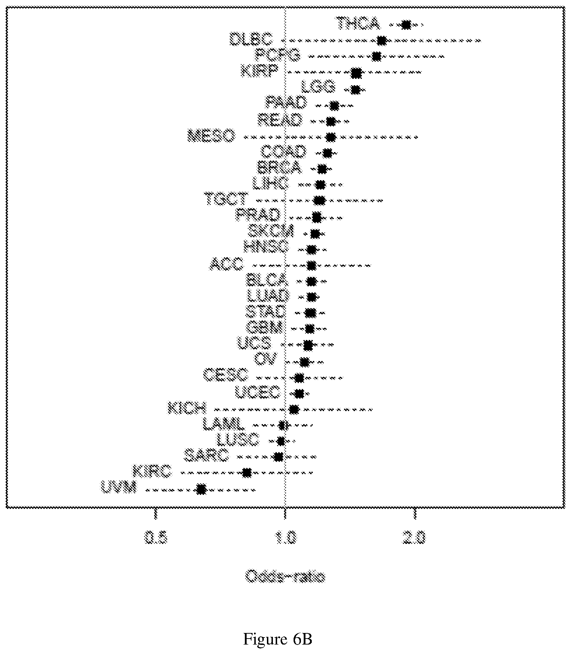

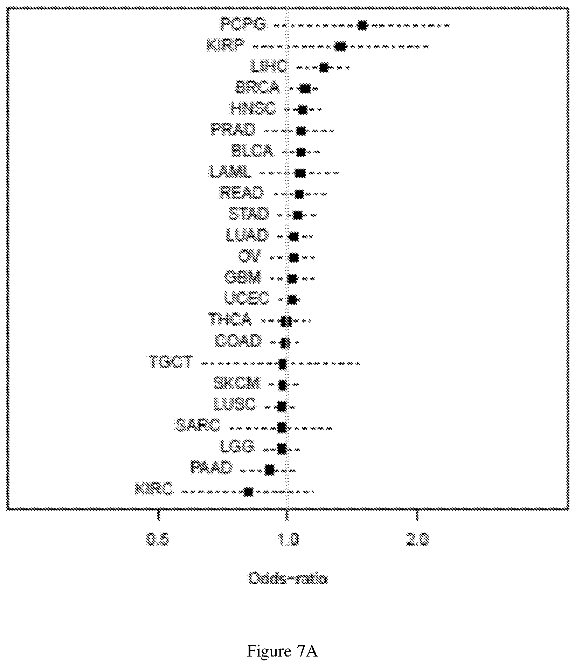

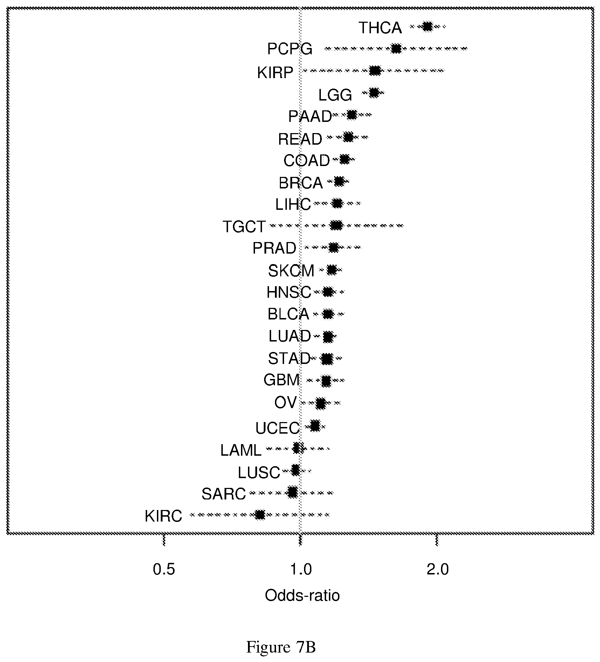

Tables 10 and 11 report odds-ratios, 95% intervals and P-values. Figures 6A and 6B display these 95% intervals, and Figures 7A and 7B repeat the same display using only the cancer types with ≥100 subjects. The salient feature is that in the within-residues analysis most intervals contain the value OR=1 (which corresponds to no predictive power), whereas in the within-subjects analysis they're focused on OR> 1 for more than half of the cancer types. As expected, the 95% intervals are wider for those cancer types with less subjects.

Table 10: Odds ratios, 95% intervals and P-value of the within-residues analysis separately for each cancer subtype

| | OR | 95% CI | P-value |
|---|---|---|---|
| ACC | 1.110 | 0.770,1.599 | 0.5767 |
| BLCA | 1.072 | 0.976,1.177 | 0.1477 |
| BRCA | 1.099 | 1.011,1.196 | 0.0274 |
| CESC | 1.100 | 0.818,1.480 | 0.5291 |
| COAD | 0.986 | 0.914,1.064 | 0.7250 |
| DLBC | 1.920 | 0.786,4.692 | 0.1522 |
| GBM | 1.025 | 0.913,1.152 | 0.6715 |
| HNSC | 1.086 | 0.990,1.190 | 0.0798 |
| KICH | 1.046 | 0.690,1.586 | 0.8328 |
| KIRC | 0.812 | 0.573,1.151 | 0.2423 |
| KIRP | 1.327 | 0.835,2.108 | 0.2319 |
| LAML | 1.068 | 0.869,1.314 | 0.5312 |
| LGG | 0.965 | 0.880,1.059 | 0.4547 |
| LIHC | 1.215 | 1.054,1.401 | 0.0074 |
| LUAD | 1.038 | 0.950,1.134 | 0.4100 |
| LUSC | 0.969 | 0.891,1.054 | 0.4610 |
| MESO | 1.264 | 0.804,1.989 | 0.3101 |
| OV | 1.037 | 0.912,1.179 | 0.5793 |
| PAAD | 0.908 | 0.783,1.052 | 0.1989 |
| PCPG | 1.487 | 0.937,2.361 | 0.0922 |
| PRAD | 1.072 | 0.887,1.295 | 0.4740 |
| READ | 1.067 | 0.928,1.226 | 0.3627 |
| SARC | 0.967 | 0.736,1.270 | 0.8077 |
| SKCM | 0.976 | 0.906,1.050 | 0.5104 |
| STAD | 1.054 | 0.955,1.163 | 0.2988 |
| TGCT | 0.977 | 0.634,1.506 | 0.9168 |
| THCA | 0.991 | 0.870,1.129 | 0.8959 |
| UCEC | 1.020 | 0.956,1.088 | 0.5434 |
| UCS | 1.058 | 0.872,1.282 | 0.5685 |
| UVM | 0.664 | 0.441,0.998 | 0.0487 |

Table 11: Odds ratios, 95% intervals and P-value of the within-subjects analysis separately for each cancer subtype

| | OR | 95% CI | P-value |
|---|---|---|---|
| ACC | 1.155 | 0.842,1.583 | 0.3715 |
| BLCA | 1.151 | 1.069,1.240 | 0.0002 |
| BRCA | 1.224 | 1.152,1.300 | 0.0000 |

(continued)

| | OR | 95% CI | P-value |
|---|---|---|---|
| CESC | 1.082 | 0.864,1.353 | 0.4930 |
| COAD | 1.252 | 1.183,1.326 | 0.0000 |
| DLBC | 1.671 | 0.985,2.836 | 0.0570 |
| GBM | 1.137 | 1.039,1.244 | 0.0050 |
| HNSC | 1.155 | 1.077,1.240 | 0.0001 |
| KICH | 1.046 | 0.690,1.586 | 0.8328 |
| KIRC | 0.812 | 0.573,1.151 | 0.2422 |
| KIRP | 1.463 | 1.016,2.107 | 0.0408 |
| LAML | 0.989 | 0.849,1.151 | 0.8825 |
| LGG | 1.460 | 1.379,1.546 | 0.0000 |
| LIHC | 1.206 | 1.077,1.349 | 0.0011 |
| LUAD | 1.151 | 1.079,1.228 | 0.0000 |
| LUSC | 0.982 | 0.918,1.049 | 0.5846 |
| MESO | 1.275 | 0.804,2.020 | 0.3014 |
| OV | 1.106 | 1.007,1.214 | 0.0356 |
| PAAD | 1.306 | 1.185,1.439 | 0.0000 |
| PCPG | 1.635 | 1.144,2.336 | 0.0070 |
| PRAD | 1.188 | 1.025,1.376 | 0.0219 |
| READ | 1.280 | 1.156,1.417 | 0.0000 |
| SARC | 0.961 | 0.780,1.185 | 0.7118 |
| SKCM | 1.171 | 1.106,1.239 | 0.0000 |
| STAD | 1.146 | 1.062,1.237 | 0.0005 |
| TGCT | 1.202 | 0.862,1.676 | 0.2784 |
| THCA | 1.914 | 1.752,2.091 | 0.0000 |
| UCEC | 1.079 | 1.028,1.132 | 0.0021 |
| UCS | 1.131 | 0.978,1.308 | 0.0966 |
| UVM | 0.640 | 0.475,0.862 | 0.0033 |

**Example 4: Groups of High-Frequency Mutation Residues**

The global and cancer-type specific analyses were repeated selecting only highly-mutated sets of residues (listed below). For instance, the 10 residues highly mutated in BRCA were selected and fit the within-subjects model, fist using all subjects (global OR) and then using only subjects with each cancer subtype. These odds-ratios are listed in Tables 12-23. In a number of instances the number of mutations in the selected residues/subjects was too small to obtain reliable estimates, in these instances no estimate is reported.

Table 12: Within-subjects analysis for residues with high mutation frequency in BRCA

| | OR | CI.low | CI.high | pvalue |
|---|---|---|---|---|
| Global | 1.254 | 1.182 | 1.331 | 0.0000 |
| ACC | | | | |
| BLCA | 1.179 | 0.933 | 1.490 | 0.1673 |
| BRCA | 1.072 | 0.967 | 1.189 | 0.1880 |

(continued)

| | OR | CI.low | CI.high | pvalue |
|---|---|---|---|---|
| CESC | 1.607 | 0.835 | 3.096 | 0.1557 |
| COAD | 1.262 | 1.053 | 1.512 | 0.0117 |
| DLBC | | | | |
| GBM | 2.005 | 1.302 | 3.086 | 0.0016 |
| HNSC | 1.420 | 1.154 | 1.748 | 0.0009 |
| KICH | | | | |
| KIRC | 0.314 | 0.082 | 1.207 | 0.0918 |
| KIRP | 1.062 | 0.378 | 2.982 | 0.9086 |
| LAML | | | | |
| LGG | 2.059 | 2.053 | 2.065 | 0.0000 |
| LIHC | 1.504 | 0.831 | 2.722 | 0.1775 |
| LUAD | 1.427 | 0.893 | 2.279 | 0.1370 |
| LUSC | 1.104 | 0.832 | 1.464 | 0.4935 |
| MESO | | | | |
| OV | 2.160 | 1.498 | 3.114 | 0.0000 |
| PAAD | 2.104 | 1.081 | 4.097 | 0.0286 |
| PCPG | | | | |
| PRAD | 0.718 | 0.429 | 1.199 | 0.2051 |
| READ | 1.633 | 1.074 | 2.482 | 0.0217 |
| SARC | 1.237 | 0.638 | 2.400 | 0.5293 |
| SKCM | 0.853 | 0.463 | 1.574 | 0.6118 |
| STAD | 1.578 | 1.232 | 2.022 | 0.0003 |
| TGCT | 0.943 | 0.342 | 2.598 | 0.9095 |
| THCA | 0.265 | 0.090 | 0.787 | 0.0168 |
| UCEC | 1.116 | 0.905 | 1.376 | 0.3036 |
| UCS | 2.056 | 1.144 | 3.696 | 0.0160 |
| UVM | | | | |

Table 13: Within-subjects analysis for residues with high mutation frequency in COAD

| | OR | CI.low | CI.high | pvalue |
|---|---|---|---|---|
| Global | 1.047 | 0.993 | 1.105 | 0.0902 |
| ACC | | | | |
| BLCA | 0.627 | 0.467 | 0.841 | 0.0018 |
| BRCA | 0.892 | 0.720 | 1.104 | 0.2916 |
| CESC | 1.828 | 0.795 | 4.200 | 0.1554 |
| COAD | 1.034 | 0.903 | 1.184 | 0.6274 |
| DLBC | | | | |
| GBM | 0.759 | 0.529 | 1.089 | 0.1346 |
| HNSC | 1.032 | 0.786 | 1.354 | 0.8223 |

(continued)

| | OR | CI.low | CI.high | pvalue |
|---|---|---|---|---|
| KICH | | | | |
| KIRC | | | | |
| KIRP | 1.465 | 0.633 | 3.395 | 0.3727 |
| LAML | 1.838 | 0.693 | 4.875 | 0.2213 |
| LGG | 0.811 | 0.569 | 1.156 | 0.2465 |
| LIHC | 1.400 | 0.681 | 2.878 | 0.3605 |
| LUAD | 0.795 | 0.626 | 1.009 | 0.0592 |
| LUSC | 0.895 | 0.607 | 1.320 | 0.5761 |
| MESO | | | | |
| OV | 0.847 | 0.605 | 1.186 | 0.3331 |
| PAAD | 0.832 | 0.676 | 1.024 | 0.0827 |
| PCPG | | | | |
| PRAD | 0.536 | 0.274 | 1.049 | 0.0685 |
| READ | 0.871 | 0.677 | 1.122 | 0.2867 |
| SARC | 0.847 | 0.306 | 2.349 | 0.7503 |
| SKCM | 1.263 | 1.085 | 1.470 | 0.0026 |
| STAD | 1.196 | 0.928 | 1.543 | 0.1675 |
| TGCT | 0.723 | 0.270 | 1.933 | 0.5176 |
| THCA | 1.477 | 1.291 | 1.690 | 0.0000 |
| UCEC | 0.844 | 0.659 | 1.082 | 0.1815 |
| UCS | 1.153 | 0.695 | 1.915 | 0.5814 |
| UVM | | | | |

Table 14: Within-subjects analysis for residues with high mutation frequency in HNSC

| | OR | CI.low | CI.high | pvalue |
|---|---|---|---|---|
| Global | 1.115 | 1.048 | 1.187 | 0.0006 |
| ACC | | | | |
| BLCA | 1.047 | 0.847 | 1.294 | 0.6707 |
| BRCA | 1.090 | 0.967 | 1.229 | 0.1565 |
| CESC | 1.908 | 0.905 | 4.023 | 0.0896 |
| COAD | 1.022 | 0.857 | 1.218 | 0.8090 |
| DLBC | | | | |
| GBM | 1.184 | 0.766 | 1.828 | 0.4467 |
| HNSC | 1.077 | 0.896 | 1.296 | 0.4294 |
| KICH | | | | |
| KIRC | | | | |
| KIRP | 0.945 | 0.342 | 2.606 | 0.9127 |
| LAML | | | | |
| LGG | 1.298 | 1.288 | 1.308 | 0.0000 |

(continued)

| | OR | CI.low | CI.high | pvalue |
|---|---|---|---|---|
| LIHC | 1.196 | 0.621 | 2.304 | 0.5927 |
| LUAD | 0.796 | 0.553 | 1.146 | 0.2199 |
| LUSC | 0.982 | 0.754 | 1.281 | 0.8957 |
| MESO | | | | |
| OV | 1.187 | 0.763 | 1.848 | 0.4468 |
| PAAD | 1.592 | 0.869 | 2.916 | 0.1325 |
| PCPG | | | | |
| PRAD | 0.776 | 0.482 | 1.250 | 0.2973 |
| READ | 1.767 | 1.175 | 2.655 | 0.0062 |
| SARC | 0.996 | 0.368 | 2.691 | 0.9933 |
| SKCM | 2.004 | 0.454 | 8.846 | 0.3590 |
| STAD | 1.421 | 1.094 | 1.845 | 0.0085 |
| TGCT | 1.438 | 0.355 | 5.828 | 0.6107 |
| THCA | | | | |
| UCEC | 1.192 | 0.948 | 1.500 | 0.1332 |
| UCS | 1.569 | 0.956 | 2.572 | 0.0745 |
| UVM | | | | |

Table 15: Within-subjects analysis for residues with high mutation frequency in KIRC

| | OR | CI.low | CI.high | pvalue |
|---|---|---|---|---|
| Global | 0.892 | 0.534 | 1.489 | 0.6616 |
| ACC | | | | |
| BLCA | | | | |
| BRCA | | | | |
| CESC | | | | |
| COAD | | | | |
| DLBC | | | | |
| GBM | | | | |
| HNSC | | | | |
| KICH | | | | |
| KIRC | 0.829 | 0.492 | 1.396 | 0.4809 |
| KIRP | | | | |
| LAML | | | | |
| LGG | | | | |
| LIHC | | | | |
| LUAD | | | | |
| LUSC | | | | |
| MESO | | | | |
| OV | | | | |

(continued)

| | OR | CI.low | CI.high | pvalue |
|---|---|---|---|---|
| PAAD | | | | |
| PCPG | | | | |
| PRAD | | | | |
| READ | | | | |
| SARC | | | | |
| SKCM | | | | |
| STAD | | | | |
| TGCT | | | | |
| THCA | | | | |
| UCEC | | | | |
| UCS | | | | |
| UVM | | | | |

Table 16: Within-subjects analysis for residues with high mutation frequency in LGG

| | OR | CI.low | CI.high | pvalue |
|---|---|---|---|---|
| Global | 1.247 | 1.136 | 1.369 | 0.0000 |
| ACC | | | | |
| BLCA | 1.264 | 0.620 | 2.577 | 0.5186 |
| BRCA | 1.021 | 0.663 | 1.571 | 0.9251 |
| CESC | | | | |
| COAD | 1.069 | 0.706 | 1.617 | 0.7532 |
| DLBC | | | | |
| GBM | 1.678 | 1.084 | 2.598 | 0.0202 |
| HNSC | 1.182 | 0.738 | 1.893 | 0.4873 |
| KICH | | | | |
| KIRC | | | | |
| KIRP | | | | |
| LAML | 1.640 | 0.901 | 2.984 | 0.1054 |
| LGG | 1.131 | 1.025 | 1.248 | 0.0140 |
| LIHC | 1.680 | 0.717 | 3.939 | 0.2324 |
| LUAD | 1.813 | 0.505 | 6.509 | 0.3613 |
| LUSC | 0.878 | 0.425 | 1.813 | 0.7249 |
| MESO | 1.250 | 0.307 | 5.088 | 0.7557 |
| OV | 1.085 | 0.659 | 1.785 | 0.7486 |
| PAAD | 0.721 | 0.348 | 1.495 | 0.3791 |
| PCPG | | | | |
| PRAD | 0.673 | 0.282 | 1.604 | 0.3716 |
| READ | 0.952 | 0.485 | 1.870 | 0.8862 |
| SARC | | | | |

(continued)

| | OR | CI.low | CI.high | pvalue |
|---|---|---|---|---|
| SKCM | 1.682 | 0.959 | 2.949 | 0.0696 |
| STAD | 1.360 | 0.865 | 2.139 | 0.1826 |
| TGCT | | | | |
| THCA | | | | |
| UCEC | 1.105 | 0.642 | 1.901 | 0.7182 |
| UCS | 2.208 | 0.872 | 5.593 | 0.0947 |
| UVM | | | | |

Table 17: Within-subjects analysis for residues with high mutation frequency in LUAD

| | OR | CI.low | CI.high | pvalue |
|---|---|---|---|---|
| Global | 1.400 | 1.275 | 1.538 | 0.0000 |
| ACC | | | | |
| BLCA | 1.110 | 0.591 | 2.086 | 0.7452 |
| BRCA | 2.102 | 0.674 | 6.557 | 0.2008 |
| CESC | 3.952 | 0.964 | 16.207 | 0.0563 |
| COAD | 1.700 | 1.363 | 2.120 | 0.0000 |
| DLBC | | | | |
| GBM | 56.989 | 0.024 | 132782.426 | 0.3068 |
| HNSC | | | | |
| KICH | | | | |
| KIRC | | | | |
| KIRP | 2.730 | 1.010 | 7.381 | 0.0478 |
| LAML | 4.266 | 1.238 | 14.699 | 0.0215 |
| LGG | | | | |
| LIHC | 4.777 | 1.103 | 20.694 | 0.0365 |
| LUAD | 1.112 | 0.949 | 1.303 | 0.1876 |
| LUSC | 1.797 | 0.373 | 8.644 | 0.4647 |
| MESO | | | | |
| OV | 1.541 | 0.508 | 4.668 | 0.4448 |
| PAAD | 1.515 | 1.191 | 1.928 | 0.0007 |
| PCPG | | | | |
| PRAD | | | | |
| READ | 1.384 | 0.954 | 2.009 | 0.0870 |
| SARC | | | | |
| SKCM | 2.282 | 0.472 | 11.028 | 0.3048 |
| STAD | 2.060 | 1.130 | 3.758 | 0.0184 |
| TGCT | 1.917 | 0.641 | 5.731 | 0.2442 |
| THCA | | | | |
| UCEC | 1.321 | 0.968 | 1.801 | 0.0791 |

(continued)

| | OR | CI.low | CI.high | pvalue |
|---|---|---|---|---|
| UCS | 2.429 | 0.882 | 6.686 | 0.0859 |
| UVM | | | | |

Table 18: Within-subjects analysis for residues with high mutation frequency in LUSC

| | OR | CI.low | CI.high | pvalue |
|---|---|---|---|---|
| Global | 1.108 | 1.102 | 1.114 | 0.0000 |
| ACC | | | | |
| BLCA | 1.173 | 0.934 | 1.475 | 0.1702 |
| BRCA | 1.256 | 1.057 | 1.494 | 0.0097 |
| CESC | 1.781 | 0.894 | 3.549 | 0.1009 |
| COAD | 1.182 | 0.933 | 1.497 | 0.1661 |
| DLBC | | | | |
| GBM | 1.278 | 0.565 | 2.889 | 0.5562 |
| HNSC | 1.096 | 0.887 | 1.355 | 0.3970 |
| KICH | | | | |
| KIRC | | | | |
| KIRP | | | | |
| LAML | | | | |
| LGG | 0.913 | 0.484 | 1.722 | 0.7777 |
| LIHC | 1.142 | 0.579 | 2.253 | 0.7017 |
| LUAD | 0.776 | 0.588 | 1.024 | 0.0733 |
| LUSC | 0.916 | 0.787 | 1.067 | 0.2619 |
| MESO | | | | |
| OV | 0.895 | 0.622 | 1.289 | 0.5526 |
| PAAD | | | | |
| PCPG | | | | |
| PRAD | | | | |
| READ | 1.503 | 0.633 | 3.568 | 0.3554 |
| SARC | | | | |
| SKCM | 1.547 | 0.524 | 4.563 | 0.4292 |
| STAD | 1.295 | 0.846 | 1.983 | 0.2346 |
| TGCT | 1.340 | 0.470 | 3.820 | 0.5845 |
| THCA | | | | |
| UCEC | 1.239 | 0.837 | 1.832 | 0.2838 |
| UCS | 1.306 | 0.636 | 2.682 | 0.4667 |
| UVM | | | | |

Table 19: Within-subjects analysis for residues with high mutation frequency in PRAD

| | OR | CI.low | CI.high | pvalue |
|---|---|---|---|---|
| Global | 0.982 | 0.754 | 1.279 | 0.8917 |

(continued)

| | OR | CI.low | CI.high | pvalue |
|---|---|---|---|---|
| ACC | | | | |
| BLCA | | | | |
| BRCA | | | | |
| CESC | | | | |
| COAD | | | | |
| DLBC | | | | |
| GBM | | | | |
| HNSC | | | | |
| KICH | | | | |
| KIRC | | | | |
| KIRP | | | | |
| LAML | | | | |
| LGG | | | | |
| LIHC | | | | |
| LUAD | | | | |
| LUSC | | | | |
| MESO | | | | |
| OV | | | | |
| PAAD | | | | |
| PCPG | | | | |
| PRAD | 0.980 | 0.753 | 1.275 | 0.8780 |
| READ | | | | |
| SARC | | | | |
| SKCM | | | | |
| STAD | | | | |
| TGCT | | | | |
| THCA | | | | |
| UCEC | | | | |
| UCS | | | | |

Table 20: Within-subjects analysis for residues with high mutation frequency in SKCM

| | OR | CI.low | CI.high | pvalue |
|---|---|---|---|---|
| Global | 1.642 | 1.637 | 1.647 | 0.0000 |
| ACC | | | | |
| BLCA | 1.390 | 0.760 | 2.545 | 0.2852 |
| BRCA | | | | |
| CESC | | | | |
| COAD | 1.512 | 1.250 | 1.829 | 0.0000 |
| DLBC | | | | |

(continued)

| | OR | CI.low | CI.high | pvalue |
|---|---|---|---|---|
| GBM | 1.428 | 0.893 | 2.284 | 0.1371 |
| HNSC | 1.547 | 0.672 | 3.561 | 0.3047 |
| KICH | | | | |
| KIRC | | | | |
| KIRP | 1.675 | 0.524 | 5.352 | 0.3844 |
| LAML | 1.208 | 0.835 | 1.748 | 0.3157 |
| LGG | 1.482 | 1.098 | 2.002 | 0.0102 |
| LIHC | 2.116 | 0.825 | 5.426 | 0.1187 |
| LUAD | 1.431 | 0.974 | 2.103 | 0.0681 |
| LUSC | 1.007 | 0.593 | 1.709 | 0.9803 |
| MESO | | | | |
| OV | 1.084 | 0.558 | 2.106 | 0.8116 |
| PAAD | | | | |
| PCPG | | | | |
| PRAD | 1.240 | 0.513 | 2.998 | 0.6330 |
| READ | 1.555 | 0.849 | 2.848 | 0.1527 |
| SARC | | | | |
| SKCM | 1.334 | 1.245 | 1.430 | 0.0000 |
| STAD | 1.093 | 0.478 | 2.497 | 0.8336 |
| TGCT | 1.040 | 0.548 | 1.972 | 0.9043 |
| THCA | 1.881 | 1.704 | 2.076 | 0.0000 |
| UCEC | 1.076 | 0.646 | 1.793 | 0.7789 |
| UCS | | | | |
| UVM | | | | |

Table 21: Within-subjects analysis for residues with high mutation frequency in STAD

| | OR | CI.low | CI.high | pvalue |
|---|---|---|---|---|
| Global | 0.999 | 0.924 | 1.080 | 0.9795 |
| ACC | 0.957 | 0.191 | 4.798 | 0.9572 |
| BLCA | 0.780 | 0.567 | 1.072 | 0.1258 |
| BRCA | 0.697 | 0.593 | 0.819 | 0.0000 |
| CESC | 2.626 | 0.989 | 6.968 | 0.0526 |
| COAD | 1.171 | 0.978 | 1.403 | 0.0863 |
| DLBC | | | | |
| GBM | 1.190 | 0.716 | 1.979 | 0.5018 |
| HNSC | 1.022 | 0.756 | 1.382 | 0.8863 |
| KICH | | | | |
| KIRC | | | | |
| KIRP | 5.501 | 1.266 | 23.897 | 0.0229 |

(continued)

| | OR | CI.low | CI.high | pvalue |
|---|---|---|---|---|
| LAML | 34.584 | 0.542 | 2205.582 | 0.0947 |
| LGG | 0.913 | 0.688 | 1.213 | 0.5311 |
| LIHC | 2.583 | 1.077 | 6.193 | 0.0334 |
| LUAD | 1.565 | 1.554 | 1.576 | 0.0000 |
| LUSC | 0.690 | 0.374 | 1.275 | 0.2362 |
| MESO | 1.302 | 0.218 | 7.772 | 0.7723 |
| OV | 1.102 | 0.710 | 1.710 | 0.6650 |
| PAAD | 1.458 | 1.067 | 1.993 | 0.0180 |
| PCPG | | | | |
| PRAD | 0.564 | 0.224 | 1.420 | 0.2243 |
| READ | 1.226 | 0.854 | 1.760 | 0.2686 |
| SARC | 0.762 | 0.283 | 2.051 | 0.5899 |
| SKCM | 2.200 | 0.875 | 5.532 | 0.0939 |
| STAD | 1.001 | 0.774 | 1.294 | 0.9940 |
| TGCT | 0.969 | 0.171 | 5.483 | 0.9715 |
| THCA | | | | |
| UCEC | 0.904 | 0.685 | 1.191 | 0.4720 |
| UCS | 0.838 | 0.474 | 1.481 | 0.5430 |
| UVM | | | | |

Table 22: Within-subjects analysis for residues with high mutation frequency in THCA

| | OR | CI.low | CI.high | pvalue |
|---|---|---|---|---|
| Global | 1.363 | 1.281 | 1.451 | 0.0000 |
| ACC | | | | |
| BLCA | 0.947 | 0.425 | 2.113 | 0.8944 |
| BRCA | | | | |
| CESC | | | | |
| COAD | 1.350 | 1.071 | 1.702 | 0.0112 |
| DLBC | | | | |
| GBM | 1.026 | 0.525 | 2.004 | 0.9412 |
| HNSC | | | | |
| KICH | | | | |
| KIRC | | | | |
| KIRP | 1.397 | 0.374 | 5.223 | 0.6192 |
| LAML | 0.347 | 0.090 | 1.335 | 0.1235 |
| LGG | 1.127 | 0.558 | 2.277 | 0.7385 |
| LIHC | 2.378 | 0.484 | 11.674 | 0.2861 |
| LUAD | 1.267 | 0.750 | 2.140 | 0.3758 |
| LUSC | 0.940 | 0.373 | 2.370 | 0.8962 |

(continued)

| | OR | CI.low | CI.high | pvalue |
|---|---|---|---|---|
| MESO | | | | |
| OV | 0.790 | 0.313 | 1.992 | 0.6171 |
| PAAD | | | | |
| PCPG | 1.511 | 0.889 | 2.569 | 0.1269 |
| PRAD | 0.771 | 0.305 | 1.949 | 0.5823 |
| READ | 1.343 | 0.670 | 2.692 | 0.4056 |
| SARC | | | | |
| SKCM | 1.354 | 1.222 | 1.500 | 0.0000 |
| STAD | 0.719 | 0.223 | 2.316 | 0.5807 |
| TGCT | 0.707 | 0.281 | 1.777 | 0.4609 |
| THCA | 1.589 | 1.423 | 1.773 | 0.0000 |
| UCEC | 0.905 | 0.408 | 2.010 | 0.8073 |
| UCS | | | | |
| UVM | | | | |

Table 23: Within-subjects analysis for residues with high mutation frequency in UCEC

| | OR | CI.low | CI.high | pvalue |
|---|---|---|---|---|
| Global | 1.288 | 1.203 | 1.378 | 0.0000 |
| ACC | | | | |
| BLCA | 1.269 | 0.818 | 1.968 | 0.2881 |
| BRCA | 1.180 | 1.016 | 1.369 | 0.0302 |
| CESC | 4.522 | 1.009 | 20.268 | 0.0487 |
| COAD | 1.507 | 1.269 | 1.790 | 0.0000 |
| DLBC | | | | |
| GBM | 1.330 | 0.771 | 2.296 | 0.3057 |
| HNSC | 0.994 | 0.684 | 1.446 | 0.9763 |
| KICH | | | | |
| KIRC | | | | |
| KIRP | 2.973 | 1.065 | 8.301 | 0.0375 |
| LAML | 5.034 | 1.288 | 19.671 | 0.0201 |
| LGG | 1.223 | 0.588 | 2.546 | 0.5899 |
| LIHC | 3.518 | 0.986 | 12.547 | 0.0525 |
| LUAD | 1.561 | 1.229 | 1.983 | 0.0003 |
| LUSC | 1.265 | 0.680 | 2.355 | 0.4582 |
| MESO | | | | |
| OV | 0.886 | 0.538 | 1.459 | 0.6346 |
| PAAD | 1.654 | 1.360 | 2.013 | 0.0000 |
| PCPG | | | | |
| PRAD | 0.965 | 0.464 | 2.009 | 0.9252 |

(continued)

| | OR | CI.low | CI.high | pvalue |
|---|---|---|---|---|
| READ | 1.405 | 1.040 | 1.898 | 0.0268 |
| SARC | 0.573 | 0.189 | 1.733 | 0.3241 |
| SKCM | 2.500 | 0.550 | 11.370 | 0.2356 |
| STAD | 1.287 | 0.970 | 1.706 | 0.0801 |
| TGCT | 1.493 | 0.524 | 4.255 | 0.4527 |
| THCA | | | | |
| UCEC | 0.965 | 0.863 | 1.078 | 0.5258 |
| UCS | 0.881 | 0.619 | 1.253 | 0.4802 |
| UVM | | | | |

Table 24: The cohort of cancer-associated substitution mutations used in the present study

| Gene | Residue | Gene | Residue | Gene | Residue | Gene | Residue |
|---|---|---|---|---|---|---|---|
| BRAF | V600E | NRAS | Q61L | ATM | R337C | TP53 | A159V |
| IDH1 | R132H | TP53 | Y163C | TP53 | G245D | SMAD4 | R361C |
| PIK3CA | H1047R | EGFR | L858R | GNAS | R201H | PIK3CA | R93Q |
| PIK3CA | E545K | KRAS | G12S | ERBB2 | V842I | FBXW7 | R689W |
| KRAS | G12D | TP53 | M237I | IDH2 | R172K | TP53 | P278S |
| KRAS | G12V | TP53 | R158L | CTNNB1 | S37C | PIK3R1 | G376R |
| TP53 | R175H | FGFR2 | S252W | PIK3CA | R108H | FGFR2 | N549K |
| PIK3CA | E542K | ERBB3 | V104M | TP53 | H214R | ERBB2 | L755S |
| TP53 | R273C | FBXW7 | R505G | PIK3CA | Q546K | CTNNB1 | G34R |
| TP53 | R248Q | TP53 | I195T | KRT15 | V205I | BRAF | K601E |
| NRAS | Q61R | CTNNB1 | S37F | NFE2L2 | R34G | CTNNB1 | S33Y |
| KRAS | G12C | PPP2R1A | P179R | SMAD4 | R361H | PIK3CA | H1047Y |
| TP53 | R273H | KRAS | Q61H | PIK3CA | M1043I | SF3B1 | R625H |
| TP53 | R282W | RAC1 | P29S | TP53 | C238Y | IDH2 | R140Q |
| TP53 | R248W | PIK3CA | C420R | TP53 | L194R | HRAS | Q61K |
| NRAS | Q61K | TP53 | Y234C | TP53 | C238F | TP53 | G245C |
| KRAS | G13D | EGFR | A289V | CTNNB1 | S45F | TP53 | V216M |
| TP53 | Y220C | CTNNB1 | S45P | TP53 | E286K | PPP6C | R264C |
| PIK3CA | R88Q | PIK3CA | Q546R | TP53 | R280K | TP53 | H193Y |
| IDH1 | R132C | BCOR | N1459S | PIK3CA | E545A | TP53 | R110L |
| AKT1 | E17K | TP53 | V272M | TP53 | C141Y | TP53 | A159P |
| BRAF | V600M | TP53 | S241F | TP53 | G266V | TP53 | C242F |
| PTEN | R130Q | PIK3CA | G118D | MAP2K1 | P124S | FBXW7 | R505C |
| KRAS | G12A | KRAS | A146T | TP53 | R337C | TP53 | P250L |
| TP53 | G245S | TP53 | K132N | NFE2L2 | D29H | TP53 | H193L |
| TP53 | H179R | CTNNB1 | T41A | SF3B1 | K700E | HRAS | G13V |
| KRAS | G12R | EGFR | G598V | TP53 | P151S | CIC | R215W |
| PTEN | R130G | TP53 | E285K | KRAS | G13C | EP300 | D1399N |
| FBXW7 | R465C | MB21D2 | Q311E | IDH1 | R132G | TP53 | P152L |
| PIK3CA | N345K | TP53 | C176Y | CDKN2A | P114L | KRAS | Q61L |
| TP53 | V157F | PIK3CA | E453K | TP53 | E271K | PIK3CA | K111E |
| ERBB2 | S310F | TP53 | R280T | TP53 | V173L | CTNNB1 | T41I |
| HRAS | Q61R | TP53 | R158H | TP53 | V173M | TP53 | S127F |
| PIK3CA | H1047L | TP53 | Y205C | CDKN2A | H83Y | SOX17 | S403I |
| TP53 | H193R | TP53 | Y236C | ERBB2 | R678Q | BRAF | G469A |

(continued)

| Gene | Residue | Gene | Residue | Gene | Residue | Gene | Residue |
|---|---|---|---|---|---|---|---|
| TP53 | R249S | FBXW7 | R479Q | NRAS | G12D | PIK3CA | Q546P |
| TP53 | R273L | TP53 | C275Y | CTNNB1 | S33C | CDKN2A | D108Y |
| FBXW7 | R465H | TP53 | G245V | TP53 | H179Y | PIK3CA | Y1021C |
| TP53 | C176F | GNAS | R201C | CTNNB1 | S33F | TP53 | G262V |
| PIK3CA | E726K | PPP2R1A | R183W | MAPK1 | E322K | NFE2L2 | E79Q |
| DNMT3 A | R882H | SPOP | W131G | PTEN | R173H | PIK3CA | E545G |
| CHD4 | R975H | NRAS | Q61H | PIK3CA | R38H | BTBD 11 | A561V |
| TP53 | G266R | MYC | S146L | ABCB1 | R467W | KCND3 | S438L |
| PTEN | R173C | CTNNB1 | S33P | MS4A8 | S3L | CTNNB1 | R587Q |
| RRAS2 | Q72L | CTNNB1 | D32Y | TP53 | R175G | CTNNB1 | G34V |
| CTNNB 1 | D32G | SF3B1 | R625C | MYH2 | R1051C | PPP2R1A | S256F |
| PIK3CA | E81K | TP53 | P278L | NFE2L2 | R34P | CHD4 | R1105W |
| CTNNB 1 | G34E | FLT3 | D835Y | KRAS | L19F | PIK3CA | R93W |
| PIK3CA | M1043V | MYCN | P44L | DKK2 | R230H | GRM5 | S406L |
| TP53 | R249G | MTOR | S2215Y | KRAS | Q61R | ERBB2 | V777L |
| TP53 | G266E | MAX | R60Q | GATA3 | A395T | ACADS | R330H |
| LUM | E240K | NFE2L2 | E82D | TP53 | A161T | PIK3R1 | L56V |
| IDH1 | R132S | CHD4 | R1338I | CREBBP | R1446C | CTNNB1 | K335I |
| HRAS | G13R | NFE2L2 | E79K | TP53 | G244C | PIK3CA | E542A |
| TP53 | C135Y | NRAS | G13D | TP53 | R249M | HRAS | G12D |
| TP53 | R213Q | RAC1 | A159V | TP53 | R273S | RHOA | E40Q |
| TP53 | P278A | GRXCR1 | R262Q | TP53 | K132R | PIK3CA | G1049R |
| TP53 | C275F | TP53 | I195F | TP53 | P151H | EGFR | L861Q |
| TP53 | D281Y | ZNF117 | R185I | CASP8 | R233W | CSMD3 | R100Q |
| CDKN2A | D84N | EGFR | L62R | TP53 | S215R | SPOP | F133V |
| PIK3R1 | N564D | FGFR2 | C382R | TP53 | P278R | LHFPL1 | R69C |
| PTEN | G132D | PIK3CA | E545Q | TP53 | R280G | CSMD3 | R334Q |
| TP53 | G279E | RHOA | E47K | MAP3K1 | S1330L | KRAS | K117N |
| TP53 | R248L | PIK3CA | V344M | FBXW7 | S582L | EGFR | R108K |
| TP53 | R337L | EGFR | R222C | TP53 | P278T | EGFR | V774M |
| TP53 | G154V | TP53 | H193P | TP53 | G105C | CAPRIN2 | E13K |
| SMARCA4 | R1192C | CTNNB1 | D32V | TP53 | Q331H | TP53 | D281E |
| ARID2 | S297F | PTEN | C136R | DNMT3A | R882C | PTEN | P246L |
| TP53 | G244S | TP53 | S241Y | TP53 | D259Y | TP53 | L130V |
| TP53 | S241C | TP53 | Y163H | TP53 | R156P | SMARCA4 | T910M |
| TP53 | G244D | SMARCA4 | R1192H | SF3B1 | E902K | FUBP1 | R430C |
| PIK3CA | G106V | TP53 | K132E | EGFR | R252C | SMARCA4 | G1232S |
| HRAS | Q61L | ARID2 | R314C | KCNQ5 | G273E | TP53 | E224D |
| HRAS | G12S | TP53 | V274F | CSMD3 | P258S | TP53 | E286G |
| MBOAT2 | R43Q | TP53 | N239D | SPOP | F133L | FBXW7 | G423V |
| TP53 | R283P | TP53 | P190L | ZNF117 | R157I | CTCF | R377C |
| NRAS | G13R | PIK3CA | R38C | CHD4 | R1162W | TP53 | R267W |
| BRAF | D594N | MTOR | E1799K | PTPN 11 | G503V | CREBBP | R1446H |
| CTNNB1 | D32N | TP53 | Q136E | MFGE8 | D170N | TP53 | C135F |
| BRAF | G466V | INTS7 | R106I | NFE2L2 | G31A | CASP8 | R68Q |
| TUSC3 | R334C | TP53 | R175C | KRAS | Q61K | BRAF | N581S |
| CDKN2A | P48L | PGM5 | T442M | APC | S2307L | SMAD2 | R120Q |
| CTNNB1 | S37A | BRAF | G469V | TP53 | D281V | ATM | R337H |
| EGFR | E114K | NSMCE1 | D244N | TP53 | V216L | TP53 | G334V |
| MYD88 | L265P | COL4A2 | R1410Q | RASA1 | R194C | TP53 | S215I |

(continued)

| Gene | Residue | Gene | Residue | Gene | Residue | Gene | Residue |
|---|---|---|---|---|---|---|---|
| MYH2 | R1388H | ABCB1 | R41C | KMT2C | R56Q | PTEN | D92E |
| NFE2L2 | D29G | TP53 | N239S | MAP2K4 | S184L | CHD8 | F668L |
| NFE2L2 | D29N | NOTCH1 | A465T | PTEN | G165E | FBXW7 | R14Q |
| BRAF | G466E | CIC | R202W | MYO6 | R928H | EP300 | R580Q |
| NFE2L2 | D29Y | PIK3CA | K111N | TP53 | G105V | DNMT3A | R736H |
| MYH2 | E1421K | MFGE8 | E168K | TGFBR2 | R528H | CIC | R1515C |
| NFE2L2 | L30F | KCNQ5 | R426C | SMAD4 | D537H | TP53 | S106R |
| PIK3CA | E453Q | PIK3CA | G1007R | TP53 | P151T | TP53 | H179N |
| RIT1 | M90I | TP53 | F270S | TP53 | C135W | TP53 | Y220S |
| TRIM23 | R289Q | TP53 | R280I | BCOR | E1076K | PTEN | R130P |
| TP53 | R213L | TP53 | L265P | CDKN2A | D108N | ZC3H13 | R1261Q |
| MAP3K1 | R306H | TP53 | T155N | SMARCA | 4 E920K | CHD8 | R1092C |
| LZTR1 | G248R | TP53 | H179D | NOTCH1 | E455K | FAT1 | K2413N |
| MAX | H28R | TP53 | T155P | KEAP1 | G480W | ZFP36L2 | D240N |
| KEAP1 | R470C | TP53 | R267P | TP53 | E258K | TP53 | E286Q |
| TP53 | C141W | TP53 | A161S | TP53 | Y205S | CIC | R215Q |
| FAT1 | E4454K | PBRM1 | R876C | TP53 | D281H | NOTCH1 | G310R |
| ERBB3 | D297Y | ARID1A | G2087R | TGFBR2 | R528C | TP53 | C242S |
| PPP2R1A | R183Q | TP53 | D259V | TRIP12 | A761V | PTEN | H93R |
| CTNNB1 | H36P | PTEN | R130L | NF1 | R1306Q | TP53 | V272G |
| LSM11 | R180W | CIC | R201W | PTEN | G129E | PTEN | R142W |
| ABCB1 | R404Q | TP53 | C277F | TP53 | C242Y | ARHGAP35 | V1317M |
| PTPN11 | T468M | ERBB2 | D769Y | TP53 | M246I | TP53 | F109C |
| ERBB3 | E332K | PIK3CA | E365K | KEAP1 | V271L | CDKN2A | M53I |
| EGFR | A289T | INTS7 | R940C | CTCF | S354F | TRIP12 | S1840L |
| EGFR | A289D | CSMD3 | R3127Q | TP53 | Y126C | PTEN | S170N |
| ERBB3 | E928G | NFE2L2 | R34Q | PIK3R1 | K567E | TP53 | L130F |
| CTNNB1 | I35S | EP300 | A1629V | NF2 | R418C | TP53 | N131I |
| CTNNB1 | S45Y | PIK3CA | V344G | ATRX | R781Q | TP53 | T211I |
| PIK3CA | D350G | MAP2K4 | R134W | NF1 | R1276Q | STAG2 | V465F |
| NRAS | G12C | PIK3CA | N1044K | SETD2 | R2109Q | TP53 | P151R |
| MYH2 | E1382K | TP53 | R273P | TP53 | H193N | ARID2 | R285Q |
| RAC1 | P29L | CIC | R1512H | TP53 | S127Y | CDK12 | R890H |
| PIK3CA | E600K | NF1 | R1870Q | SMARCA | 4 R885C | TP53 | P177R |
| PIK3CA | C901F | TP53 | G199V | TP53 | F134L | RUNX1 | R177Q |
| CSMD3 | S 1090Y | KANSL1 | A7T | TP53 | I195N | FAT1 | R881H |
| ERBB3 | V104L | TGFBR2 | E519K | FBXW7 | Y545C | TAF1 | R843W |
| MYCN | R302C | SPOP | F102V | RRAS2 | A70T | CRIPAK | R430C |
| CSMD3 | R683C | TUSC3 | F66V | KMT2D | R5351L | TP53 | L257Q |
| CSMD3 | R1529H | BTBD11 | K1003T | KMT2D | R5432Q | EP300 | Y 1414C |
| MYH2 | D756N | PIK3CA | E542G | CDKN2A | D84Y | TP53 | V218G |
| MYH2 | R793Q | KCNQ5 | R909Q | CHD8 | R578H | CREBBP | P2094L |
| HRAS | G13D | BRAF | V600G | ARID1B | P1411Q | DDX3X | E285K |
| ERBB3 | M91I | CTNNB1 | D32H | CCAR1 | R549C | TP53 | Y205H |
| MAP2K1 | P124L | ERBB2 | S310Y | TP53 | V143M | APC | E136K |
| BRAF | G469R | GRXCR1 | R19Q | TP53 | C176S | TP53 | R181H |
| SPOP | F133C | UBQLN2 | S196L | CHD8 | R1889H | PTEN | H123Y |
| SF3B1 | R425Q | MYF5 | E104K | EP300 | C1164Y | PIK3R1 | G353W |
| KCNQ5 | T693M | PIK3CA | M1004I | KEAP1 | R554Q | PTEN | C136F |
| PRKCI | R480C | FAM8A1 | E94K | ELF3 | E262Q | APC | S2601R |

(continued)

| Gene | Residue | Gene | Residue | Gene | Residue | Gene | Residue |
|---|---|---|---|---|---|---|---|
| CSMD3 | G1941E | EZH2 | E740K | PBRMI | M1487I | KMT2C | H367Y |
| MED12 | L1224F | HRAS | K117N | ARHGAP35 | R1147H | CASP8 | S99F |
| CSMD3 | P184S | GNAS | R356C | KANSL1 | R891L | TP53 | V157D |
| DCLK1 | R60C | CTCF | R377H | EP300 | S964Y | ATRX | L14F |
| ERBB2 | I767M | ATM | S2812Y | PTEN | C124S | ATM | R2691C |
| METTL14 | R298P | PGM5 | T476M | TP53 | V172F | NCOR1 | G1801V |
| EGFR | T263P | PTEN | P38S | KMT2B | E324K | ATM | R23Q |
| PIK3CA | D939G | SPOP | M117V | NCOR1 | P1081L | TP53 | V143G |
| FLT3 | R387Q | TRIM23 | N92I | KMT2C | G3665A | ACVR2A | R400H |
| MAGI2 | L114V | CAPRIN2 | R215Q | CASP8 | I333M | TET2 | A347V |
| LUM | E187K | MAP2K1 | K57N | TRIP12 | E1803K | NSD1 | A2144T |
| SULT1C4 | R85Q | LZTR1 | F243L | CHD8 | S1632L | MLLT4 | S1510N |
| MYH2 | E878K | FGFR2 | M537I | ELF3 | P30S | STK11 | G242W |
| ERBB3 | A245V | ZNF799 | R297Q | THRAP3 | R504W | KMT2C | F357L |
| DKK2 | E226K | PIK3CA | E39K | TP53 | Y220H | SETD2 | R1625C |
| MYF5 | E27K | DCLK1 | R45C | KMT2C | W430C | APC | S1400L |
| KRAS | A59T | ABCB1 | S696F | KMT2B | R1597Q | SETD2 | H1629Y |
| GRXCR1 | R190Q | CSMD3 | G1195W | PIK3R1 | L573P | CHD8 | N2372H |
| EP300 | R1627W | HIST1H2B | F E77K | KMT2C | D4425Y | KANSL1 | R1066H |
| CAPRIN2 | E905K | PIK3CA | E418K | SETD2 | R2077Q | ASXL1 | A611T |
| MAP2K1 | E203K | BRAF | S467L | TCF12 | R589H | NF1 | L844F |
| IDH1 | P33S | PIK3CA | R357Q | TP53 | A161D | SMARCA4 | R381Q |
| CHD4 | R1105Q | PIK3CA | E970K | KEAP1 | V155F | VHL | H115N |
| PIK3CA | N345T | MYC | P59L | FAT1 | R1627Q | NOTCH2 | R1726C |
| MYH2 | R1506Q | ERBB3 | R475W | NF1 | P1990Q | KANSL1 | E647K |
| DCLK1 | A18V | TAF1 | R539Q | PBRMI | R1096C | CDKN1A | D33N |
| MYH2 | R1668W | TUSC3 | R82Q | FBXW7 | R479G | KMT2D | R5214C |
| MFAP5 | R153C | MYH2 | E347K | TP53 | V274G | NOTCH1 | A1918T |
| ATM | G1663C | TP53 | D281N | TP53 | R158G | IDH1 | R132L |
| ATM | L1408I | MEN1 | W428L | RASA1 | R194H | NFE2L2 | G81C |
| CDH1 | E243K | ZC3H13 | R453Q | TP53 | I255F | FGFR2 | K659N |
| PTEN | G129V | USP28 | R141C | TP53 | L194H | FGFR2 | K659E |
| TP53 | L111P | VHL | N131K | TP53 | R248P | MS4A8 | A183V |
| ATM | N2875S | TP53 | R196P | VHL | R205C | PPP2R1A | A273V |
| SMARCB1 | R374W | BAP1 | V99M | USP28 | P235L | JAKMIP2 | D338N |
| LARP4B | E486K | SETD2 | R1335C | ARID1B | A987V | EGFR | T363I |
| RNF43 | S607L | TP53 | K120E | GATA3 | S407L | CSMD3 | L2481I |
| TP53 | H179L | ARID1B | D1734E | TP53 | A276D | CSMD3 | P3166H |
| NCOR1 | R330W | CDK12 | S475Y | WT1 | R462L | CTNNB1 | N387K |
| MYO6 | A91T | PTEN | T277I | SMARCA4 | E882K | CSMD3 | E531K |
| KMT2C | A135T | NOTCH1 | R353C | ACVR2A | R478I | SPOP | W131C |
| STAG2 | A300V | TP53 | I232T | TP53 | F134V | ZNF844 | D436N |
| KDM6A | R1255W | CDK12 | R1008W | VHL | L128H | JAKMIP2 | A334T |
| TP53 | V274D | KMT2D | R5214H | VHL | V74D | KRAS | A59G |
| KANSL1 | S808L | CREBBP | A259T | KMT2B | H1226Y | RIT1 | R86L |
| GATA3 | M293K | COL4A2 | R1651C | TP53 | S215G | EGFR | S645C |
| CASP8 | R248W | THRAP3 | R723H | TBX3 | E275K | CHD4 | R877W |
| NCOR1 | R2214C | ATM | R3008H | TP53 | M237V | MYH2 | R1181C |
| FBXW7 | R505L | TP53 | I232S | ARID1A | R1262C | MTOR | P2158Q |
| TP53 | T125M | APC | G1767C | CREBBP | W1472C | ALK | R292C |

(continued)

| Gene | Residue | Gene | Residue | Gene | Residue | Gene | Residue |
|---|---|---|---|---|---|---|---|
| GATA3 | R305Q | TP53 | R280S | FAT1 | T3356M | ARF4 | R99I |
| SETD2 | R2024Q | NCOR1 | K482N | CDKN2A | D84G | SF3B1 | E862K |
| TP53 | A138V | TP53 | E271V | TP53 | R249W | MYH2 | R1787Q |
| TP53 | S215N | TP53 | C141G | APC | S1696N | KCND3 | V94M |
| TP53 | E285V | KMT2B | R2332C | TP53 | Y126D | CTNNB1 | A391S |
| ELF3 | R126Q | TP53 | E258D | ACVR2A | E214K | COL5A2 | R1453W |
| TP53 | K139N | APC | S2026Y | TP53 | Y126N | IDH2 | R172M |
| ZC3H18 | R520C | TP53 | E171K | CDKN2A | P81L | ABCB1 | R489C |
| FBXW7 | R658Q | ARID2 | P1590Q | SMAD4 | D537E | NFE2L2 | T80K |
| TP53 | K164E | PTEN | C71Y | TP53 | C176W | KCNQ5 | A704V |
| TP53 | C135R | CCAR1 | R383H | FAT1 | R1506C | KCNQ5 | R187Q |
| ARHGAP35 | R863C | TP53 | P27S | PTEN | C136Y | TAF1 | A445V |
| MYO6 | R1169H | HLA-A | R243W | FAT1 | A2289V | OR5I1 | S95F |
| TP53 | G245R | COL4A2 | P123Q | PTEN | G165R | MYH2 | E868K |
| DDX3X | R263H | CDH1 | R732Q | ARID2 | V179I | TAF1 | A1287V |
| CDH1 | D254Y | RERE | K176N | GATA3 | M442I | PTN | E130K |
| MEN1 | R337H | TP53 | P151A | ERBB3 | R103H | LUM | G248E |
| TP53 | L265R | VHL | S111N | KMT2B | R2567C | ABCB1 | R41H |
| RB1 | R451C | RPL22 | R113C | PTPN 11 | D146Y | PTPN 11 | F71L |
| TUSC3 | H189N | MYH2 | S337R | FAM8A1 | E94Q | MS4A8 | A91V |
| COL5A2 | A592V | CHD4 | R572Q | SPOP | Y87C | GRXCR1 | G91S |
| MAGI2 | L450M | GNAS | R389C | TAF1 | R1442L | MBOAT2 | E147K |
| HRAS | G13C | MAGI2 | L603R | CSMD3 | T2652M | UBQLN2 | S62L |
| BTBD11 | R421C | FGFR2 | R210Q | MYH2 | R709H | ABCB1 | R286I |
| MYH2 | P228L | GRM5 | R128C | SF3B1 | V1192A | TAF1 | R342C |
| CSMD3 | G2578E | EGFR | S229C | PPP6C | E180K | PPP2R1A | R258H |
| MYF5 | R93Q | CHD4 | R1177H | ALK | G452W | TBX18 | S206L |
| UBQLN2 | R309S | CSMD3 | R1946C | GRXCR1 | R191Q | AKT1 | L52R |
| TBX18 | H401Y | CSMD3 | R2168Q | ABCB1 | E468K | PPP2R1A | W257L |
| JAKMIP2 | E155K | MYCN | R373Q | KCNQ5 | S280L | CSMD3 | M729I |
| PTN | E68D | CSMD3 | E171K | KCND3 | E626K | MTOR | T1977R |
| HGF | R178Q | CHD4 | F1112L | RHOA | F106L | MFGE8 | A280V |
| CSMD3 | G165R | GRM5 | R834C | EZH2 | R679H | GRID1 | R221W |
| KCND3 | T231M | SPOP | R121Q | PIK3CA | D725G | GRID1 | R631H |
| KCNQ5 | E455K | NFE2L2 | G81V | CSMD3 | L2370I | BTBD 11 | G699E |
| XYLT1 | E804K | MBOAT2 | R170C | SF3B1 | K666T | COL5A2 | D1241N |
| SF3B1 | G740E | PIK3CA | E542V | MTOR | I2500F | CTNNB1 | R515Q |
| PIK3CA | H1047Q | PIK3CA | R115L | MTOR | I2500M | METTL14 | R228Q |
| KRTAP4-11 | R41H | FGFR2 | E777K | SMAD2 | R321Q | RHOA | E172K |
| CSMD3 | R2231Q | MTOR | R2152C | TP53 | M246V | KRT15 | G232S |
| PLK2 | F363L | NFE2L2 | W24R | EP300 | E1514K | PIK3CA | C604R |
| GNAS | A109T | SPOP | E50K | CDH1 | R598Q | ERBB2 | G222C |
| GNAS | R160C | CSMD3 | R3025C | TP53 | F113C | CSMD3 | G742E |
| CAPRIN2 | R727Q | COL5A2 | D1414N | SMARCA | 4 R1243W | PTPN 11 | Q510L |
| PIK3CA | P539R | MYF5 | R129C | CTCF | P378L | SPOP | E47K |
| PDE7B | E11K | CTNNB1 | S33A | DDX3X | R528C | CSMD3 | D285N |
| TRIM48 | M17I | PIK3CA | C378F | SMARCA | 4 A1186V | ABCB1 | R1085W |
| PIK3CA | P471L | GRXCR1 | R14Q | DNMT3A | R659H | PTPN 11 | R512Q |
| DCLK1 | R93Q | PTPN 11 | R498W | PTEN | R14M | RHOA | R5W |
| LUM | R330C | CDKN2A | E88K | TP53 | P278H | RHOA | Y42C |

(continued)

| Gene | Residue | Gene | Residue | Gene | Residue | Gene | Residue |
|---|---|---|---|---|---|---|---|
| ERBB3 | T355I | MYH2 | S1741F | KMT2C | R4693Q | MYH2 | E900K |
| ERBB3 | A232V | MED12 | E79D | EGFR | R252P | RHOA | G62E |
| TRIM23 | R549Q | OR5I1 | R231C | PTEN | G36R | PIK3CA | M1004V |
| SF3B1 | R957Q | MAGI2 | P876S | SMAD2 | S276L | BRAF | H725Y |
| TAF1 | R1221Q | JAKMIP2 | R283I | FBXW7 | R505H | TRIM48 | E28K |
| PPP2R1A | S256Y | DCLK1 | R80W | TGFBR2 | D446N | KRT15 | E455K |
| PIK3CA | D350N | EGFR | S752F | GRXCR1 | R147C | GRM5 | T906P |
| MED12 | D23Y | ABCB1 | G610E | MAGI2 | D843N | GRID1 | S388L |
| CHD4 | R1068C | PRKCI | R278C | ORSI1 | L294F | CSMD3 | R395Q |
| PIK3CA | T1025A | TUSC3 | R170I | TAF1 | R1163H | HGF | E199K |
| FGFR2 | R664W | EGFR | H304Y | NFE2L2 | W24C | XYLT1 | R754H |
| ABCB1 | R958Q | PTPN 11 | G409W | ORSI1 | S89L | TP53 | I254S |
| MB21D2 | R288W | MYH2 | M858I | CSMD3 | E2280K | | |
| MTOR | F1888L | CSMD3 | R3551C | XYLTI | R754C | | |
| PIK3CA | G364R | PIK3CA | D186H | PIK3CA | P104L | | |

Table 25: The Cohort of Cancer-Associated In-Frame Insertion and Deletion Mutations used in the Present Study

| | | | | | | | | |
|---|---|---|---|---|---|---|---|---|
| EGFR | 745 | In_Frame_Del | EGFR | 746 | In_Frame_Del | EGFR | 766 | In_Frame_Ins |
| NOTCH1 | 357 | In_Frame_Del | PIK3R1 | 450 | In_Frame_Del | PIK3CA | 446 | In_Frame_Del |
| PIK3R1 | 575 | In_Frame_Del | BRAF | 486 | In_Frame_Del | MAP2K1 | 101 | In_Frame_Del |
| CTNNB1 | 44 | In_Frame_Del | TP53 | 177 | In_Frame_Del | EGFR | 709 | In_Frame_Del |
| PIK3R1 | 462 | In_Frame_Del | PIK3R1 | 566 | In_Frame_Del | EGFR | 767 | In_Frame_Ins |
| ERBB2 | 770 | In_Frame_Ins | PIK3CA | 111 | In_Frame_Del | PIK3R1 | 575 | In_Frame_Del |

**Example 5: Materials and Methods**

*Peptide Binding Affinity*

Peptide binding affinity predictions for peptides of length 8-11 were obtained for various HLA alleles using the NetMHCPan-3.0 tool, downloaded from the Center for Biological Sequence Analysis on March 21, 2016 (Nielsen and Andreatta, Genome Med., 2016, 8, 33). NetMHCPan-3.0 returns $IC_{50}$ scores and corresponding allele-based ranks, and peptides with rank < 2 and < 0.5 are considered to be weak and strong binders respectively (Nielsen and Andreatta, Genome Med., 2016, 8, 33). Allele-based ranks were used to represent peptide binding affinity.

*Residue Presentation Scoring Schemes*

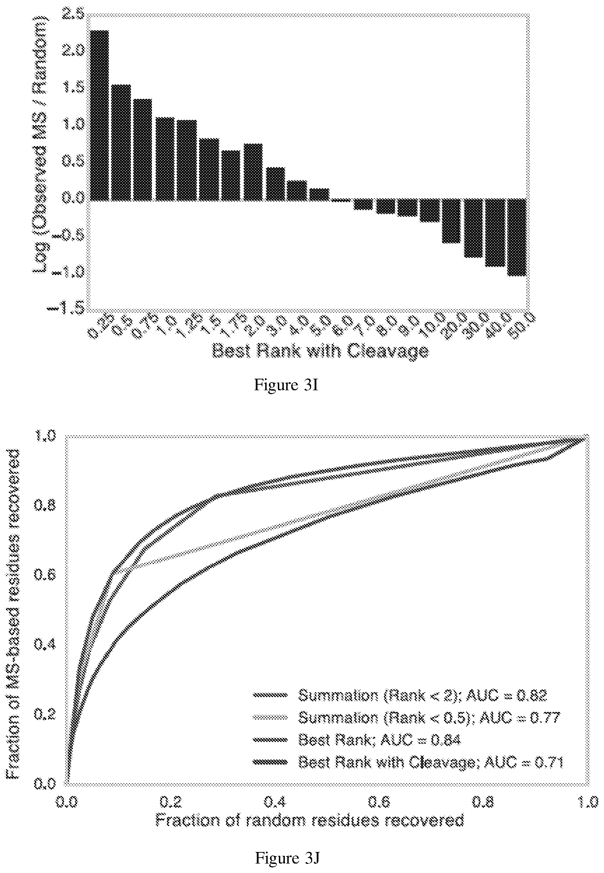

To create a residue-centric presentation score, allele-based ranks for the set of kmers of length 8-11 incorporating the residue of interest were evaluated, resulting in 38 peptides for single amino acid positions (Figure 2A). Insertion and deletion mutations were modeled by the total number of 8-11-mer peptides differing from the native sequence (Figure 3J). Several approaches to combine the HLA allele-specific ranks for residue/mutation-derived peptides into a single score representing the likelihood of being presented by MHC-I were evaluated:

*Summation* (rank < 2): The summation score is the total number out of 38 possible peptides that had rank < 2. This scoring system results in an integer value from 0 to 38, with residues of 0 being very unlikely to be presented and higher numbers being more likely to be presented.
*Summation* (rank < 0.5): The summation score is the total number out of 38 possible peptides that had rank < 0.5. This scoring system results in an integer value from 0 to 38, with residues of 0 being very unlikely to be presented and higher numbers being more likely to be presented.
*Best Rank*: The best rank score is the lowest rank of all of the 38 peptides.
*Best Rank with cleavage*: The best rank score was modified by first filtering the 38 possible peptides to remove those unlikely to be generated by proteasomal cleavage as predicted by the NetChop tool (Kesxmir et al., Protein Eng., 2002, 15, 287-296). Netchop relies on a neural network trained on observed MHC-I ligands cleaved by the human proteasome and returns a cleavage score ranging between 0 and 1 for the C terminus of each amino acid. A threshold of 0.5 is recommended by the NetChop software manual to designate peptides as likely to be generated by proteasomal cleavage. Thus, only the peptides receiving a cleavage score greater than 0.5 just prior to the first residue and just after the last residue were retained. The best rank with cleavage score is the lowest rank of the remaining peptides.

*MS-based Presentation Score Validation*

MS data was acquired from Abelin et al. (Abelin et al., Mass Immunity, 2017, 46, 315-326) that catalogs peptides observed in complex with MHC-I on the cell surface across 16 HLA alleles, with between 923 and 3609 peptides observed bound to each. These data were combined with a set of random peptides to construct a benchmark for evaluating the performance of scoring schemes for identifying residues presented on the cell surface as follows:

Converting MS peptide data to residues: The Abelin et al. MS data provides peptide observed in complex with the MHC-I, whereas the presentation score is residue-centric. For each peptide in the MS data, the residue at the center (or one residue before the center in the case of peptides of even length) was selected as the residue for calculating the residue-centric presentation score.

Selection of background peptides: 3000 residues at random were selected from the Ensembl human protein database (Release 89) (Aken et al., Nucleic Acids Res., 2017, 45 (D1), D635-D642) to ensure balanced representation of MS-bound and random residues. Since the majority of residues are expected not be presented by the MHC (Nielsen and Andreatta, Genome Med., 2016, 8, 33), the randomly selected residues may represent a reasonable approximation of a true negative set of residues that would not be presented on the cell surface.

*Scoring benchmark set residues*: Presentation scores were calculated with each scoring scheme for all of the selected residues from the Abelin et al. data and the 3000 random residues against each of the 16 HLA alleles.

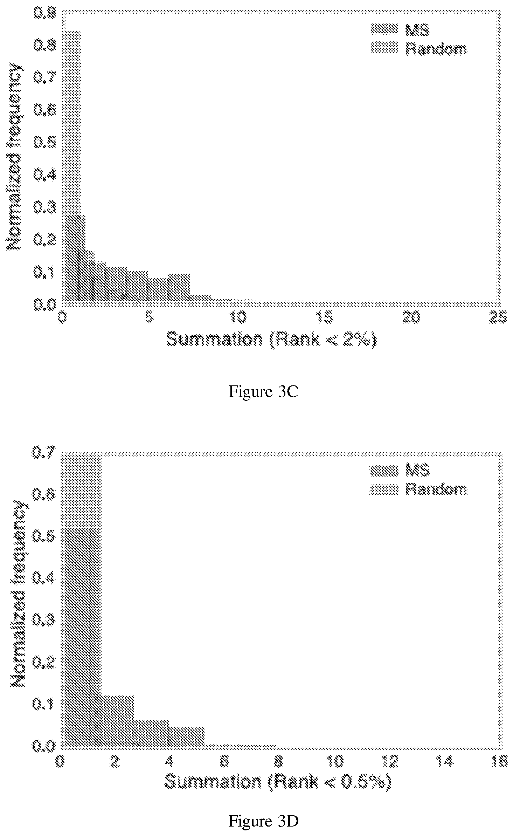

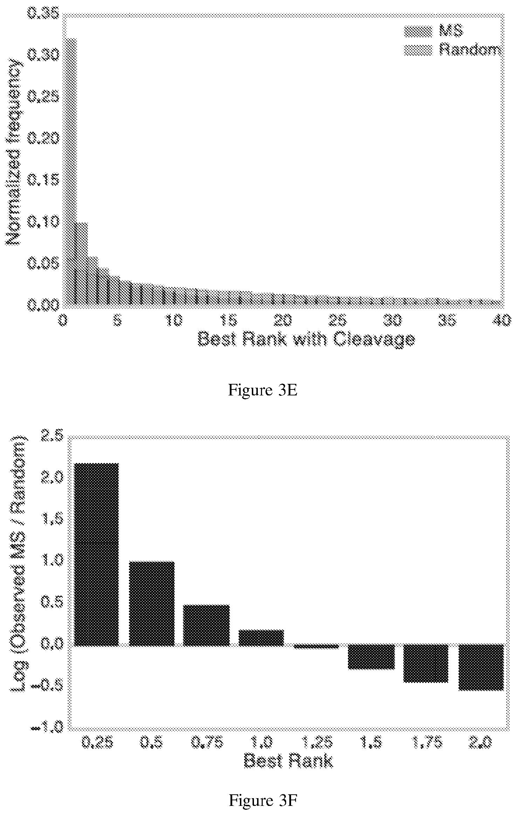

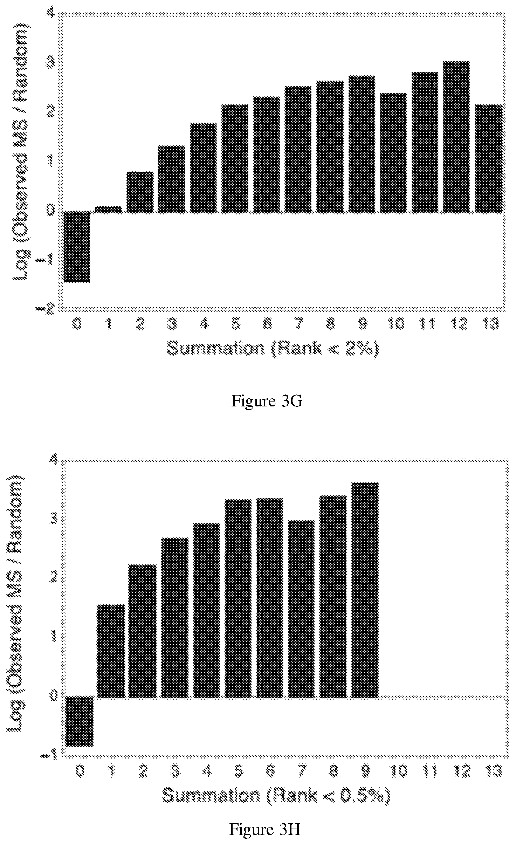

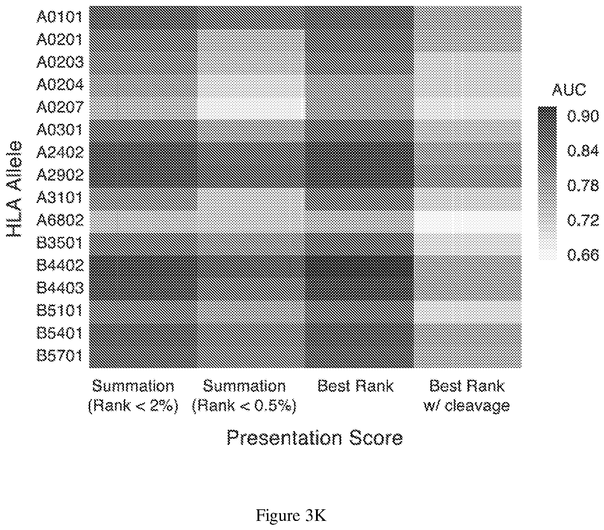

*Evaluating scoring scheme performance using the benchmark*: For each scoring scheme, scores were pooled across the 16 alleles. The distribution of scores for the MS-observed residues was compared to the distribution of scores for the random residues for each score formulation (Figure 3). For the best rank, residues were grouped at score intervals of 0.25 and for the summation, residues were grouped at integer values between 0 and 38. At each scoring interval, the fraction of MS-observed residues falling was divided into the interval by the fraction of random residues falling into that interval.

*Visualizing score performance with Receiver Operating Characteristic (ROC) Curves:* ROC curves (Figures 3J and 3K) were plotted and compared for each score formulation by calculating the True Positive Rate (% of observed MS residues predicted to bind at a given threshold) and the False Positive Rate (% of random residues predicted to bind at a given threshold) across a range of thresholds as follows:

Summation (rank < 2): 0 through 38 by increments of 1
Summation (rank < 0.5): 0 through 38 by increments of 1
Best Rank: 0 through 100 by increments of 0.1
Best Rank with Cleavage: 0 through 100 by increments of 0.1

Overall score performance was assessed using the area under the curve (AUC) statistic. The best rank presentation score was selected for all subsequent analyses.

*MS-based Evaluation of the Presentation of Mutated Residues Present in Cancer Cell Lines*

The list of somatic mutations present in the genomes of five cancer cell lines (SKOV3, A2780, OV90, HeLa and A375) was acquired from the Cosmic Cell Lines Project (Forbes et al., Nucleic Acids Res., 2015, 43, D805-D811). The mutations were restricted to the missense mutations observed in genes present in the Ensembl protein database and removed all known common germline variants reported by the Exome Variant Server. Furthermore, the cell line expression data from the Genomics of Drug Sensitivity Center was used to exclude mutations observed in genes that are expressed in the lowest quantile of the specific cell line. For each of these mutated residues, the presentation score for HLA-A*02:01, an allele which had previously been studied in these cell lines, was calculated (Method Details). Then the database of MS-derived peptides from each cell line was searched to determine whether the mutation was observed in complex with the MHC-I on the cell surface. Since the database only contains peptides mapping to the consensus human proteome reference, the native versions of the peptides were searched. As long as the mutation does not disrupt the peptide binding motif, the mutated version should still be presented by the MHC allele which can be determined using MHC binding predictions in IEDB (Marsh, S.G.E., Parham, P., and Barber, L.D., 1999, The HLA FactsBook, Academic Press). For each cell line, the fraction of mutations predicted to be strong and weak binders that should be presented based on the corresponding native sequences observed in the MS data was evaluated (see, Tables 1A, 1B, 2A, 2B, 3A, 3B, 4A, 4B, 5A, and 5B).

## Claims

**1.** A computer implemented method for determining whether a subject is at risk of having or developing a cancer, the method comprising:

a) genotyping the subject's major histocompatibility complex class I (MHC-I); and
b) scoring the ability of the subject's MHC-I to present a mutant cancer-associated peptide based upon a library of known cancer-associated peptide sequences derived from subjects, wherein the produced score is the MHC-I presentation score;

wherein:

i) if the subject is a poor MHC-I presenter of specific mutant cancer-associated peptides, the subject has an increased likelihood of having or developing the cancer for which the specific mutant cancer-associated peptides are associated; or
ii) if the subject is a good MHC-I presenter of specific mutant cancer-associated peptides, the subject has a decreased likelihood of having or developing the cancer for which the specific mutant cancer-associated peptides are associated;

wherein the step of scoring the ability of the subject's MHC-I to present a mutant cancer-associated peptide comprises using a predicted MHC-I affinity for a given mutation $x_{ij}$, where x is the MHC-I affinity of subject *i* for mutation *j* to fit a mixed-effects logistic regression model that follows a model equation obtained from a large dataset of subjects from which MHC-I genotypes and presence of peptides of interest can be obtained:

$$\text{logit}\,(P(y_{ij} = 1 \mid x_{ij})) = \eta_i + \gamma \log(x_{ij})$$

wherein:

$y_{ij}$ is a binary mutation matrix $y_{ij} \in \{0,1\}$ indicating whether a subject *i* has a mutation *j*;
$x_{ij}$ is a binary mutation matrix indicating predicted MHC-I binding affinity of subject *i* having mutation *j;*
$\gamma$ measures the effect of the log-affinities on the mutation probability; and
$\eta_i \sim N(0, \phi_\eta)$ are random effects capturing different mutation rates among subjects,
wherein the model tests the null hypothesis that $\gamma = 0$ and calculates odds ratios for MHC-I affinity of a mutation and presence of a cancer.

**2.** The method according to claim 1, further comprising:
c) determining whether a liquid biopsy sample obtained from the subject comprises DNA encoding a mutant cancer-associated peptide based upon a library of cancer-associated mutations obtained from subjects.

**3.** The method according to claim 2, wherein the liquid biopsy sample is blood, saliva, urine, or other body fluid.

**4.** The method according to claim 2, wherein the library of cancer-associated mutations is obtained by whole genome sequencing of subjects.

**5.** The method according to claim 1, wherein the predicted MHC-I affinity for a given mutation $x_{ij}$ is a Subject Harmonic-mean Best Rank (PHBR) score.

**6.** The method according to claim 5, wherein the PHBR score is obtained by aggregating MHC-I binding affinities of a set of mutant cancer-associated peptides by referring to a pre-determined dataset of peptides binding to MHC-I molecules encoded by at least 16 different HLA alleles.

**7.** The method according to claim 1, wherein the mutant cancer-associated peptide contains an amino acid substitution, insertion, or deletion, and wherein the set of peptides consists of at least 38 of all possible 8-, 9-, 10- and 11-amino acid long peptides incorporating the substitution, insertion, or deletion at every position along the peptide.

**8.** The method according to any one of claims 1 to 7, wherein the cancer is an adrenocortical carcinoma (ACC), a bladder urothelial carcinoma (BLCA), a breast invasive carcinoma (BRCA), a cervical squamous cell carcinoma and endocervical adenocarcinoma (CESC), a colon adenocarcinoma (COAD), a lymphoid neoplasm diffuse large B-cell lymphoma (DLBC), a glioblastoma multiforme (GBM), a head and neck squamous cell carcinoma (HNSC), a kidney chromophobe (KICH), a kidney renal clear cell carcinoma (KIRC), a kidney renal papillary cell carcinoma (KIRP), an acute myeloid leukemia (LAML), a brain lower grade glioma (LGG), a liver hepatocellular carcinoma (LIHC), a lung adenocarcinoma (LUAD), lung squamous cell carcinoma (LUSC), a mesothelioma (MESO), an ovarian serous cystadenocarcinoma (OV), a pancreatic adenocarcinoma (PAAD), a pheochromocytoma and paraganglioma (PCPG), a prostate adenocarcinoma (PRAD), a rectum adenocarcinoma (READ), a sarcoma (SARC), a skin cutaneous melanoma (SKCM), a stomach adenocarcinoma (STAD), a testicular germ cell tumors (TGCT), a thyroid carcinoma (THCA), a uterine corpus endometrial carcinoma (UCEC), a uterine carcinosarcoma (UCS), or a uveal melanoma (UVM).

**9.** The method according to any one of claims 6 to 8, wherein the set of mutant cancer-associated peptides comprises any one or more of B-Raf Proto-Oncogene (BRAF) V600E mutation, Phosphatidylinositol-4,5-Bisphosphate 3-Kinase Catalytic Subunit Alpha (PIK3CA) E545K mutation, PIK3CA E542K mutation, PIK3CA H1047R mutation, Kirsten Rat Sarcoma Viral Oncogene Homolog (KRAS) G12D mutation, KRAS G13D mutation, KRAS G12V mutation, KRAS A146T mutation, TP53 R175H mutation, TP53 H179R mutation, TP53 mutation, TP53 R248Q mutation, TP53 R273C mutation, TP53 R273H mutation, TP53 R282W mutation, Keratin Associated Protein 4-11 (KRTAP4-11) L161V mutation, Mab-21 Domain Containing 2 (MB21D2) Q311E mutation, HLA-A Q78R mutation, Harvey Rat Sarcoma Viral Oncogene Homolog (HRAS) G13V mutation, Isocitrate Dehydrogenase (NADP(+)) 1 (IDH1) R132H mutation, IDH1 R132C mutation, IDH1 R132G mutation, IDH2 R172K mutation, IDH1 R132S mutation, Capicua Transcriptional Repressor (CIC) R215W mutation, Phosphoglucomutase 5 (PGM5) I98V mutation, Tripartite Motif Containing 48 (TRIM48) Y192H mutation, and F-Box And WD Repeat Domain Containing 7 (FBXW7) R465C mutation, wherein the presence of any one of these mutations indicates the presence of or increased risk of developing breast invasive carcinoma.

**10.** The method according to any one of claims 6 to 8, wherein the set of mutant cancer-associated peptides comprises any one or more of BRAF V600E mutation, Neuroblastoma RAS Viral Oncogene Homolog (NRAS) Q61R mutation, NRAS Q61K mutation, NRAS Q61L mutation, IDH1 R132S mutation, Mitogen-Activated Protein Kinase Kinase 1 (MAP2K1) P124S mutation, Rac Family Small GTPase 1 (RAC1) P29S mutation, Protein Phosphatase 6 Catalytic Subunit (PPP6C) R301C mutation, Cyclin Dependent Kinase Inhibitor 2A (CDKN2A) P114L mutation, Keratin Associated Protein 4-11 (KRTAP4-11) L161V mutation, KRTAP4-11 M93V mutation, HRAS Q61R mutation, HLA-A Q78R mutation, Zinc Finger Protein 799 (ZNF799) E589G mutation, Zinc Finger Protein 844 (ZNF844) R447P mutation, and RNA Binding Motif Protein 10 (RBM10) E184D mutation, wherein the presence of any one of these mutations indicates the presence of or increased risk of developing colon adenocarcinoma.

**11.** The method according to any one of claims 6 to 8, wherein the set of mutant cancer-associated peptides comprises any one or more of IDH1 R132H mutation, IDH1 R132C mutation, IDH1 R132G mutation, IDH1 R132S mutation, IDH2 R172K mutation, TP53 H179R mutation, TP53 R273C mutation, TP53 R273H mutation, CIC R215W mutation, and HLA-A Q78R mutation, wherein the presence of any one of these mutations indicates the presence of or increased risk of developing head and neck squamous cell carcinoma or brain lower grade glioma.

**12.** The method according to any one of claims 6 to 8, wherein the set of mutant cancer-associated peptides comprises any one or more of BRAF V600E mutation, PIK3CA E545K mutation, KRAS G12D mutation, KRAS G13D mutation, KRAS A146T mutation, TP53 R175H mutation, KRAS G12V mutation, TP53 R248Q mutation, TP53 R273C mutation TP53 R273H mutation, TP53 R282W mutation, PGM5 I98V mutation, TRIM48 Y192H mutation, PIK3CA E545K mutation, KRAS G13D mutation, PIK3CA H1047R mutation, and FBXW7 R465C mutation, wherein the presence of any one of these mutations indicates the presence of or increased risk of developing lung adenocarcinoma.

**13.** The method according to any one of claims 6 to 8, wherein the set of mutant cancer-associated peptides comprises any one or more of PIK3CA H1047R mutation, PIK3CA E545K mutation, PIK3CA E542K mutation, TP53 R175H mutation, PIK3CA N345K mutation, AKT Serine/Threonine Kinase 1 (AKT1) E17K mutation, Splicing Factor 3b Subunit 1 (SF3B1) K700E mutation, and PIK3CA H1047L mutation, wherein the presence of any one of these mutations indicates the presence of or increased risk of developing lung squamous cell carcinoma.

14. The method according to any one of claims 6 to 8, wherein the set of mutant cancer-associated peptides comprises any one or more of BRAF V600E mutation, PIK3CA E545K mutation, KRAS G12D mutation, KRAS G13D mutation, KRAS A146T mutation, KRAS G12V mutation, TP53 R175H mutation, TP53 H179R mutation, TP53 R248Q mutation TP53 R273C mutation, TP53 R273H mutation, TP53 R282W mutation, IDH1 R132H mutation, IDH1 R132C mutation, IDH1 R132G mutation, IDH1 R132S mutation, IDH2 R172K mutation, CIC R215W mutation, or HLA-A Q78R mutation, NRAS Q61R mutation, NRAS Q61K mutation, NRAS Q61L mutation, MAP2K1 P124S mutation, RAC1 P29S mutation, PPP6C R301C mutation, CDKN2A P114L mutation, KRTAP4-11 L161V mutation, KRTAP4-11 M93V mutation, HRAS Q61R mutation, ZNF799 E589G mutation, ZNF844 R447P mutation, and RBM10 E184D mutation, wherein the presence of any one of these mutations indicates the presence of or increased risk of developing skin cutaneous melanoma.

15. The method according to any one of claims 6 to 8, wherein the set of mutant cancer-associated peptides comprises any one or more of KRAS G12C mutation, KRAS G12V mutation, Epidermal Growth Factor Receptor (EGFR) L858R mutation, KRAS G12D mutation, KRAS G12A mutation, U2 Small Nuclear RNA Auxiliary Factor 1 (U2AF1) S34F mutation, KRTAP4-11 L161V mutation, KRTAP4-11 R121K mutation, Eukaryotic Translation Elongation Factor 1 Beta 2 (EEF1B2) R42H mutation, and KRTAP4-11 M93V mutation, wherein the presence of any one of these mutations indicates the presence of or increased risk of developing stomach adenocarcinoma.

16. The method according to any one of claims 6 to 8, wherein the set of mutant cancer-associated peptides comprises any one or more of BRAF V600E mutation, PIK3CA E545K mutation, KRAS G12D mutation, KRAS G13D mutation, TP53 R175H mutation, KRAS G12V mutation, TP53 R248Q mutation, KRAS A146T mutation, TP53 R273H mutation, HRAS Q61R mutation, HLA-A Q78R mutation, TP53 R282W mutation, NRAS Q61R mutation, NRAS Q61K mutation, IDH1 R132C mutation, MAP2K1 P124S mutation, RAC1 P29S mutation, NRAS Q61L mutation, PPP6C R301C mutation, CDKN2A P114L mutation, KRTAP4-11 L161V mutation, KRTAP4-11 M93V mutation, ZNF799 E589G mutation, ZNF844 R447P mutation, and RBM10 E184D mutation, wherein the presence of any one of these mutations indicates the presence of or increased risk of developing thyroid carcinoma.

17. The method according to any one of claims 6 to 8, wherein the set of mutant cancer-associated peptides comprises any one or more of BRAF V600E mutation, PIK3CA H1047R mutation, PIK3CA E545K mutation, PIK3CA E542K mutation, TP53 R175H mutation, PIK3CA N345K mutation, AKT Serine/Threonine Kinase 1 (AKT1) E17K mutation, Splicing Factor 3b Subunit 1 (SF3B1) K700E mutation, KRAS G12C mutation, KRAS G12V mutation, Epidermal Growth Factor Receptor (EGFR) L858R mutation, KRAS G12D mutation, KRAS G12A mutation, KRAS G12V mutation, KRAS G13D mutation, TP53 R175H mutation, TP53 R248Q mutation, KRAS A146T mutation, TP53 R273H mutation, TP53 R282W mutation, U2 Small Nuclear RNA Auxiliary Factor 1 (U2AF1) S34F mutation, KRTAP4-11 L161V mutation, KRTAP4-11 R121K mutation, Eukaryotic Translation Elongation Factor 1 Beta 2 (EEF1B2) R42H mutation, and KRTAP4-11 M93V mutation, wherein the presence of any one of these mutations indicates the presence of or increased risk of developing uterine corpus endometrial carcinoma.

## Patentansprüche

1. Computerimplementiertes Verfahren zur Bestimmung, ob für ein Individuum ein Risiko für eine Erkrankung an oder eine Entstehung einer Krebserkrankung besteht, wobei das Verfahren Folgendes umfasst:

   a) die Genotypisierung des Haupthistokompatibilitätskomplexes Klasse I (MHC-I) des Individuums und
   b) die Bewertung der Fähigkeit des MHC-I des Individuums zur Präsentation eines mutanten krebsassoziierten Peptids basierend auf einer Bibliothek bekannter, von Individuen stammender krebsassoziierter Peptidsequenzen, wobei es sich beim erzeugten Score um den Score der MHC-I-Präsentation handelt;
   wobei:

      i) wenn das Individuum ein schlechter MHC-I-Presenter für spezifische mutante krebsassoziierte Peptide ist, das Individuum eine erhöhte Wahrscheinlichkeit für eine Erkrankung an oder eine Entstehung der Krebserkrankung aufweist, mit der die spezifischen krebsassoziierten Peptide assoziiert sind; oder,
      ii) wenn das Individuum ein guter MHC-I-Presenter für spezifische mutante krebsassoziierte Peptide ist, das Individuum eine verminderte Wahrscheinlichkeit für eine Erkrankung an oder eine Entstehung der Krebserkrankung aufweist, mit der die spezifischen krebsassoziierten Peptide assoziiert sind;

   wobei der Schritt der Bewertung der Fähigkeit des MHC-I des Individuums zur Präsentation eines mutanten krebsassoziierten Peptids die Verwendung einer vorhergesagten MHC-I-Affinität für eine gegebene Mutation $x_{ij}$ umfasst, wobei x die MHC-I-Affinität eines Individuums *i* für die Mutation *j* ist, um ein logistisches Regressionsmodell mit gemischten Effekten anzupassen, das einer Modellgleichung folgt, die aus einem großen Datensatz von Individuen erhalten wird, aus dem MHC-I-Genotypen und das Vorhandensein von interessierenden Peptiden erhalten werden können:

$$\mathrm{Logit}\ (P(y_{ij} = 1 \mid x_{ij})) = \eta_i + \gamma \log(x_{ij})$$

wobei:

$y_{ij}$ eine binäre Mutationsmatrix ist, wobei $y_{ij} \in \{0,1\}$ angibt, ob ein Individuum *i* eine Mutation *j* aufweist;
$X_{ij}$ eine binäre Mutationsmatrix ist, welche die vorhergesagte MHC-I-Bindungsaffinität eines Individuums *i* mit der Mutation *j* angibt;
$\gamma$ ein Maß für die Auswirkung der Log-Affinitäten auf die Mutationswahrscheinlichkeit ist und
$\eta_i \sim N(0, \phi_\eta)$ zufällige Effekte sind, die verschiedene Mutationsraten zwischen Individuen erfassen,
wobei mit dem Modell die Nullhypothese geprüft wird, dass $\gamma = 0$ ist, und Odds-Ratios für die MHC-I-Affinität einer Mutation und das Vorhandensein einer Krebserkrankung berechnet werden.

**2.** Verfahren nach Anspruch 1, das weiterhin Folgendes umfasst:
c) basierend auf einer von Individuen erhaltenen Bibliothek krebsassoziierter Mutationen die Bestimmung, ob eine vom Individuum erhaltene Flüssigbiopsieprobe DNA umfasst, die für ein mutantes krebsassoziiertes Peptid codiert.

**3.** Verfahren nach Anspruch 2, wobei es sich bei der Flüssigbiopsieprobe um Blut, Speichel, Urin oder eine andere Körperflüssigkeit handelt.

**4.** Verfahren nach Anspruch 2, wobei die Bibliothek krebsassoziierter Mutationen durch eine Gesamtgenomsequenzierung von Individuen erhalten wird.

**5.** Verfahren nach Anspruch 1, wobei die vorhergesagte MHC-I-Affinität für eine gegebene Mutation $x_{ij}$ ein Score für den harmonischen Mittelwert des besten Rangs eines Individuums (PHBR) ist.

**6.** Verfahren nach Anspruch 5, wobei der PHBR-Score erhalten wird, indem MHC-I-Bindungsaffinitäten eines Satzes von mutanten krebsassoziierten Peptiden unter Bezugnahme auf einen vorbestimmten Datensatz von Peptiden aggregiert werden, die an MHC-I-Molekülen binden, die von mindestens 16 verschiedenen HLA-Allelen codiert werden.

**7.** Verfahren nach Anspruch 1, wobei das mutante krebsassoziierte Peptid eine Aminosäuren-Substitution, - Insertion oder -Deletion enthält und wobei der Satz von Peptiden aus mindestens 38 aller möglichen Peptide mit einer Länge von 8, 9, 10 und 11 Aminosäuren an jeder Position entlang des Peptids besteht, welches die Substitution, Insertion oder Deletion enthält.

**8.** Verfahren nach einem der Ansprüche 1 bis 7, wobei es sich bei der Krebserkrankung um ein Karzinom der Nebennierenrinde (ACC), ein Urothelkarzinom der Blase (BLCA), ein invasives Mammakarzinom (BRCA), ein zervikales Plattenepithelkarzinom und ein endozervikales Adenokarzinom (CESC), ein Adenokarzinom des Kolons (COAD), ein diffuses großzelliges B-Zell-Lymphom einer lymphoiden Neubildung (DLBC), ein Glioblastoma multiforme (GBM), ein Plattenepithelkarzinom der Kopf-Hals-Region (HNSC), ein Nieren-Chromophob (KICH), ein klarzelliges Nierenzellkarzinom der Niere (KIRC), ein papilläres Nierenzellkarzinom der Niere (KIRP), eine akute myeloische Leukämie (LAML), ein niedriggradiges Hirngliom (LGG), ein hepatozelluläres Karzinom der Leber (LIHC), ein Adenokarzinom der Lunge (LUAD), ein Plattenepithelkarzinom der Lunge (LUSC), ein Mesotheliom (MESO), ein seröses Zystadenokarzinom des Eierstocks (OV), ein Adenokarzinom des Pankreas (PAAD), ein Phäochromozytom und Paragangliom (PCPG), ein Adenokarzinom der Prostata (PRAD), ein Adenokarzinom des Rektums (READ), ein Sarkom (SARC), ein kutanes Melanom der Haut (SKCM), ein Adenokarzinom des Magens (STAD), einen testikulären Keimzell-Tumor (TGCT), ein Schilddrüsenkarzinom (THCA), ein endometriales Karzinom des Gebärmutterkörpers (UCEC), ein Karzinosarkom der Gebärmutter (UCS) oder ein Aderhautmelanom (UVM) handelt.

**9.** Verfahren nach einem der Ansprüche 6 bis 8, wobei der Satz von mutanten krebsassoziierten Peptiden eine Mutation V600E des Proto-Onkogens B-Raf (BRAF), Mutation E545K der katalytischen Untereinheit Alpha von Phosphatidylinosit-4,5-bisphosphat-3-kinase (PIK3CA), PIK3CA-Mutation E542K, PIK3CA-Mutation H1047R, Mutation G12D des Kirsten-Ratten-Sarkom-Virusonkogen-Homologs (KRAS), KRAS-Mutation G13D, KRAS-Mutation G12V, KRAS-Mutation A146T, TP53-Mutation R175H, TP53-Mutation H179R, TP53-Mutation, TP53-Mutation R248Q, TP53-Mutation R273C, TP53-Mutation R273H, TP53-Mutation R282W, Mutation L161V des Keratin-assoziierten Proteins 4-11 (KRTAP4-11), Mutation L161V, Mutation Q311E der Mab-21-Domäne, die 2 enthält (MB21D2), HLA-A-Mutation Q78R, Mutation G13V des Harvey-Ratten-Sarkom-Virusonkogen-Homologs (HRAS), Mutation R132H von Isocitrat-Dehydrogenase (NADP(+)) 1 (IDH1), IDH1-Mutation R132C, IDH1-Mutation R132G, IDH2-Mutation R172K, IDH1-Mutation R132S, Mutation R215W des transkriptionellen Repressors von Capicua (CIC), Mutation I98V von Phosphoglucomutase 5 (PGM5), Mutation Y192H des Tripartite Motif Containing 48 (TRIM48) und/oder Mutation R465C der F-Box and WD Repeat Domain Containing 7 (FBXW7) umfasst, wobei das Vorhandensein einer dieser Mutationen das Vorhandensein oder ein erhöhtes Risiko für die Entwicklung eines invasiven Mammakarzinoms angibt.

**10.** Verfahren nach einem der Ansprüche 6 bis 8, wobei der Satz von mutanten krebsassoziierten Peptiden eine BRAF-Mutation V600E, Mutation Q61R des viralen Neuroblastom-RAS-Onkogen-Homologs (NRAS), NRAS-Mutation Q61K, NRAS-Mutation Q61L, IDH1-Mutation R132S, Mutation P124S der Mitogen-aktivierten Proteinkinase-Kinase 1 (MAP2K1), Mutation P29S der kleinen GTPase 1 der Rac-Familie (RAC1), Mutation R301C der katalytischen Untereinheit der Proteinphosphatase 6 (PPP6C), Mutation P114L des Cyclin-abhängigen Kinaseinhibitors 2A (CDKN2A), Mutation L161V des Keratin-assoziierten Proteins 4-11 (KRTAP4-11), KRTAP4-11-Mutation M93V, HRAS-Mutation Q61R, HLA-A-Mutation Q78R, Mutation E589G des Zinkfingerproteins 799 (ZNF799), Mutation R447P des Zinkfingerproteins 844 (ZNF844) und/oder Mutation E184D des RNA-Bindungsmotif-Proteins 10 (RBM10) umfasst, wobei das Vorhandensein einer dieser Mutationen das Vorhandensein oder ein erhöhtes Risiko für die Entwicklung eines Adenokarzinoms des Kolons angibt.

**11.** Verfahren nach einem der Ansprüche 6 bis 8, wobei der Satz von mutanten krebsassoziierten Peptiden eine IDH1-Mutation R132H, IDH1-Mutation R132C, IDH1-Mutation R132G, IDH1-Mutation R132S, IDH2-Mutation R172K, TP53-Mutation H179R, TP53-Mutation R273C, TP53-Mutation R273H, CIC-Mutation R215W und/oder HLA-A-Mutation Q78R umfasst, wobei das Vorhandensein einer dieser Mutationen das Vorhandensein oder ein erhöhtes Risiko für die Entwicklung eines Plattenepithelkarzinoms der Kopf-Hals-Region oder eines niedriggradigen Hirngglioms angibt.

**12.** Verfahren nach einem der Ansprüche 6 bis 8, wobei der Satz von mutanten krebsassoziierten Peptiden eine BRAF-Mutation V600E, PIK3CA-Mutation E545K, KRAS-Mutation G12D, KRAS-Mutation G13D, KRAS-Mutation A146T, TP53-Mutation R175H, KRAS-Mutation G12V, TP53-Mutation R248Q, TP53-Mutation R273C, TP53-Mutation R273H, TP53-Mutation R282W, PGM5-Mutation I98V, TRIM48-Mutation Y192H, PIK3CA-Mutation E545K, KRAS-Mutation G13D, PIK3CA-Mutation H1047R und/oder FBXW7-Mutation R465C umfasst, wobei das Vorhandensein einer dieser Mutationen das Vorhandensein oder ein erhöhtes Risiko für die Entwicklung eines Adenokarzinoms der Lunge angibt.

**13.** Verfahren nach einem der Ansprüche 6 bis 8, wobei der Satz von mutanten krebsassoziierten Peptiden eine PIK3CA-Mutation H1047R, PIK3CA-Mutation E545K, PIK3CA-Mutation E542K, TP53-Mutation R175H, PIK3CA-Mutation N345K, Mutation E17K der AKT-Serin/Threonin-Kinase 1 (AKT1), Mutation K700E von Untereinheit 1 des Spleißfaktors 3b (SF3B1) und/oder PIK3CA-Mutation H1047L umfasst, wobei das Vorhandensein einer dieser Mutationen das Vorhandensein oder ein erhöhtes Risiko für die Entwicklung eines Plattenepithelkarzinoms der Lunge angibt.

**14.** Verfahren nach einem der Ansprüche 6 bis 8, wobei der Satz von mutanten krebsassoziierten Peptiden eine BRAF-Mutation V600E, PIK3CA-Mutation E545K, KRAS-Mutation G12D, KRAS-Mutation G13D, KRAS-Mutation A146T, KRAS-Mutation G12V, TP53-Mutation R175H, TP53-Mutation H179R, TP53-Mutation R248Q, TP53-Mutation R273C, TP53-Mutation R273H, TP53-Mutation R282W, IDH1-Mutation R132H, IDH1-Mutation R132C, IDH1-Mutation R132G, IDH1-Mutation R132S, IDH2-Mutation R172K, CIC-Mutation R215W oder HLA-A-Mutation Q78R, NRAS-Mutation Q61R, NRAS-Mutation Q61K, NRAS-Mutation Q61L, MAP2K1-Mutation P124S, RAC1-Mutation P29S, PPP6C-Mutation R301C, CDKN2A-Mutation P114L, KRTAP4-11-Mutation L161V, KRTAP4-11-Mutation M93V, HRAS-Mutation Q61R, ZNF799-Mutation E589G, ZNF844-Mutation R447P und/oder RBM10-Mutation E184D umfasst, wobei das Vorhandensein einer dieser Mutationen das Vorhandensein oder ein erhöhtes Risiko für die Entwicklung eines kutanen Melanoms der Haut angibt.

**15.** Verfahren nach einem der Ansprüche 6 bis 8, wobei der Satz von mutanten krebsassoziierten Peptiden eine KRAS-Mutation G12C, KRAS-Mutation G12V, Mutation L858R des epidermalen Wachstumsfaktor-Rezeptors (EGFR), KRAS-Mutation G12D, KRAS-Mutation G12A, Mutation S34F des kleinen nukleären RNA-U2-Hilfsfaktors 1 (U2AF1), KRTAP4-11-Mutation L161V, KRTAP4-11-Mutation R121K, Mutation R42H des eukaryotischen Translationselongationsfaktors 1 Beta 2 (EEF1B2) und/oder KRTAP4-11-Mutation M93V umfasst, wobei das Vorhandensein einer dieser Mutationen das Vorhandensein oder ein erhöhtes Risiko für die Entwicklung eines Adenokarzinoms des Magens angibt.

**16.** Verfahren nach einem der Ansprüche 6 bis 8, wobei der Satz von mutanten krebsassoziierten Peptiden eine BRAF-Mutation V600E, PIK3CA-Mutation E545K, KRAS-Mutation G12D, KRAS-Mutation G13D, TP53-Mutation R175H, KRAS-Mutation G12V, TP53-Mutation R248Q, KRAS-Mutation A146T, TP53-Mutation R273H, HRAS-Mutation Q61R, HLA-A-Mutation Q78R, TP53-Mutation R282W, NRAS-Mutation Q61R, NRAS-Mutation Q61K, IDH1-Mutation R132C, MAP2K1-Mutation P124S, RAC1-Mutation P29S, NRAS-Mutation Q61L, PPP6C-Mutation R301C, CDKN2A-Mutation P114L, KRTAP4-11-Mutation L161V, KRTAP4-11-Mutation M93V, ZNF799-Mutation E589G, ZNF844-Mutation R447P und/oder RBM10-Mutation E184D umfasst, wobei das Vorhandensein einer dieser Mutationen das Vorhandensein oder ein erhöhtes Risiko für die Entwicklung eines Schilddrüsenkarzinoms angibt.

**17.** Verfahren nach einem der Ansprüche 6 bis 8, wobei der Satz von mutanten krebsassoziierten Peptiden eine BRAF-Mutation V600E, PIK3CA-Mutation H1047R, PIK3CA-Mutation E545K, PIK3CA-Mutation E542K, TP53-Mutation R175H, PIK3CA-Mutation N345K, Mutation E17K der AKT-Serin/Threonin-Kinase 1 (AKT1), Mutation K700E der Untereinheit 1 des Spleißfaktors 3b (SF3B1), KRAS-Mutation G12C, KRAS-Mutation G12V, Mutation L858R des epidermalen Wachstumsfaktor-Rezeptors (EGFR), KRAS-Mutation G12D, KRAS-Mutation G12A, KRAS-Mutation G12V, KRAS-Mutation G13D, TP53-Mutation R175H, TP53-Mutation R248Q, KRAS-Mutation A146T, TP53-Mutation R273H, TP53-Mutation R282W, Mutation S34F des kleinen nukleären RNA-U2-Hilfsfaktors 1 (U2AF1), KRTAP4-11-Mutation L161V, KRTAP4-11-Mutation R121K, Mutation R42H des eukaryotischen Translationselongationsfaktors 1 Beta 2 (EEF1B2) und/oder KRTAP4-11-Mutation M93V umfasst, wobei das Vorhandensein einer dieser Mutationen das Vorhandensein oder ein erhöhtes Risiko für die Entwicklung eines endometrialen Karzinoms des Gebärmutterkörpers angibt.

**Revendications**

**1.** Méthode mise en œuvre par ordinateur pour déterminer si un sujet risque de présenter ou de développer un cancer, la méthode comprenant :

a) le génotypage du complexe majeur d'histocompatibilité de classe I (CMH-I) du sujet ; et
b) l'évaluation de la capacité du CMH-I du sujet à présenter un peptide mutant associé au cancer sur la base d'une banque de séquences peptidiques connues associées au cancer provenant de sujets, le score généré étant le score de présentation par CMH-I ;

dans laquelle :

i) si le sujet est un faible présentateur par CMH-I de peptides mutants spécifiques associés au cancer, le sujet présente une probabilité accrue d'avoir ou de développer le cancer auquel sont associés les peptides mutants spécifiques associés au cancer ; ou
ii) si le sujet est un fort présentateur par CMH-I de peptides mutants spécifiques associés au cancer, le sujet présente une probabilité réduite d'avoir ou de développer le cancer auquel sont associés les peptides mutants spécifiques associés au cancer ;
dans laquelle l'étape d'évaluation de la capacité du CMH-I du sujet à présenter un peptide mutant associé au cancer comprend l'utilisation d'une affinité prédite de CMH-I pour une mutation donnée $x_{ij}$, où x est l'affinité de CMH-I du sujet *i* pour la mutation *j* pour ajuster un modèle de régression logistique à effets mixtes qui suit une équation modèle obtenue à partir d'un grand ensemble de données de sujets à partir duquel des génotypes de CMH-I et la présence de peptides d'intérêt peuvent être obtenus :

$$\mathrm{logit}(P(y_{ij} = 1 | x_{ij})) = \eta_i + \gamma \log(x_{ij})$$

dans laquelle :

$y_{ij}$ est une matrice de mutation binaire $y_{ij} \in \{0,1\}$ indiquant si un sujet *i* présente une mutation *j* ;
$x_{ij}$ est une matrice de mutation binaire indiquant l'affinité de liaison prédite de CMH-I du sujet *i* ayant la

mutation $j$ ;
$\gamma$ mesure l'effet des affinités logarithmiques sur la probabilité de mutation ; et
$\eta_i$ ~ N(O, $\Phi_\eta$) sont des effets aléatoires traduisant différents taux de mutation parmi les sujets,
dans laquelle le modèle teste l'hypothèse nulle selon laquelle $\gamma = 0$ et calcule les rapports de cotes pour l'affinité de CMH-I d'une mutation et la présence d'un cancer.

**2.** Méthode selon la revendication 1, comprenant en outre :
c) déterminer du fait qu'un échantillon de biopsie liquide obtenu à partir du sujet comprend ou non de l'ADN codant pour un peptide mutant associé au cancer sur la base d'une banque de mutations associées au cancer obtenues à partir de sujets.

**3.** Méthode selon la revendication 2, dans laquelle l'échantillon de biopsie liquide est du sang, de la salive, de l'urine ou un autre fluide corporel.

**4.** Méthode selon la revendication 2, dans laquelle la banque de mutations associées au cancer est obtenue par séquençage du génome entier de sujets.

**5.** Méthode selon la revendication 1, dans laquelle l'affinité de CMH-I prédite pour une mutation donnée $x_{ij}$ est un score PHBR (Patient Harmonic-mean Best Rank).

**6.** Méthode selon la revendication 5, dans laquelle le score PHBR est obtenu par agrégation des affinités de liaison CMH-I d'un ensemble de peptides mutants associés au cancer en référence à un ensemble de données prédéterminé de peptides se liant à des molécules CMH-I codées par au moins 16 allèles HLA différents.

**7.** Méthode selon la revendication 1, dans laquelle le peptide mutant associé au cancer contient une substitution, insertion ou délétion d'acide aminé, et dans laquelle l'ensemble de peptides est constitué d'au moins 38 de l'ensemble des peptides possibles de 8, 9, 10 et 11 acides aminés de longueur incorporant la substitution, l'insertion ou la délétion à chaque position le long du peptide.

**8.** Méthode selon l'une quelconque des revendications 1 à 7, dans laquelle le cancer est un carcinome corticosurrénalien (ACC), un carcinome urothélial de la vessie (BLCA), un carcinome invasif du sein (BRCA), un carcinome épidermoïde du col de l'utérus et un adénocarcinome endocervical (CESC), un adénocarcinome du côlon (COAD), un néoplasme lymphoïde diffus à grandes cellules B (DLBC), un glioblastome multiforme (GBM), un carcinome épidermoïde de la tête et du cou (HNSC), un carcinome rénal à cellules chromophobes (KICH), un carcinome rénal à cellules claires (KIRC), un carcinome rénal à cellules papillaires (KIRP), une leucémie myéloïde aiguë (LAML), un gliome cérébral de bas grade (LGG), un carcinome hépatocellulaire du foie (LIHC), un adénocarcinome pulmonaire (LUAD), un carcinome épidermoïde pulmonaire (LUSC), un mésothéliome (MESO), un cystadénocarcinome séreux ovarien (OV), un adénocarcinome pancréatique (PAAD), un phéochromocytome et un paragangliome (PCPG), un adénocarcinome de la prostate (PRAD), un adénocarcinome du rectum (READ), un sarcome (SARC), un mélanome cutané (SKCM), un adénocarcinome de l'estomac (STAD), une tumeur germinale testiculaire (TGCT), un carcinome thyroïdien (THCA), un carcinome endométrial du corps utérin (UCEC), un carcinosarcome utérin (UCS) ou un mélanome uvéal (UVM).

**9.** Méthode selon l'une quelconque des revendications 6 à 8, dans laquelle l'ensemble de peptides mutants associés au cancer comprend une ou plusieurs mutations parmi : la mutation V600E du proto-oncogène B-Raf (BRAF), la mutation E545K de la sous-unité catalytique alpha de la phosphatidylinositol-4,5-bisphosphate 3-kinase (PIK3CA), la mutation E542K de PIK3CA, la mutation H1047R de PIK3CA, la mutation G12D de l'homologue de l'oncogène viral du sarcome du rat de Kirsten (KRAS), la mutation G13D de KRAS, la mutation G12V de KRAS, la mutation A146T de KRAS, la mutation R175H de TP53, la mutation H179R de TP53, la mutation R248Q de TP53, la mutation R273C de TP53, la mutation R273H de TP53, la mutation R282W de TP53, la mutation L161V de la protéine associée à la kératine 4-11 (KRTAP4-11), la mutation Q311E de la protéine contenant le domaine Mab-21 2 (MB21D2), la mutation Q78R de HLA-A, la mutation G13V de l'homologue de l'oncogène viral du sarcome du rat de Harvey (HRAS), la mutation R132H de l'isocitrate déshydrogénase (NADP(+)) 1 (IDH1), la mutation R132C d'IDH1, la mutation R132G d'IDH1, la mutation R172K d'IDH2, la mutation R132S d'IDH1, la mutation R215W du répresseur transcriptionnel Capicua (CIC), la mutation I98V de la phosphoglucomutase 5 (PGM5), la mutation Y192H de la protéine contenant un motif tripartite 48 (TRIM48) et la mutation R465C de la protéine contenant les domaines de répétition F-Box et WD 7 (FBXW7), la présence de l'une quelconque de ces mutations indiquant la présence d'un carcinome invasif du sein ou un risque accru de développer un carcinome invasif du sein.

10. Méthode selon l'une quelconque des revendications 6 à 8, dans laquelle l'ensemble de peptides mutants associés au cancer comprend une ou plusieurs mutations parmi : la mutation V600E de BRAF, la mutation Q61R de l'homologue de l'oncogène viral du neuroblastome RAS (NRAS), la mutation Q61K de NRAS, la mutation Q61L de NRAS, la mutation R132S d'IDH1, la mutation P124S de la protéine kinase 1 activée par les mitogènes (MAP2K1), la mutation P29S de la petite GTPase 1 de la famille Rac (RAC1), la mutation R301C de la sous-unité catalytique 6 de protéine phosphatase (PPP6C), la mutation P114L de l'inhibiteur de kinase dépendante des cyclines 2A (CDKN2A), la mutation L161V de la protéine associée à la kératine 4-11 (KRTAP4-11), la mutation M93V de KRTAP4-11, la mutation Q61R de HRAS, la mutation Q78R de HLA-A, la mutation E589G de la protéine à doigt de zinc 799 (ZNF799), la mutation R447P de la protéine à doigt de zinc 844 (ZNF844) et la mutation E184D de la protéine à motif de liaison à l'ARN 10 (RBM10), la présence de l'une quelconque de ces mutations indiquant la présence d'un adénocarcinome du côlon ou un risque accru de développer un adénocarcinome du côlon.

11. Méthode selon l'une quelconque des revendications 6 à 8, dans laquelle l'ensemble de peptides mutants associés au cancer comprend une ou plusieurs mutations parmi : la mutation R132H d'IDH1, la mutation R132C d'IDH1, la mutation R132G d'IDH1, la mutation R132S d'IDH1, la mutation R172K d'IDH2, la mutation H179R de TP53, la mutation R273C de TP53, la mutation R273H de TP53, la mutation R215W de CIC et la mutation Q78R de HLA-A, la présence de l'une quelconque de ces mutations indiquant la présence d'un carcinome épidermoïde de la tête et du cou ou un gliome cérébral de bas grade ou un risque accru de développer un carcinome épidermoïde de la tête et du cou ou un gliome cérébral de bas grade.

12. Méthode selon l'une quelconque des revendications 6 à 8, dans laquelle l'ensemble de peptides mutants associés au cancer comprend une ou plusieurs mutations parmi : la mutation V600E de BRAF, la mutation E545K de PIK3CA, la mutation G12D de KRAS, la mutation G13D de KRAS, la mutation A146T de KRAS, la mutation R175H de TP53, la mutation G12V de KRAS, la mutation R248Q de TP53, la mutation R273C de TP53, la mutation R273H de TP53, la mutation R282W de TP53, la mutation I98V de PGM5, la mutation Y192H de TRIM48, la mutation E545K de PIK3CA, la mutation G13D de KRAS, la mutation H1047R de PIK3CA et la mutation R465C de FBXW7, la présence de l'une quelconque de ces mutations indiquant la présence d'un adénocarcinome pulmonaire ou un risque accru de développer un adénocarcinome pulmonaire.

13. Méthode selon l'une quelconque des revendications 6 à 8, dans laquelle l'ensemble de peptides mutants associés au cancer comprend une ou plusieurs mutations parmi : la mutation H1047R de PIK3CA, la mutation E545K de PIK3CA, la mutation E542K de PIK3CA, la mutation R175H de TP53, la mutation N345K de PIK3CA, la mutation E17K de sérine/thréonine kinase AKT 1 (AKT1), la mutation K700E de la sous-unité 1 du facteur d'épissage 3b (SF3B1) et la mutation H1047L de PIK3CA, la présence de l'une quelconque de ces mutations indiquant la présence d'un carcinome épidermoïde pulmonaire ou un risque accru de développer un carcinome épidermoïde pulmonaire.

14. Méthode selon l'une quelconque des revendications 6 à 8, dans laquelle l'ensemble de peptides mutants associés au cancer comprend une ou plusieurs mutations parmi : la mutation V600E de BRAF, la mutation E545K de PIK3CA, la mutation G12D de KRAS, la mutation G13D de KRAS, la mutation A146T de KRAS, la mutation G12V de KRAS, la mutation R175H de TP53, la mutation H179R de TP53, la mutation R248Q de TP53, la mutation R273C de TP53, la mutation R273H de TP53, la mutation R282W de TP53, la mutation R132H de IDH1, la mutation R132C de IDH1, la mutation R132G de IDH1, la mutation R132S de IDH1, la mutation R172K de IDH2, la mutation R215W de CIC, la mutation Q78R de HLA-A, la mutation Q61R de NRAS, la mutation Q61K de NRAS, la mutation Q61L de NRAS, la mutation P124S de MAP2K1, la mutation P29S de RAC1, la mutation R301C de PPP6C, la mutation P114L de CDKN2A, la mutation L161V de KRTAP4-11, la mutation M93V de KRTAP4-11, la mutation Q61R de HRAS, la mutation E589G de ZNF799, la mutation R447P de ZNF844 et la mutation E184D de RBM10, la présence de l'une quelconque de ces mutations indiquant la présence d'un mélanome cutané ou un risque accru de développer un mélanome cutané.

15. Méthode selon l'une quelconque des revendications 6 à 8, dans laquelle l'ensemble de peptides mutants associés au cancer comprend une ou plusieurs mutations parmi : la mutation G12C de KRAS, la mutation G12V de KRAS, la mutation L858R du récepteur du facteur de croissance épidermique (EGFR), la mutation G12D de KRAS, la mutation G12A de KRAS, la mutation S34F du facteur auxiliaire 1 du petit ARN nucléaire U2 (U2AF1), la mutation L161V de KRTAP4-11, la mutation R121K de KRTAP4-11, la mutation R42H du facteur d'élongation de traduction eucaryote 1 bêta 2 (EEF1B2) et la mutation M93V de KRTAP4-11, la présence de l'une quelconque de ces mutations indiquant la présence d'un adénocarcinome de l'estomac ou un risque accru de développer un adénocarcinome de l'estomac.

16. Méthode selon l'une quelconque des revendications 6 à 8, dans laquelle l'ensemble de peptides mutants associés au cancer comprend une ou plusieurs mutations parmi : la mutation V600E de BRAF, la mutation E545K de PIK3CA, la mutation G12D de KRAS, la mutation G13D de KRAS, la mutation R175H de TP53, la mutation G12V de KRAS, la mutation R248Q de TP53, la mutation A146T de KRAS, la mutation R273H de TP53, la mutation Q61R de HRAS, la mutation Q78R de HLA-A, la mutation R282W de TP53, la mutation Q61R de NRAS, la mutation Q61K de NRAS, la mutation R132C de IDH1, la mutation P124S de MAP2K1, la mutation P29S de RAC1, la mutation Q61L de NRAS, la mutation R301C de PPP6C, la mutation P114L de CDKN2A, la mutation L161V de KRTAP4-11, la mutation M93V de KRTAP4-11, la mutation E589G de ZNF799, la mutation R447P de ZNF844 et la mutation E184D de RBM10, la présence de l'une de ces mutations indiquant la présence d'un carcinome thyroïdien ou un risque accru de développer un carcinome thyroïdien.

**17.** Méthode selon l'une quelconque des revendications 6 à 8, dans laquelle l'ensemble de peptides mutants associés au cancer comprend une ou plusieurs mutations parmi : la mutation V600E de BRAF, la mutation H1047R de PIK3CA, la mutation E545K de PIK3CA, la mutation E542K de PIK3CA, la mutation R175H de TP53, la mutation N345K de PIK3CA, la mutation E17K de sérine/thréonine kinase AKT 1 (AKT1), la mutation K700E de la sous-unité 1 du facteur d'épissage 3b (SF3B1), la mutation G12C de KRAS, la mutation G12V de KRAS, la mutation L858R du récepteur du facteur de croissance épidermique (EGFR), la mutation G12D de KRAS, la mutation G12A de KRAS, la mutation G12V de KRAS, la mutation G13D de KRAS, la mutation R175H de TP53, la mutation R248Q de TP53, la mutation A146T de KRAS, la mutation R273H de TP53, la mutation R282W de TP53, la mutation S34F du facteur auxiliaire 1 du petit ARN nucléaire U2 (U2AF1), la mutation L161V de KRTAP4-11, la mutation R121K de KRTAP4-11, la mutation R42H du facteur d'élongation de traduction eucaryote 1 bêta 2 (EEF1B2), la mutation M93V de KRTAP4-11, la présence de l'une quelconque de ces mutations indiquant la présence d'un carcinome de l'endomètre du corps utérin ou un risque accru de développer un carcinome de l'endomètre du corps utérin.

Frequent
Oncogenic Mutations
A
B
C
BRAF V600E (10%)
IDH1 R132H (8%)
PIK3CA E545K (5%)
PIK3CA H1047R (4%)
KRAS G12D (3%)
KRAS G12V (3%)
PIK3CA E542K (3%)
TP53 R175H (2%)
TP53 R273C (2%)
TP53 R248Q (2%)
KRAS G12C (2%)
NRAS Q61R (2%)
TP53_R273H (2%)
IDH1_R132C (1%)
Cancer Risk
Low
High

Figure 1

Amino Acid Residue

38 possible peptides containing the residue
8-mers
9-mers
10-mers
11-mers
Residue-centric presentation score

Figure 2A

Peptides dectected by mass-spectrometry
Single-allele cell line
m/z
Random peptides
HLA allele-specific peptide rank assignment
Best Ranked Peptides
1.2
1.9
3.3
6.4
7.1
7.1
5.6
14.2
21.9
38.3
61.4
79.1

Figure 2B

Fraction of MS-based residues recovered
1.0
0.8
0.6
0.4
0.2
0.0
0.0
0.2
0.4
0.6
0.8
1.0
Fraction of random residues recovered
AUC = 0.84
Best Rank

Figure 2C

Fraction of mutations observed
0.25
0.20
0.15
0.10
0.05
0.00
Binding Strength
Strong (< 0.5)
Weak
(>= 0.5, < 2)
A2780
OV90
HeLa
SKOV3
A375
Cell line

Figure 2D

16
14
12
10
8
6
4
2
0
Number of indels
21
25
Number of Unique Peptides

Figure 3A

0.8
0.7
0.6
0.5
0.4
0.3
0.2
0.1
0.0
Normalized frequency
MS
Random
0
5
10
15
20
25
30
35
40
Best Rank

Figure 3B

MS
Random
Normalized frequency
0.9
0.8
0.7
0.6
0.5
0.4
0.3
0.2
0.1
0.0
0
5
10
15
20
25
Summation (Rank < 2%)

Figure 3C

MS
Random
Normalized frequency
0.7
0.6
0.5
0.4
0.3
0.2
0.1
0.0
0
2
4
6
8
10
12
14
16
Summation (Rank < 0.5%)

Figure 3D

0.35
0.30
0.25
0.20
0.15
0.10
0.05
0.00
Normalized frequency
MS
Random
0
5
10
15
20
25
30
35
40
Best Rank with Cleavage

Figure 3E

2.5
2.0
1.5
1.0
0.5
0.0
-0.5
-1.0
Log (Observed MS / Random)
0.25
0.5
0.75
1.0
1.25
1.5
1.75
2.0
Best Rank

Figure 3F

Log (Observed MS / Random)
4
3
2
1
0
−1
−2
0 1 2 3 4 5 6 7 8 9 10 11 12 13
Summation (Rank < 2%)

Figure 3G

Log (Observed MS / Random)
4
3
2
1
0
−1
0 1 2 3 4 5 6 7 8 9 10 11 12 13
Summation (Rank < 0.5%)

Figure 3H

Log (Observed MS / Random)
2.5
2.0
1.5
1.0
0.5
0.0
−0.5
−1.0
−1.5
0.25
0.5
0.75
1.0
1.25
1.5
1.75
2.0
3.0
4.0
5.0
6.0
7.0
8.0
9.0
10.0
20.0
30.0
40.0
50.0
Best Rank with Cleavage

Figure 3I

Fraction of MS-based residues recovered
1.0
0.8
0.6
0.4
0.2
0.0
0.0
0.2
0.4
0.6
0.8
1.0
Fraction of random residues recovered
Summation (Rank < 2); AUC = 0.82
Summation (Rank < 0.5); AUC = 0.77
Best Rank; AUC = 0.84
Best Rank with Cleavage; AUC = 0.71

Figure 3J

A0101
A0201
A0203
A0204
A0207
A0301
A2402
A2902
A3101
A6802
B3501
B4402
B4403
B5101
B5401
B5701
HLA Allele
AUC
0.90
0.84
0.78
0.72
0.66
Summation (Rank < 2%)
Summation (Rank < 0.5%)
Best Rank
Best Rank w/ cleavage
Presentation Score

Figure 3K

Total number of peptides measured
$10^6$
$10^5$
$10^4$
$10^3$
$10^2$
10
0
A*02:01
A*24:02
B*57:01
Allele


Figure 4A

Fraction of predicted peptides recovered
0.40
0.35
0.30
0.25
0.20
0.15
0.10
0.05
0.00
High presentation score
Low presentation score
*
*
Random residues
Oncogenic residues


Figure 4B

Generalized additive model
Probability of mutation
0.0010
0.0015
0.0020
0.0025
0.0030
0.0035
0
2
4
6
8
10

Figure 5A

Estimated logit(prob) vs log(affinity)
Logit mutation probability
-0.5
0.0
0.5
-4
-2
0
2
4

Figure 5B

Discretizing affinity
Probability of mutation
0.0000
0.0005
0.0010
0.0015
0
2
4
6
8

Figure 5C

DLBC
PCPG
KIRP
MESO
LIHC
ACC
CESC
BRCA
HNSC
PRAD
BLCA
LAML
READ
UCS
STAD
KICH
LUAD
OV
GBM
UCEC
THCA
COAD
TGCT
SKCM
LUSC
SARC
LGG
PAAD
KIRC
UVM
0.5
1.0
2.0
Odds-ratio

Figure 6A

THCA
DLBC
PCPG
KIRP
LGG
PAAD
READ
MESO
COAD
BRCA
LIHC
TGCT
PRAD
SKCM
HNSC
ACC
BLCA
LUAD
STAD
GBM
UCS
OV
CESC
UCEC
KICH
LAML
LUSC
SARC
KIRC
UVM
0.5
1.0
2.0
Odds−ratio

Figure 6B

PCPG
KIRP
LIHC
BRCA
HNSC
PRAD
BLCA
LAML
READ
STAD
LUAD
OV
GBM
UCEC
THCA
COAD
TGCT
SKCM
LUSC
SARC
LGG
PAAD
KIRC
0.5
1.0
2.0
Odds-ratio

Figure 7A

THCA
PCPG
KIRP
LGG
PAAD
READ
COAD
BRCA
LIHC
TGCT
PRAD
SKCM
HNSC
BLCA
LUAD
STAD
GBM
OV
UCEC
LAML
LUSC
SARC
KIRC
0.5
1.0
2.0
Odds-ratio

Figure 7B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 2016102359 A
- WO 2012159754 A
- US 6689576 B
- US 6395503 B
- US 6087188 A
- US 6287767 B
- US 6165800 A
- US 6126870 A


### Non-patent literature cited in the description


- **HANAHAN** ; **WEINBERG**. *Cell*, 2011, vol. 144, 646-674
- **BRAHMER et al.** *N. Engl. J. Med.*, 2012, vol. 366, 2455-2465
- **HODI et al.** *N. Engl. J. Med.*, 2010, vol. 363, 711-723
- **TOPALIAN et al.** *N. Engl. J. Med.*, 2012, vol. 366, 2443-2454
- **RIZVI et al.** *Science*, 2015, vol. 348, 124-128
- **ROONEY et al.** *Cell*, 2015, vol. 160, 48-61
- **SCHUMACHER** ; **SCHREIBER**. *Science*, 2015, vol. 348, 69-74
- **DUPAGE et al.** *Nature*, 2012, vol. 482, 405-409
- **KAPLAN et al.** *Proc. Natl. Acad. Sci. USA*, 1998, vol. 95, 7556-7561
- **KOEBEL et al.** *Nature*, 2007, vol. 450, 903-907
- **MATSUSHITA et al.** *Nature*, 2012, vol. 482, 400-404
- **SHANKARAN et al.** *Nature*, 2001, vol. 410, 1107-111
- **ROBINSON et al.** *Nucleic Acids Res.*, 2015, vol. 43, D423-D431
- **LU et al.** *Int. Immunol.*, 2016, vol. 28, 365-370
- **ABELIN et al.** *Mass Immunity*, 2017, vol. 46, 315-326
- **SCHULZE et al.** *Nature Cell. Biol.*, 2001, vol. 3, E190
- **KLEIN et al.** *J. Exp. Med.*, 2001, vol. 194, 1625-1638
- *Nature*, 2014, vol. 507, 315-22
- **JIANG et al.** *Bioinformatics*, 2007, vol. 23, 306-13
- **HARTL**. A Primer of Population Genetics. Washington University, Saint Louis Sinauer Associates, Inc., 1981
- **LYNCH** ; **WALSH**. Genetics and Analysis of Quantitative Traits. Sinauer Associates, Inc., 1998
- **DAVOLI et al.** *Cell*, 2013, vol. 155, 948-962
- **WAN et al.** *BMC Bioinformatics*, 2006, vol. 7, 463
- **MATZARAKI et al.** *Genome Biol.*, 2017, vol. 18, 76
- **VITA et al.** *Nucleic Acids Res.*, 2015, vol. 43, D405-D412
- **NIELSEN** ; **ANDREATTA**. *Genome Med.*, 2016, vol. 8, 33
- **PATTERSON et al.** *Mol. Cancer Ther.*, 2016, vol. 15, 313-322
- **TROLLE et al.** *J. Immunol.*, 2016, vol. 196, 1480-1487
- **KESXMIR et al.** *Protein Eng.*, 2002, vol. 15, 287-296
- **AKEN et al.** *Nucleic Acids Res.*, 2017, vol. 45 (D1), D635-D642
- **FORBES et al.** *Nucleic Acids Res.*, 2015, vol. 43, D805-D811
- **MARSH, S.G.E.** ; **PARHAM, P.** ; **BARBER, L.D.** The HLA FactsBook. Academic Press, 1999